# EUROPEAN PATENT SPECIFICATION

(11) **EP 1 966 191 B1**
(45) Date of publication and mention of the grant of the patent: **17.03.2010**
(21) Application number: 06841410.1
(22) Date of filing: 18.12.2006
(51) Int. Cl.: C07D 401/12, A61K 31/497, A61P 25/00

(54) **SUBSTITUTED PYRAZINONE DERIVATIVES AS ALPHA2C-ADRENORECEPTOR ANTAGONISTS**
SUBSTITUIERTE PYRADINON-DERIVATE ALS ALPHA2-ADRENOREZEPTOR-ANTAGONISTEN
DERIVES DE PYRAZINONE SUBSTITUEE COMME ANTAGONISTES DU RECEPTEUR ADRENERGIQUE ALPHA2C

(30) Priority: 21.12.2005 EP 05112646
(43) Date of publication of application: 10.09.2008
(73) Proprietor: Janssen Pharmaceutica N.V., 2340 Beerse (BE)
(72) Inventor: ANDRÉS-GIL, José, Ignacio, E-45007 Toledo (ES); ALCÁZAR-VACA, Manuel, Jesús, E-45007 Toledo (ES); LINARES DE LA MORENA, Maria Lourdes, E-45007 Toledo (ES); MARTINEZ GONZALEZ, Sonia, E-45007 Toledo (ES); OYARZABAL SANTAMARINA, Julen, E-45007 Toledo (ES); PASTOR-FERNÁNDEZ, Joaquin, E-45007 Toledo (ES); VEGA RAMIRO, Juan, Antonio, E-45007 Toledo (ES); DRINKENBURG, Wilhelmus, Helena, Ignatius, Maria, B-2340 Beerse (BE)
(74) Representative: Quaghebeur, Luc
(86) International application number: PCT/EP2006/069815
(87) International publication number: WO 2007/071639

(56) References cited:
- EP-A- 1 379 520
- WO-A-2004/067513

## Description

### Field of the Invention

The present invention concerns substituted pyrazinone derivatives having selective α_{2C}-adrenoceptor antagonist activity, as well as having 5-HT reuptake inhibition activity. It further relates to their preparation, pharmaceutical compositions comprising them and their use as a medicine, especially for the treatment of central nervous system disorders.

### Background of the Invention

Adrenergic receptors form the interface between the endogenous catecholamines epinephrine and norepinephrine and a wide array of target cells in the body to mediate the biological effects of the sympathetic nervous system. They are divided into three major subcategories, α₁, α₂ and β. To date, nine distinct adrenergic receptor subtypes have been cloned from several species: α_{1A}, α_{1B}, α_{1D}, α_{2A}, α_{2B}, α_{2C}, β₁, β₂ and β₃ (Hieble, J. P.; et al. J. Med. Chem. 1995, 38, 3415-3444). Available α₂ ligands have only marginal subtype selectivity. A complicating factor is that α₂-adrenoceptor ligands, which are imidazoles or imidazolines, also bind with moderate-to-high affinity to non-adrenoceptor imidazoline binding sites.

The three α₂-adrenoceptor subtypes share many common properties. They are G-protein-coupled receptors with seven transmembrane domains of the aminebinding subfamily. All three subtypes are coupled to the Gi/o signalling system, inhibiting the activity of adenylate cyclase, the opening of voltage-gated Ca²⁺ channels and the opening of K⁺ channels. The three receptors are encoded by distinct genes (Bylund, D. B.; et al. Pharmacol. Rev. 1994, 46, 121-136 and Hieble, J. P. et al. Pharmaco/. Commun. 1995, 6, 91-97), localized to different chromosomes; in humans the gene for α_{2A} is found on chromosome 10, the α_{2B}-gene on chromosome 2 and the α_{2C}-gene on chromosome 4. The subtypes are well conserved across mammalian species. In rats and mice, however, there is a single amino acid substitution which decreases the affinity of the rodent α_{2A}-adrenoceptor for the classical α₂-antagonists, yohimbine and rauwolscine. The general consensus is that this so-called α_{2D}-adrenoceptor subtype represents the rodent homologue of the human α_{2A}-subtype.

The α₂-adrenoceptor subtypes are differentially distributed in cells and tissues, clearly endowing the receptors with different physiological functions and pharmacological activity profiles. Different regulatory regions in the receptor genes and different protein structures also confer different regulatory properties on the three receptors, both with regard to receptor synthesis and post-translational events.

α₂-Adrenergic receptors were initially characterized as presynaptic receptors that serve as parts of a negative feedback loop to regulate the release of norepinephrine. Soon it was shown that α₂-adrenoceptors are not restricted to presynaptic locations but also have postsynaptic functions. The α_{2A}-adrenoceptor is the major inhibitory presynaptic receptor (autoreceptor) regulating release of norepinephrine from sympathetic neurons as part of a feedback loop. The α_{2C}-adrenoceptor turned out to function as an additional presynaptic regulator in all central and peripheral nervous tissues investigated. However, the relative contributions of α_{2A} and α_{2C}-receptors differed between central and peripheral nerves, with the α_{2C}-subtype being more prominent in sympathetic nerve endings than in central adrenergic neurons (Philipp, M. et al. Am. J. Physiol. Regul. Integr. Comput. Physiol. 2002,283, R287-R295 and Kable, J. W. et al. J. Pharmacol. Exp. Ther. 2000, 293, 1-7). The α_{2C}-adrenoceptor is particularly suited to control neurotransmitter release at low action potential frequencies. In contrast, the α_{2A}-adrenoceptor seems to operate primarily at high stimulation frequencies in sympathetic nerves and may thus be responsible for controlling norepinephrine release during maximal sympathetic activation (Bucheler, M. M. et al. Neuroscience 2002, 109, 819-826). α_{2B}-Adrenoceptors are located on postsynaptic cells to mediate the effects of catecholamines released from sympathetic nerves, e.g., vasoconstriction. α₂-Adrenergic receptors not only inhibit release of their own neurotransmitters but can also regulate the exocytosis of a number of other neurotransmitters in the central and peripheral nervous system. In the brain, α_{2A}- and α_{2C}-adrenoceptors can inhibit dopamine release in basal ganglia as well as serotonin secretion in mouse hippocampal or brain cortex slices. In contrast, the inhibitory effect of α₂-adrenoceptor agonists on gastrointestinal motility was mediated solely by the α_{2A}-subtype. Part of the functional differences between α_{2A}- and α_{2C}-receptors may be explained by their distinct subcellular localization patterns. When expressed in rat fibroblasts, α_{2A}- and α_{2B}-adrenoceptors are targeted to the plasma membrane. On stimulation with agonist, only α_{2B}-adrenoceptors are reversibly internalized into endosomes. α_{2C}-Adrenoceptors are primarily localized in an intracellular membrane compartment, from where they can be translocated to the cell surface after exposure to cold temperature (see a.o. Docherty J.R. et. al. Eur. J. Pharmacol. 1998, 361, 1-15).

The establishment of genetically engineered mice lacking or overexpressing α₂-adrenoceptor subtypes has yielded Important information for understanding the subtype specific functions (MacDonald, E. et al. Trends Pharmacol. Sci. 1997, 18, 211-219). The examination of the phenotype of these strains of mice demonstrated that the α_{2A}-subtype is responsible for inhibition of neurotransmitter release from central and peripheral sympathetic nerves and for most of the centrally mediated effects of α₂-agonists. The α_{2B} subtype is primarily responsible for the initial peripheral hypertensive responses evoked by the α₂-agonists and takes part in the hypertension induced by salt (Link et al. Science 1996, 273, 803-805 and Makaritsis, K. P. et al. Hypertension 1999, 33, 14-17).

Clarification of the physiological role of the α_{2C} subtype proved more difficult. Despite a rather wide distribution in the CNS, its role did not appear critical in the mediation of the cardiovascular effects of nonselective α₂-agonists. Its participation has been suggested in the hypothermia induced by dexmedetomidine and in the hyperlocomotion induced by D-amphetamine (Rohrer, D. K. et al. Annu. Rev. Pharmacol Toxicol. 1998, 38, 351-373). Another potentially important response mediated by the α_{2C}-adrenoceptor is constriction of cutaneous arteries, leading to a reduction in cutaneous blood flow (Chotani, M: A. et al. Am. J. Physiol. Heart Circ. Physiol. 2004, 286, 59-67). Recent studies carried out on double knockout mice have suggested that α_{2C}-adrenoceptor is also expressed at the presynaptic level where, together with α_{2A}, it actively participates in the control of neurotransmitter release. While α_{2A}-adrenoceptor is particularly efficient at high stimulation frequencies, α_{2C}-adrenoceptor acts rather at low stimulation frequencies. Moreover, it has been suggested that α_{2C} subtype participates in the modulation of motor behavior and the memory processes (Bjorklund, M. et al. Neuroscience 1999, 88, 1187-1198 and Tanila, H. et al. Eur. J. Neurosci. 1999, 11, 599-603). Other central effects triggered by this subtype include also the startle reflex and aggression response to stress and locomotion (Sallinen, J. et al. J. Neurosci. 1998, 18, 3035-3042 and Sallinen. J. et al. Neuroscience 1998, 86, 959-965). Last, it was recently pointed out that the α_{2C}-adrenoceptor might contribute to α₂-agonist-mediated spinal analgesia and adrenergic-opioid synergy (Fairbanks, C. A. et al. J. Pharm.Exp. Ther. 2002, 300, 282-290).

Because of their widespread distribution in the central nervous system, α₂-receptors affect a number of behavioral functions. The effect of altered α_{2C}-adrenergic receptor expression has been evaluated in several different behavioral paradigms (Kable J.W. et al., Journal of Pharmacology and Experimental Therapeutics, 2000, 293 (1): 1-7), proving that α_{2C}-adrenergic antagonists may have therapeutic value in the treatment of stress-related psychiatric disorders. In each of the behavioral paradigms, it is unclear whether the α_{2C}-subtype plays some direct role in mediating behavior or whether altered α_{2C}-receptor expression produces effects because of altered metabolism or downstream modulation of other neurotransmitter systems. Interestingly, α_{2C}-receptor-deficient mice had enhanced startle responses, diminished pre-pulse inhibition, and shortened attack latency in the isolation aggression test. Thus drugs acting via the α_{2C}-adrenoceptor may have therapeutic value in disorders associated with enhanced startle responses and sensorimotor gating deficits, such as schizophrenia, attention deficit disorder, posttraumatic stress disorder, and drug withdrawal. In addition to the α_{2C}-subtype, the α_{2A}-adrenoceptor has an important.

With more and more studies of the α₂-adrenoceptor physiology in gene-targeted mice being published, the situation becomes more complicated than initially anticipated. Indeed, only a few biological functions of α₂-receptors were found to be mediated by one single α₂-adrenergic receptor subtype. For other α₂-receptor-mediated functions, two different strategies seem to have emerged to regulate adrenergic signal transduction: some biological functions are controlled by two counteracting α₂-receptor subtypes, and some require two receptor subtypes with similar but complementary effects. Because the α_{2A}-subtype mediates most of the classical effects of α₂-adrenergic agonists, it is doubtful that an α_{2A}-selective agonist would have a substantially better clinical profile than the currently available agents. Drugs acting at α_{2B}- or α_{2C}-adrenergic receptors are likely to have fewer of the classical α₂-adrenergic side effects than α_{2A-}specific agents. It would appear likely that α_{2C}-selective agents may be useful in at least some nervous system disorders, in particular central nervous system disorders.

### Background prior art

Analysis of the pipeline databases to date indicate that there are several adrenergic α₂-antagonists in the market, by companies including Akzo Nobel (Organon), Novartis, Pfizer, and Schering AG. None of those compounds are selective for any of the three α₂-adrenoceptors. These compounds are indicated mainly for depression, hypertensive disorders and dyskinesias associated with Parkinson's disease. Companies with α₂-adrenoceptor antagonists in clinical development include Britannia Pharmaceuticals, IVAX, Juvantia Pharmaceuticals, MAP Pharmaceuticals, Novartis, Novo Nordisk, Organon, Pierre Fabre, and Sanofi-Aventis.

Regarding the development of selective α_{2C}-adrenoceptor antagonists to date, OPC-28326 is the only compound in clinical development (in Phase 2 by Otsuka Pharmaceuticals for hypertensive disorders and peripheral vascular disease). The rest of the α_{2C} antagonists are in preclinical development by Oy Juvantia Pharma Ltd (JP 1514 and JP 1302, published in WO 01/64645 and WO 04/067513) and by Novartis AG (NVP-ABE651 and NVP-ABE697, published in WO 01/55132 and J. Label Compd. Radiopharm 2002, 45, 1180), indicated mainly for depression and schizophrenia. In addition, several compounds are listed at the very early stages of development (biological testing) by Juvantia and Kyowa Hakko, for depression and Parkinson's disease.

### Description of the Invention

It is the object of the present invention to provide compounds with a binding affinity towards α₂-adrenoceptor receptors, in particular towards α_{2C}-adrenoceptor receptors, in particular as an antagonist.

This goal was achieved by a novel substituted pyrazinone derivative according to the general Formula (I) 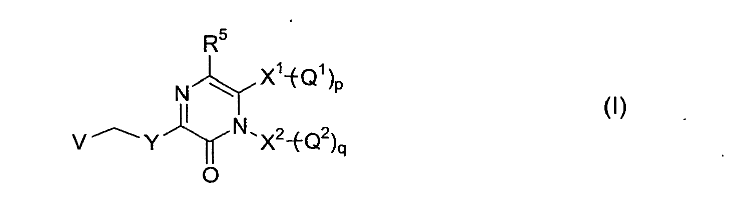 a pharmaceutically acceptable acid or base addition salt thereof, a stereochemically isomeric form thereof, an *N-*oxide form thereof or a quaternary ammonium salt thereof, wherein :
- V: is a naphthyl-radical, wherein one CH-unit in the napthyl-moiety may optionally be replaced by a *N-*atom;
- Y: is a bivalent radical of Formula (II) 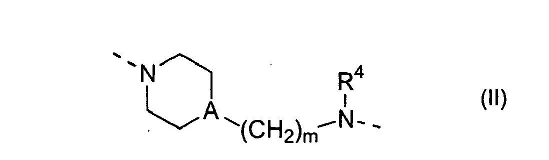 wherein

- A: is a nitrogen or a carbon-atom ;
- m: is an integer equal to zero, 1 or 2 ;
- R⁴: is selected from the group of hydrogen ; alkyl and phenylcarboxylalkyl ;
- R⁵: is selected from the group of hydrogen and halo ;
- X¹, X²: are each, independently from each other, a covalent bond, a saturated or an unsaturated (C₁₋₈)-hydrocarbon radical, wherein one or more bivalent -CH₂-units and/or one or more monovalent CH₃-units may optionally be replaced by a respective bivalent or monovalent phenyl-unit ; and wherein one or more hydrogen atoms may be replaced by a radical selected from the group of oxo ; (C₁₋₃)alkyloxy ; halo ; cyano ; nitro ; formyl ; hydroxy ; amino ; trifluoromethyl ; mono- and di((C₁₋₃)alkyl)amino ; carboxy ; and thio ;
- p, q: are each, independently from each other, an integer equal to 1 or 2;
- Q¹, Q²: are each, independently from each other, a radical selected from the group of hydrogen ; -NR¹R²; Pir; -OR³ and Het; wherein two radicals -OR³ may be taken together to form a bivalent radical, -O-(CH₂)ᵣ-O- wherein r is an integer equal to 1, 2 or 3 ;
- R¹ and R²: are each, independently from each other, a radical selected from the group of hydrogen ; alkyl ; alkenyl ; alkynyl ; aryl ; arylalkyl ; alkylcarbonyl ; alkenylcarbonyl ; alkyloxy ; alkyloxyalkyl ; alkyloxycarbonyl ; alkyloxyalkylcarbonyl ; alkyloxycarbonylalkyl ; alkyloxycarbonylalkylcarbonyl; alkylsulfonyl ; arylcarbonyl ; aryloxyalkyl ; arylalkylcarbonyl ; arylsulfonyl ; Het ; Het-alkyl ; Het-alkylcarbonyl ; Het-carbonyl ; Het-carbonylalkyl ; alkyl-NR^{a}R^{b} ; carbonyl-NR^{a}R^{b} ; carbonylalkyl-NR^{a}R^{b} ; alkylcarbonyl-NR^{a}R^{b} ; and alkylcarbonylalkyl-NR^{a}R^{b} ; wherein R^{a} and R^{b} are each independently selected from the group of hydrogen, alkyl, alkylcarbonyl, alkyloxyalkyl, alkyloxycarbonylalkyl, aryl, arylalkyl, Het and alkyl-NR^{c}R^{d}, wherein R^{c} and R^{d} are each independently from each other hydrogen or alkyl ;
- Pir: is a radical containing at least one N, by which it is attached to the X-radical, selected from the group of pyrrolidinyl ; imidazolidinyl ; pyrazolidinyl ; piperidinyl ; piperazinyl ; pyrrolyl ; pyrrolinyl ; imidazolinyl ; pyrrazolinyl ; pyrrolyl ; imidazolyl ; pyrazolyl ; triazolyl ; azepyl ; diazepyl ; morpholinyl ; thiomorpholinyl ; indolyl ; isoindolyl; indolinyl ; indazolyl ; benzimidazolyl ; and 1,2,3,4-tetrahydro-isoquinolinyl ; wherein each Pir-radical is optionally substituted by 1, 2 or 3 radicals selected from the group of hydroxy ; halo ; oxo ; (C₁₋₃)alkyl; trifluoromethyl ; phenyl ; benzyl ; pyrrolidinyl ; and pyridinyloxy ;
- R³: is a radical selected from the group of hydrogen ; alkyl ; aryl ; arylalkyl ; Het ; and Het-alkyl ;
- Het: is a heterocyclic radical selected from the group of pyrrolidinyl ; imidazolidinyl ; pyrazolidinyl ; piperidinyl ; piperazinyl ; pyrrolyl ; pyrrolinyl ; imidazolinyl ; pyrrazolinyl ; pyrrolyl ; imidazolyl ; pyrazolyl ; triazolyl ; pyridinyl ; pyridazinyl ; pyrimidinyl ; pyrazinyl ; triazinyl ; azepyl ; diazepyl ; morpholinyl ; thiomorpholinyl ; indolyl ; isoindolyl ; indolinyl ; indazolyl ; benzimidazolyl ; 1,2,3,4-tetrahydro-isoquinolinyl ; furyl ; thienyl ; oxazolyl ; isoxazolyl ; thiazolyl ; thiadiazolyl ; isothiazolyl ; dioxolyl ; dithianyl ; tetrahydrofuryl ; tetrahydropyranyl ; quinolinyl ; isoquinolinyl ; quinoxalinyl ; benzoxazolyl ; benzisoxazolyl; benzothiazolyl ; benzisothiazolyl ; benzofuranyl ; benzothienyl ; benzopiperidinyl ; chromenyl ; and imidazo[1,2-a]pyridinyl ; wherein each Het-radical is optionally substituted by one or more radicals selected from the group of halo ; oxo ; (C₁₋₃)alkyl; (C₁₋₃)alkylcarbonyl ; (C₁₋₃)alkenylthio ; imidazolyl-(C₁₋₃)alkyl ; and (C₁₋₃)alkyloxycarbonyl ;
- aryl: is naphthalenyl or phenyl, each optionally substituted with 1, 2 or 3 substituents, each independently from each other, selected from the group of oxo ; (C₁₋₃)alkyl; (C₁₋₃)alkyloxy ; halo ; cyano ; nitro ; formyl; hydroxy; amino ; trifluoromethyl ; mono- and di((C₁₋₃)alkyl)amino ; carboxy ; and thio ;
- alkyl: is a straight or branched saturated hydrocarbon radical having from 1 to 8 carbon atoms ; or is a cyclic saturated hydrocarbon radical having from 3 to 7 carbon atoms ; or is a cyclic saturated hydrocarbon radical having from 3 to 7 carbon atoms attached to a straight or branched saturated hydrocarbon radical having from 1 to 8 carbon atoms ; wherein each radical is optionally substituted on one or more carbon atoms with one or more radicals selected from the group of oxo ; (C₁₋₃)alkyloxy ; halo ; cyano ; nitro ; formyl ; hydroxy ; amino ; carboxy ; and thio ;
- alkenyl: is an alkyl radical as defined above, further having one or more double bonds ;
- alkynyl: is an alkyl radical as defined above, further having one or more triple bonds; and
- arylalkyl: is an alkyl radical as defined above, further having one or more CH₃-groups replaced by phenyl.

The invention also relates to a pharmaceutical composition comprising a pharmaceutically acceptable carrier or diluent and, as active ingredient, a therapeutically effective amount of a compound according to the invention, in particular a compound according to Formula (I), a pharmaceutically acceptable acid or base addition salt thereof, a stereochemically isomeric form thereof, an *N-*oxide form thereof or a quaternary ammonium salt thereof.

The invention also relates to the use of a compound according to the invention for the preparation of a medicament for the prevention and/or treatment of a disorder or disease responsive to antagonism of the α₂-adrenergic receptor, in particular to antagonism of the α_{2C}-adrenergic receptor.

In particular, the invention relates to the use of a compound according to the invention for the preparation of a medicament for the prevention and/or treatment of central nervous system disorders, mood disorders, anxiety disorders, stress-related disorders associated with depression and/or anxiety, cognitive disorders, personality disorders, schizoaffective disorders, Parkinson's disease, dementia of the Alzheimer's type, chronic pain conditions, neurodegenerative diseases, addiction disorders, mood disorders and sexual dysfunction.

The compounds according to the invention may also be suitable as add-on treatment and/or prophylaxis in the above listed diseases in combination with antidepressants, anxiolytics and/or antipsychotics which are currently available or in development or which will become available in the future, to improve efficacy and/or onset of action. This is evaluated in rodent models in which antidepressants, anxiolytics and/or antipsychotics are shown to be active. For example, compounds are evaluated in combination with antidepressants, anxiolytics and/or antipsychotics for attenuation of stress-induced hyperthermia.

The invention therefore also relates to the use of the compounds according to the invention for use as an add-on treatment with one or more other compounds selected from the group of antidepressants, anxiolytics and antipsychotics, to a pharmaceutical composition comprising the compounds according to the invention and one or more other compounds selected from the group of antidepressants, anxiolytics and antipsychotics, as well as to a process for the preparation of such pharmaceutical compositions and to the use of such a composition for the manufacture of a medicament, in particular to improve efficacy and/or onset of action in the treatment of depression and/or anxiety.

### Detailed description of the invention

In one embodiment, the invention relates to a compound according to general Formula (I), a pharmaceutically acceptable acid or base addition salt thereof, a stereochemically isomeric form thereof, an *N*-oxide form thereof or a quaternary ammonium salt thereof, wherein V is selected from the group of radicals (z-1), (z-2), (z-3), (z-4), (z-5) and (z-6).

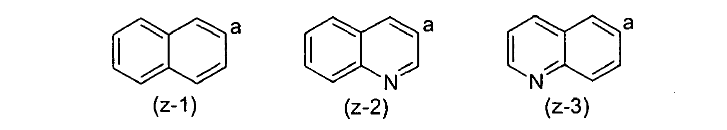

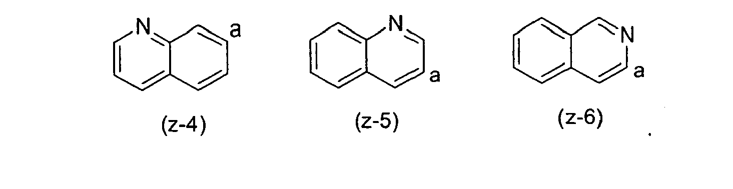

More particularly, V is selected from the group of radicals (z-1), (z-2), (z-3), (z-5), and (z-6).

In another embodiment, the invention relates to a compound according to general Formula (I), a pharmaceutically acceptable acid or base addition salt thereof, a stereochemically isomeric form thereof, an *N-*oxide form thereof or a quaternary ammonium salt thereof, wherein A is a carbon atom, m is zero and R⁴ is hydrogen.

In another embodiment, the invention relates to a compound according to general Formula (I), a pharmaceutically acceptable acid or base addition salt thereof, a stereochemically isomeric form thereof, an *N-*oxide form thereof or a quaternary ammonium salt thereof, wherein the moiety -CH₂-Y- is attached to V by the atom, denoted by "a".

In another embodiment, the invention relates to a compound according to general Formula (I), a pharmaceutically acceptable acid or base addition salt thereof, a stereochemically isomeric form thereof, an *N-*oxide form thereof or a quaternary ammonium salt thereof, wherein R⁵ is chloro.

In another embodiment, the invention relates to a compound according to general Formula (I), a pharmaceutically acceptable acid or base addition salt thereof, a stereochemically isomeric form thereof, an *N-*oxide form thereof or a quaternary ammonium salt thereof, wherein each of X¹ and X², independently from each other, are selected from the group of a covalent bond ; -CH₂- ; -CH₂CH₂- ; -CH₂CH₂CH₂- ; -CH₂CH₂CH₂CH₂- ; -CH₂CH=CHCH₂-; -CH₂C≡CCH₂- ; -CH(CH₃)CH(CH₃)- ; -C(=O)CH₂- ; -C(=O)CH₂CH₂- ; -C(=O)CH₂CH₂CH₂- ; -CH₂C(=O)- ; -CH₂CH₂C(=O)- ; -CH₂CH₂CH₂C(=O)- ; -CH₂CH₂CH₂CH₂C(=O)- ; -CH₂CH₂C(=O)CH₂- ; -C₆H₄- ; -CH₂C₆H₄- ; -CH₂CH₂C₆H₄- ; -CH₂CH₂CH₂C₆H₄- ; -C₆H₄CH₂- ; -C₆H₄CH₂CH₂- ; -C₆H₄CH₂CH₂CH₂- ; -CH₂C₆H₄CH₂- ; -CH₂CH₂C₆H₄CH₂CH₂- ; -C₆H₄C(=O)- ; -C₆H₄CH₂C(=O)- ; -C₆H₄CH₂CH₂C(=O)- ; and -CH₂CH₂C(=O)C₆H₄-.

In another embodiment, the invention relates to a compound according to general Formula (I), a pharmaceutically acceptable acid or base addition salt thereof, a stereochemically isomeric form thereof, an *N-*oxide form thereof or a quaternary ammonium salt thereof, wherein each of X¹ and X², independently from each other, are selected from the group of a covalent bond ; -CH₂- ; -CH₂CH₂- ; -CH₂CH₂CH₂- ; -CH₂CH₂CH₂CH₂- ; -CH₂CH=CHCH₂-; -CH₂C≡CCH₂- ; -CH₂C(=O)- ; -CH₂CH₂C(=O)- ; -CH₂CH₂CH₂C(=O)- ; -CH₂CH₂CH₂CH₂C(=O)- ; -C₆H₄- ; -CH₂C₆H₄- ; -CH₂CH₂CH₂C₆H₄- ; -C₆H₄CH₂- ; -CH₂C₆H₄CH₂- ; -C₆H₄C(=O)- ; -C₆H₄CH₂C(=O)- ; -C₆H₄CH₂CH₂C(=O)- ; and -CH₂CH₂C(=O)C₆H₄-.

In another embodiment, the invention relates to a compound according to general Formula (I), a pharmaceutically acceptable acid or base addition salt thereof, a stereochemically isomeric form thereof, an *N-*oxide form thereof or a quaternary ammonium salt thereof, wherein one or more hydrogen atoms in each of X¹ and X² are optionally replaced by a radical selected from the group of oxo ; (C₁₋₃)alkyloxy ; halo ; cyano ; nitro ; and formyl.

In another embodiment, the invention relates to a compound according to general Formula (I), a pharmaceutically acceptable acid or base addition salt thereof, a stereochemically isomeric form thereof, an *N-*oxide form thereof or a quaternary ammonium salt thereof, wherein X¹ is a covalent bond, Q¹ is hydrogen and p is 1.

In another embodiment, the invention relates to a compound according to general Formula (I), a pharmaceutically acceptable acid or base addition salt thereof, a stereochemically isomeric form thereof, an *N-*oxide form thereof or a quaternary ammonium salt thereof, wherein R¹ and R² are each, independently from each other, a radical selected from the group of hydrogen ; alkyl ; alkenyl ; alkynyl ; aryl ; arylalkyl ; alkylcarbonyl ; alkenylcarbonyl ; alkyloxyalkyl ; alkyloxycarbonyl ; alkyloxyalkylcarbonyl ; alkyloxycarbonylalkyl ; alkyloxycarbonylalkylcarbonyl ; arylcarbonyl ; aryloxyalkyl ; arylalkylcarbonyl ; Het-alkyl ; Het-alkylcarbonyl ; Het-carbonyl ; Het-carbonylalkyl ; alkyl-NR^{a}R^{b} ; carbonyl-NR^{a}R^{b} ; carbonylalkyl-NR^{a}R^{b} ; alkylcarbonyl-NR^{a}R^{b} ; and alkylcarbonylalkyl-NR^{a}R^{b} ; wherein each of R^{a} and R^{b} independently are selected from the group of hydrogen, alkyl, alkylcarbonyl, alkyloxyalkyl, alkyloxycarbonylalkyl, aryl, arylalkyl, Het and alkyl-NR^{c}R^{d}, wherein R^{c} and R^{d} are each independently from each other hydrogen or alkyl.

In another embodiment, the invention relates to a compound according to general Formula (I), a pharmaceutically acceptable acid or base addition salt thereof, a stereochemically isomeric form thereof, an *N-*oxide form thereof or a quaternary ammonium salt thereof, wherein Pir is a radical containing at least one N, by which it is attached to the X-radical, selected from the group of pyrrolidinyl ; piperidinyl ; piperazinyl ; imidazolyl; morpholinyl ; isoindolyl ; wherein each Pir-radical is optionally substituted by 1, 2 or 3 radicals selected from the group of hydroxy ; halo ; oxo ; (C₁₋₃)alkyl; trifluoromethyl ; phenyl ; benzyl ; pyrrolidinyl ; and pyridinyloxy. With the X-radical is meant either or both of the X¹-radical and the X²-radical.

More particularly, the invention relates to a compound according to general Formula (I), a pharmaceutically acceptable acid or base addition salt thereof, a stereochemically isomeric form thereof, an *N-*oxide form thereof or a quaternary ammonium salt thereof, wherein Het is a heterocyclic radical selected from the group of piperidinyl ; piperazinyl ; triazolyl; pyridinyl ; pyrimidinyl ; morpholinyl ; indolyl ; furyl ; thienyl ; isoxazolyl; thiazolyl ; tetrahydrofuryl ; tetrahydropyranyl ; quinolinyl ; isoquinolinyl ; benzofuranyl ; benzothienyl ; and benzopiperidinyl ; wherein each Het-radical is optionally substituted by one or more radicals selected from the group of oxo ; (C₁₋₃)alkyl; (C₁₋₃)alkylcarbonyl ; and imidazolyl-(C₁₋₃)alkyl.

Most particularly, the invention relates to a compound according to general Formula (I), a pharmaceutically acceptable acid or base addition salt thereof, a stereochemically isomeric form thereof, an *N-*oxide form thereof or a quaternary ammonium salt thereof, wherein aryl is phenyl, optionally substituted with 1, 2 or 3 substituents, each independently from each other, selected from the group of (C₁₋₃)alkyl; halo; and trifluoromethyl.

In another embodiment, the invention relates to a compound according to general Formula (I), a pharmaceutically acceptable acid or base addition salt thereof, a stereochemically isomeric form thereof, an *N-*oxide form thereof or a quaternary ammonium salt thereof, wherein
- V: is selected from the group of radicals (z-1), (z-2), (z-3), (z-5) and (z-6) ; wherein the moiety -CH₂-Y- is attached to V by the atom, denoted by "a";
- Y: is a bivalent radical of Formula (II) wherein A is a nitrogen or a carbon-atom ; m is an integer equal to zero or 2 ; and R⁴ is selected from the group of hydrogen ; alkyl and phenylcarboxylalkyl ;
- R⁵: is selected from the group of hydrogen and halo ;
- X¹, X²: are each, independently from each other, are selected from the group of a covalent bond ; -CH₂- ; -CH₂CH₂- ; -CH₂CH₂CH₂- ; -CH₂CH₂CH₂CH₂- ; -CH₂CH=CHCH₂-; -CH₂C≡CCH₂- ; -CH₂C(=O)- ; -CH₂CH₂C(=O)- ; -CH₂CH₂CH₂C(=O)- ; -CH₂CH₂CH₂CH₂C(=O)- ; -C₆H₄- ; -CH₂C₆H₄- ; -CH₂CH₂CH₂C₆H₄-; -C₆H₄CH₂- ; -CH₂C₆H₄CH₂- ; -C₆H₄C(=O)- ; -C₆H₄CH₂C(=O)- ; -C₆H₄CH₂CH₂C(=O)- ; and -CH₂CH₂C(=O)C₆H₄- ; and wherein one or more hydrogen atoms may be replaced by a radical selected from the group of oxo ; (C₁₋₃)alkyloxy ; halo ; cyano ; nitro; and formyl ;
- p,: q are each, independently from each other, an integer equal to 1 or 2;
- Q¹, Q²: are each, independently from each other, a radical selected from the group of hydrogen ; -NR¹R² ; Pir ; -OR³ and Het; wherein two radicals -OR³ may be taken together to form a bivalent radical -O-(CH₂)ᵣ-Owherein r is an integer equal to 1, 2 or 3 ;
- R¹ and R²: are each, independently from each other, a radical selected from the group of hydrogen ; alkyl ; alkenyl ; alkynyl ; aryl ; arylalkyl ; alkylcarbonyl ; alkenylcarbonyl ; alkyloxyalkyl ; alkyloxycarbonyl ; alkyloxyalkylcarbonyl ; alkyloxycarbonylalkyl ; alkyloxycarbonylalkylcarbonyl; arylcarbonyl ; aryloxyalkyl ; arylalkylcarbonyl ; Het-alkyl ; Het-alkylcarbonyl ; Het-carbonyl ; Het-carbonylalkyl ; alkyl-NR^{a}R^{b} ; carbonyl-NR^{a}R^{b} ; carbonylalkyl-NR^{a}R^{b} ; alkylcarbonyl-NR^{a}R^{b} ; and alkylcarbonylalkyl-NR^{a}R^{b} ; wherein R^{a} and R^{b} are each independently selected from the group of hydrogen, alkyl, alkylcarbonyl, alkyloxyalkyl, alkyloxycarbonylalkyl, aryl, arylalkyl, Het and alkyl-NR^{c}R^{d}, wherein R^{c} and R^{d} are each independently from each other hydrogen or alkyl ;
- Pir: is a radical containing at least one N, by which it is attached to the X-radical, selected from the group of pyrrolidinyl ; piperidinyl ; piperazinyl ; imidazolyl; morpholinyl ; isoindolyl ; wherein each Pir-radical is optionally substituted by 1, 2 or 3 radicals selected from the group of hydroxy ; halo; oxo ; (C₁₋₃)alkyl ; trifluoromethyl ; phenyl ; benzyl ; pyrrolidinyl ; and pyridinyloxy ;
- R³: is a radical selected from the group of hydrogen ; alkyl ; aryl ; arylalkyl; Het ; and Het-alkyl ;
- Het: is a heterocyclic radical selected from the group of piperidinyl ; piperazinyl ; triazolyl; pyridinyl ; pyrimidinyl ; morpholinyl ; indolyl ; furyl ; thienyl ; isoxazolyl; thiazolyl ; tetrahydrofuryl ; tetrahydropyranyl ; quinolinyl ; isoquinolinyl ; benzofuranyl ; benzothienyl ; and benzopiperidinyl ; wherein each Het-radical is optionally substituted by one or more radicals selected from the group of oxo ; (C₁₋₃)alkyl; (C₁₋₃)alkylcarbonyl ; and imidazolyl-(C₁₋₃)alkyl ;
- aryl: is phenyl, optionally substituted with 1, 2 or 3 substituents, each independently from each other, selected from the group of (C₁₋₃)alkyl; halo ; and trifluoromethyl ;
- alkyl: is a straight or branched saturated hydrocarbon radical having from 1 to 8 carbon atoms ; or is a cyclic saturated hydrocarbon radical having from 3 to 7 carbon atoms ; or is a cyclic saturated hydrocarbon radical having from 3 to 7 carbon atoms attached to a straight or branched saturated hydrocarbon radical having from 1 to 8 carbon atoms ; wherein each radical is optionally substituted on one or more carbon atoms with one or more radicals selected from the group of oxo ; (C₁₋₃)alkyloxy ; halo ; cyano ; nitro ; formyl ; hydroxy ; amino ; carboxy ; and thio ;
- alkenyl: is an alkyl radical as defined above, further having one or more double bonds ;
- alkynyl: is an alkyl radical as defined above, further having one or more triple bonds ; and
- arylalkyl: is an alkyl radical as defined above, further having one or more CH₃-groups replaced by phenyl.

In the framework of this application, alkyl is a straight or branched saturated hydrocarbon radical having from 1 to 8 carbon atoms ; or is a cyclic saturated hydrocarbon radical having from 3 to 7 carbon atoms ; or is a cyclic saturated hydrocarbon radical having from 3 to 7 carbon atoms attached to a straight or branched saturated hydrocarbon radical having from 1 to 8 carbon atoms ; wherein each radical is optionally substituted on one or more carbon atoms with one or more radicals selected from the group of oxo ; (C₁₋₃)alkyloxy; halo ; cyano ; nitro ; formyl ; hydroxy ; amino ; carboxy ; and thio. Particularly, alkyl is methyl, ethyl, propyl, isopropyl, butyl, tert-butyl, pentyl, hexyl, pentyl, octyl, cyclopropyl, cyclopentyl, cyclohexyl, cyclohexylmethyl and cyclohexylethyl.

Finally, the invention relates to a compound according to general Formula (I), a pharmaceutically acceptable acid or base addition salt thereof, a stereochemically isomeric form thereof, an *N-*oxide form thereof or a quaternary ammonium salt thereof, wherein one or more the following restrictions, alone or in combination, apply :
- V is selected from the group of radicals (z-1), (z-2), (z-3), (z-4), (z-5) and (z-6) ; or
- V is selected from the group of radicals (z-1), (z-2), (z-3), (z-5), and (z-6) ;
- A is a carbon atom, m is zero and R⁴ is hydrogen ;
- the moiety -CH₂-Y- is attached to V by the atom, denoted by "a" ;
- R⁵ is chloro ;
- each of X¹ and X², independently from each other, are selected from the group of a covalent bond ; -CH₂- ; -CH₂CH₂-; -CH₂CH₂CH₂- ; -CH₂CH₂CH₂CH₂- ; -CH₂CH=CHCH₂- ; -CH₂C≡CCH₂- ; -CH(CH₃)CH(CH₃)- ; -C(=O)CH₂- ; -C(=O)CH₂CH₂- ; -C(=O)CH₂CH₂CH₂- ; -CH₂C(=O)- ; -CH₂CH₂C(=O)- ; -CH₂CH₂CH₂C(=O)- ; -CH₂CH₂CH₂CH₂C(=O)- ; -CH₂CH₂C(=O)CH₂- ; -C₆H₄- ; -CH₂C₆H₄- ; -CH₂CH₂C₆H₄- ; -CH₂CH₂CH₂C₆H₄- ; -C₆H₄CH₂- ; -C₆H₄CH₂CH₂- ; -C₆H₄CH₂CH₂CH₂- ; -CH₂C₆H₄CH₂- ; -CH₂CH₂C₆H₄CH₂CH₂- ; -C₆H₄C(=O)- ; -C₆H₄CH₂C(=O)- ; -C₆H₄CH₂CH₂C(=O)- ; and -CH₂CH₂C(=O)C₆H₄- ; or
- each of X¹ and X², independently from each other, are selected from the group of a covalent bond ; -CH₂- ; -CH₂CH₂- ; -CH₂CH₂CH₂- ; -CH₂CH₂CH₂CH₂- ; -CH₂CH=CHCH₂-; -CH₂C≡CCH₂- ; -CH₂C(=O)- ; -CH₂CH₂C(=O)- ; -CH₂CH₂CH₂C(=O)- ; -CH₂CH₂CH₂CH₂C(=O)- ; -C₆H₄- ; -CH₂C₆H₄- ; -CH₂CH₂CH₂C₆H₄- ; -C₆H₄CH₂- ; -CH₂C₆H₄CH₂- ; -C₆H₄C(=O)- ; -C₆H₄CH₂C(=O)- ; -C₆H₄CH₂CH₂C(=O)- ; and -CH₂CH₂C(=O)C₆H₄- ;
- one or more hydrogen atoms in each of X¹ and X² are optionally replaced by a radical selected from the group of oxo ; (C₁₋₃)alkyloxy ; halo ; cyano ; nitro ; and formyl ;
- X¹ is a covalent bond, Q¹ is hydrogen and p is 1 ;
- R¹ and R² are each, independently from each other, a radical selected from the group of hydrogen ; alkyl ; alkenyl ; alkynyl ; aryl ; arylalkyl ; alkylcarbonyl ; alkenylcarbonyl ; alkyloxyalkyl ; alkyloxycarbonyl ; alkyloxyalkylcarbonyl ; alkyloxycarbonylalkyl ; alkyloxycarbonylalkylcarbonyl ; arylcarbonyl ; aryloxyalkyl ; arylalkylcarbonyl ; Het-alkyl ; Het-alkylcarbonyl ; Het-carbonyl ; Het-carbonylalkyl ; alkyl-NR^{a}R^{b} ; carbonyl-NR^{a}R^{b} ; carbonylalkyl-NR^{a}R^{b} ; alkylcarbonyl-NR^{a}R^{b} ; and alkylcarbonylalkyl-NR^{a}R^{b} ; wherein each of R^{a} and R^{b} independently are selected from the group of hydrogen, alkyl, alkylcarbonyl, alkyloxyalkyl, alkyloxycarbonylalkyl, aryl, arylalkyl, Het and alkyl-NR^{c}R^{d}, wherein R^{c} and R^{d} are each independently from each other hydrogen or alkyl ;
- Pir is a radical containing at least one N, by which it is attached to the X-radical, selected from the group of pyrrolidinyl ; piperidinyl ; piperazinyl ; imidazolyl; morpholinyl ; isoindolyl ; wherein each Pir-radical is optionally substituted by 1, 2 or 3 radicals selected from the group of hydroxy ; halo ; oxo ; (C₁₋₃)alkyl; trifluoromethyl ; phenyl ; benzyl ; pyrrolidinyl ; and pyridinyloxy ;
- Het is a heterocyclic radical selected from the group of piperidinyl ; piperazinyl ; triazolyl; pyridinyl ; pyrimidinyl ; morpholinyl ; indolyl ; furyl ; thienyl ; isoxazolyl; thiazolyl ; tetrahydrofuryl ; tetrahydropyranyl ; quinolinyl ; isoquinolinyl ; benzofuranyl ; benzothienyl ; and benzopiperidinyl ; wherein each Het-radical is optionally substituted by one or more radicals selected from the group of oxo ; (C₁₋₃)alkyl; (C₁₋₃)alkylcarbonyl ; and imidazolyl-(C₁₋₃)alkyl ;
- aryl is phenyl, optionally substituted with 1, 2 or 3 substituents, each independently from each other, selected from the group of (C₁₋₃)alkyl; halo; and trifluoromethyl.

In the framework of this application, alkenyl is an alkyl radical as defined above having one or more double bonds. Particularly, alkenyl is ethenyl, propenyl and butynyl.

In the framework of this application, alkynyl is an alkyl radical as defined above having one or more triple bonds. Particularly, alkynyl is ethynyl and propynyl.

In the framework of this application, arylalkyl is an alkyl radical as defined above, having one or more -CH₃-radicals replaced by phenyl-radical. Examples of such radicals are benzyl, diphenylmethyl and 1,1-diphenylethyl.

In the framework of this application, halo is a substituent selected from the group of fluoro, chloro, bromo and iodo and haloalkyl is a straight or branched saturated hydrocarbon radical having from 1 to 6 carbon atoms or a cyclic saturated hydrocarbon radical having from 3 to 7 carbon atoms, wherein one or more carbon atoms is substituted with one or more halo atoms. Particularly, halo is bromo, fluoro or chloro and particularly, haloalkyl is trifluoromethyl.

In the framework of this application, with "compounds according to the invention" is meant a compound according to the general Formula (I), a pharmaceutically acceptable acid or base addition salt thereof, a stereochemically isomeric form thereof, an *N-*oxide form thereof or a quaternary ammonium salt thereof.

The pharmaceutically acceptable acid addition salts are defined to comprise the therapeutically active non-toxic acid addition salts forms that the compounds according to Formula (I) are able to form. Said salts can be obtained by treating the base form of the compounds according to Formula (I) with appropriate acids, for example inorganic acids, for example hydrohalic acid, in particular hydrochloric acid, hydrobromic acid, sulphuric acid, nitric acid and phosphoric acid ; organic acids, for example acetic acid, hydroxyacetic acid, propanoic acid, lactic acid, pyruvic acid, oxalic acid, malonic acid, succinic acid, maleic acid, fumaric acid, malic acid, tartaric acid, citric acid, methanesulfonic acid, ethanesulfonic acid, benzenesulfonic acid, p-toluenesulfonic acid, cyclamic acid, salicylic acid, p-aminosalicylic acid and pamoic acid.

Conversely said acid addition salt forms can be converted into the free base form by treatment with an appropriate base.

The compounds according to Formula (I) containing acidic protons may also be converted into their therapeutically active non-toxic metal or amine addition salts forms (base addition salts) by treatment with appropriate organic and inorganic bases. Appropriate base salts forms comprise, for example, the ammonium salts, the alkaline and earth alkaline metal salts, in particular lithium, sodium, potassium, magnesium and calcium salts, salts with organic bases, e.g. the benzathine, *N-*methyl-D-glucamine, hybramine salts, and salts with amino acids, for example arginine and lysine.

Conversely, said salts forms can be converted into the free forms by treatment with an appropriate acid.

Quaternary ammonium salts of compounds according to Formula (I) defines said compounds which are able to form by a reaction between a basic nitrogen of a compound according to Formula (I) and an appropriate quaternizing agent, such as, for example, an optionally substituted alkylhalide, arylhalide or arylalkylhalide, in particular methyliodide and benzyliodide. Other reactants with good leaving groups may also be used, such as, for example, alkyl trifluoromethanesulfonates, alkyl methanesulfonates and alkyl p-toluenesulfonates. A quaternary ammonium salt has a positively charged nitrogen. Pharmaceutically acceptable counterions include chloro, bromo, iodo, trifluoroacetate and acetate ions.

The term addition salt as used in the framework of this application also comprises the solvates that the compounds according to Formula (I) as well as the salts thereof, are able to form. Such solvates are, for example, hydrates and alcoholates.

The *N-*oxide forms of the compounds according to Formula (I) are meant to comprise those compounds of Formula (I) wherein one or several nitrogen atoms are oxidized to the so-called *N-*oxide, particularly those *N-*oxides
wherein one or more tertiary nitrogens (e.g. of the piperazinyl or piperidinyl radical) are *N-*oxidized. Such *N-*oxides can easily be obtained by a skilled person without any inventive skills and they are obvious alternatives for the compounds according to Formula (I) since these compounds are metabolites, which are formed by oxidation in the human body upon uptake. As is generally known, oxidation is normally the first step involved in drug metabolism (Textbook of Organic Medicinal and Pharmaceutical Chemistry, 1977, pages 70-75). As is also generally known, the metabolite form of a compound can also be administered to a human instead of the compound per se, with much the same effects.

The compounds of Formula (I) may be converted to the corresponding *N-*oxide forms following art-known procedures for converting a trivalent nitrogen into its *N-*oxide form. Said *N-*oxidation reaction may generally be carried out by reacting the starting material of Formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. *tert*-butyl hydroperoxide. Suitable solvents are, for example, water, lower alkanols, e.g. ethanol and the like, hydrocarbons, e.g. toluene, ketones, e.g. 2-butanone, halogenated hydrocarbons, e.g. dichloromethane, and mixtures of such solvents.

The term "stereochemically isomeric forms" as used hereinbefore defines all the possible isomeric forms that the compounds of Formula (I) may possess. Unless otherwise mentioned or indicated, the chemical designation of compounds denotes the mixture of all possible stereochemically isomeric forms, said mixtures containing all diastereomers and enantiomers of the basic molecular structure. More in particular, stereogenic centers may have the R- or S-configuration; substituents on bivalent cyclic (partially) saturated radicals may have either the cis- or trans-configuration. Compounds encompassing double bonds can have an E or Z-stereochemistry at said double bond. Stereochemically isomeric forms of the compounds of Formula (I) are obviously intended to be embraced within the scope of this invention.

Following CAS nomenclature conventions, when two stereogenic centers of known absolute configuration are present in a molecule, an *R* or *S* descriptor is assigned (based on Cahn-Ingold-Prelog sequence rule) to the lowest-numbered chiral center, the reference center. The configuration of the second stereogenic center is indicated using relative descriptors [*R*,R**] or [*R*,S**], where R* is always specified as the reference center and [*R*,R**] indicates centers with the same chirality and [*R*,S**] indicates centers of unlike chirality. For example, if the lowest-numbered chiral center in the molecule has an *S* configuration and the second center is *R*, the stereo descriptor would be specified as *S*-[*R*,S**]. If "α" and "β" are used : the position of the highest priority substituent on the asymmetric carbon atom in the ring system having the lowest ring number, is arbitrarily always in the "α" position of the mean plane determined by the ring system. The position of the highest priority substituent on the other asymmetric carbon atom in the ring system (hydrogen atom in compounds according to Formula (I)) relative to the position of the highest priority substituent on the reference atom is denominated "α", if it is on the same side of the mean plane determined by the ring system, or "β", if it is on the other side of the mean plane determined by the ring system.

The invention also comprises derivative compounds (usually called "pro-drugs") of the pharmacologically-active compounds according to the invention, which are degraded *in vivo* to yield the compounds according to the invention. Pro-drugs are usually (but not always) of lower potency at the target receptor than the compounds to which they are degraded. Pro-drugs are particularly useful when the desired compound has chemical or physical properties that make its administration difficult or inefficient. For example, the desired compound may be only poorly soluble, it may be poorly transported across the mucosal epithelium, or it may have an undesirably short plasma half-life. Further discussion on pro-drugs may be found in Stella, V. J. et al., "Prodrugs", Drug Delivery Systems, 1985, pp. 112-176, and Drugs, 1985, 29, pp. 455-473.

Pro-drugs forms of the pharmacologically-active compounds according to the invention will generally be compounds according to Formula (I), the pharmaceutically acceptable acid or base addition salts thereof, the stereochemically isomeric forms thereof and the *N-*oxide form thereof, having an acid group which is esterified or amidated. Included in such esterified acid groups are groups of the formula -COOR^{x}, where R^{x} is a C₁₋₆alkyl, phenyl, benzyl or one of the following groups :

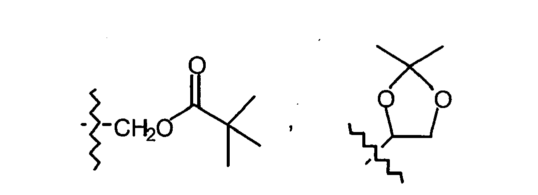

Amidated groups include groups of the formula - CONR^{y}R^{z}, wherein R^{y} is H, C₁₋₆alkyl, phenyl or benzyl and R^{z} is -OH, H, C₁₋₆alkyl, phenyl or benzyl. Compounds according to the invention having an amino group may be derivatised with a ketone or an aldehyde such as formaldehyde to form a Mannich base. This base will hydrolyze with first order kinetics in aqueous solution.

In the framework of this application, with "compounds according to the invention" is meant a compound according to the general Formula (I), the pharmaceutically acceptable acid or base addition salts thereof, the stereochemically isomeric forms thereof, the *N-*oxide form thereof and a prodrug thereof.

In the framework of this application, an element, in particular when mentioned in relation to a compound according to Formula (I), comprises all isotopes and isotopic mixtures of this element, either naturally occuring or synthetically produced, either with natural abundance or in an isotopically enriched form. In particular, when hydrogen is mentioned, it is understood to refer to ¹H, ²H, ³H and mixtures thereof ; when carbon is mentioned, it is understood to refer to ¹¹C, ¹²C, ¹³C, ¹⁴C and mixtures thereof; when nitrogen is mentioned, it is understood to refer to ¹³N, ¹⁴N, ¹⁵N and mixtures thereof; when oxygen is mentioned, it is understood to refer to ¹⁴O, ¹⁵O, ¹⁶O, ¹⁷O, ¹⁸O and mixtures thereof; and when fluor is mentioned, it is understood to refer to ¹⁸F, ¹⁹F and mixtures thereof.

The compounds according to the invention therefore also comprise compounds with one or more isotopes of one or more element, and mixtures thereof, including radioactive compounds, also called radiolabelled compounds,
wherein one or more non-radioactive atoms has been replaced by one of its radioactive isotopes. By the term "radiolabelled compound" is meant any compound according to Formula (I), an *N-*oxide form, a pharmaceutically acceptable addition salt or a stereochemically isomeric form thereof, which contains at least one radioactive atom. For example, compounds can be labelled with positron or with gamma emitting radioactive isotopes. For radioligand-binding techniques (membrane receptor assay), the ³H-atom or the ¹²⁵I-atom is the atom of choice to be replaced. For imaging, the most commonly used positron emitting (PET) radioactive isotopes are ¹¹C, ¹⁸F, ¹⁵O and ¹³N, all of which are accelerator produced and have half-lives of 20, 100, 2 and 10 minutes respectively. Since the half-lives of these radioactive isotopes are so short, it is only feasible to use them at institutions which have an accelerator on site for their production, thus limiting their use. The most widely used of these are ¹⁸F, ^{99m}Tc, ²⁰¹TI and ¹²³I. The handling of these radioactive isotopes, their production, isolation and incorporation in a molecule are known to the skilled person.

In particular, the radioactive atom is selected from the group of hydrogen, carbon, nitrogen, sulfur, oxygen and halogen. Particularly, the radioactive atom is selected from the group of hydrogen, carbon and halogen.

In particular, the radioactive isotope is selected from the group of ³H, ¹¹C, ¹⁸F, ¹²I, ¹²³I, ¹²⁵I, ¹³¹I, ⁷⁵Br, ⁷⁶Br, ⁷⁷Br and ⁸²Br. Particularly, the radioactive isotope is selected from the group of ³H, ¹¹C and ¹⁸F.

### Preparation

The compounds according to the invention can generally be prepared by a succession of steps, each of which is known to the skilled person. In particular, the pyrazinone derivatives can be prepared according to one or more of the following preparation methods.

### Preparation of the intermediate compound (I-4).

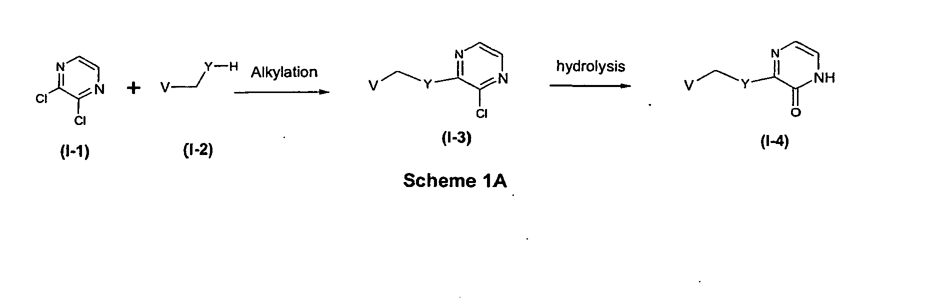

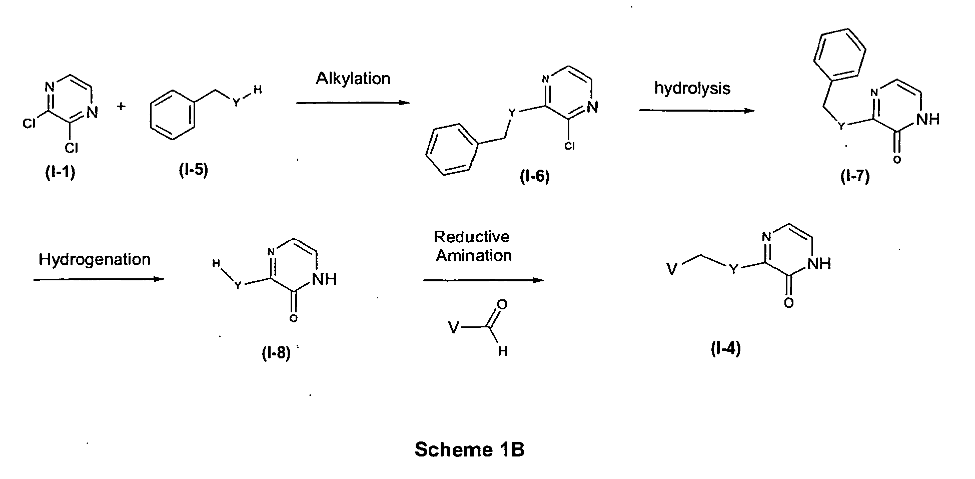

Alkylation reactions of the starting material 2,3-dichloropyrazine (I-1) with aminoderivatives (I-2) (Scheme 1A) or (I-5) (Scheme 1B) may be performed in an aprotic solvent, such as, for instance DMF or DMSO, in the presence of an inorganic base, such as K₂CO₃, Na₂CO₃, NaOH or KOH, at a convenient temperature, either by conventional heating or under microwave irradiation, for a period of time to ensure the completion of the reaction, which may typically be about 16 hours under conventional heating.

Hydrolysis reactions may be performed either in acidic inorganic solvents, such as 10% HCl_{aq}, using a co-solvent such as THF, by conventional heating or under microwave heating, for a period of time to ensure the completion of the reaction, which may typically be about 16 hours under conventional heating, or under basic conditions, such as in NaOH_{aq} or in a DMSO solvent, for a period of time to ensure the completion of the reaction, which may typically be about 0.5 hours under microwave irradiation.

Hydrogenation may be performed in an alcoholic solvent, such as MeOH, in the presence of AcOH and Pd/C, under conventional heating, for a period of time to ensure the completion of the reaction, which may typically be about 16 hours at about 50 °C.

The reductive amination reaction may be performed in an aprotic solvent such as 1,2-dichloroethane, in the presence of the reducing agent such as triacetoxyborohydride, for a period of time to ensure the completion of the reaction, which may typically be about 16 hours at room temperature.

The intermediate compound (I-4) is the starting compound for the compounds of the reaction schemes below. All variables are as defined in Formula (I), unless otherwise specified.

### Preparation of final compounds in which X² is a saturated or an unsaturated (C₁₋₈)-hydrocarbon radical.

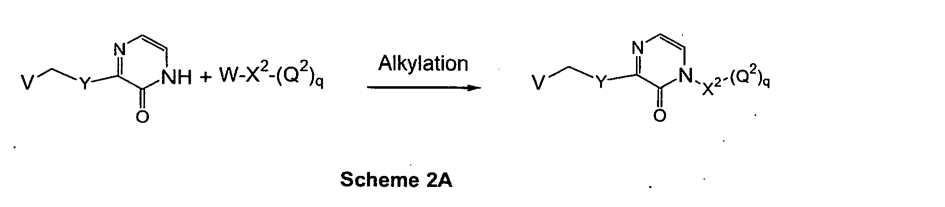

The W-radical in the compound W-X²-(Q²)_{q} is a leaving group, such as for instance Cl-, Br-, MeSO₂O- and p-MePhSO₂O- ; X² is a saturated or an unsaturated (C₁₋₈)-hydrocarbon radical and V, Y, Q² and p are defined as in Formula (I), . The alkylation reaction may be performed in an aprotic solvent, such as CH₃CN, DMF or THF in the presence of an inorganic base, such as K₂CO₃, Na₂CO₃, Cs₂CO₃, or an organic base such as TBD, PS-TBD, at a convenient temperature, either under conventional heating or microwave irradiation, for a period of time to ensure the completion of the reaction, which may typically be about 20 minutes at about 120° C under microwave irradiation.

### Preparation of final compounds in which X² is an phenyl-radical, or X² is a covalent bond and Q² is a heteroaryl-radical.

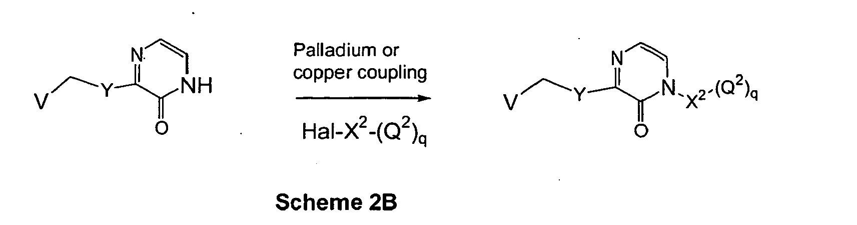

The Hal-radical in Hal-X²-(Q²)ₚ particularly represents a Br- or I-radical or a suitable equivalent radical such as B(OH)₂. X² is an optionally substituted phenyl ; or X² is a covalent bond and Q² is an optionally substituted heteroaryl. V, Y, Q² and p are defined as in Formula (I). The palladium coupling reaction is performed in an aprotic solvent such as toluene or dioxane, in the presence of a palladium catalyst such as Pd(AcO)₂ or Pd(dba)₃, in the presence of a suitable base such as Cs₂CO₃ or t-BuONa and of a ligand, such as BINAP or Xantphos, at a convenient temperature, either by conventional heating or under microwave irradiation, for a period of time to ensure the completion of the reaction. As an alternative, a copper coupling reaction may also be used to prepare the (hetero)aryl derivatives. The reaction is performed using an aprotic solvent, such as dioxane or DMF, in the presence of Cul, an inorganic base such K₃PO₄ and MeNH(CH₂)₂NHMe as a ligand, heating at a convenient temperature under traditional heating or microwave irradiation, for a period of time to ensure the completion of the reaction, which is typically about 25 minutes at about 175 °C under microwave irradiation.

### Preparation of final compounds in which X² is phenyl and Q² is NR₁Rᵤ

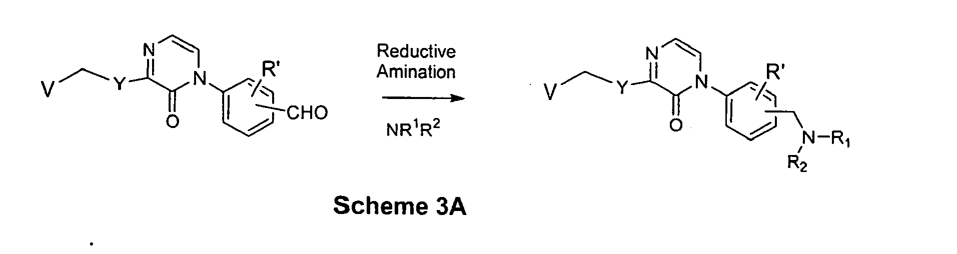

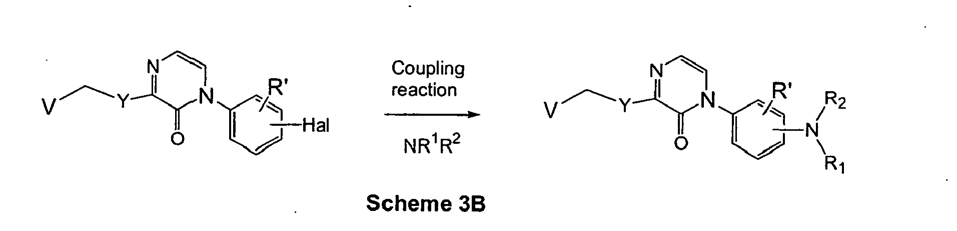

The transformations of different functional groups Q², present in the final compounds prepared by scheme 2B, into different functional groups present in other final compounds according to Formula (I), can be performed by synthesis methods well known by the person skilled in the art, such as reductive amination (Scheme 3A) or coupling reactions (Scheme 3B). V, Y, R¹ and R² are defined as in Formula (I). R' is an optional substitution of the phenyl-moiety as defined in Formula (I), such as for example oxo ; (C₁₋₃)alkyloxy ; halo ; cyano; nitro ; formyl ; hydroxy; amino ; trifluoromethyl ; mono- and di((C₁₋₃)alkyl)amino; carboxy; and thio.

### Preparation of final compounds : amides

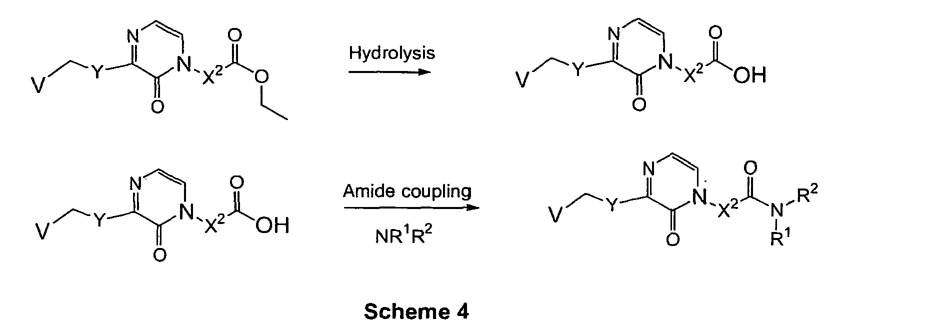

When the -X²-Q² -moiety (or part of it) is an amide derivative, preparation may be performed starting from the ester derivative, which was synthesized by either methods shown in Schemes 2A or 2B. Thus, basic hydrolysis of the ester group by standard and well known reaction techniques, in an aprotic solvent such as THF or dioxane, in the presence of an inorganic base, such as LiOH, KOH, or H₂O, at room temperature, for a period of time to ensure the completion of the reaction, yields the corresponding carboxylic acid derivative. Amide coupling of this carboxylic acid with different amines is performed using standard reaction conditions, for example, using HATU as coupling agent, in an aprotic solvent such as THF, DMF, CH₂Cl₂ (DCM), at room temperature, for a period of time to ensure the completion of the reaction. V, Y, X², R¹ and R² are defined as in Formula (I).

### Preparation of final compounds : modified amines

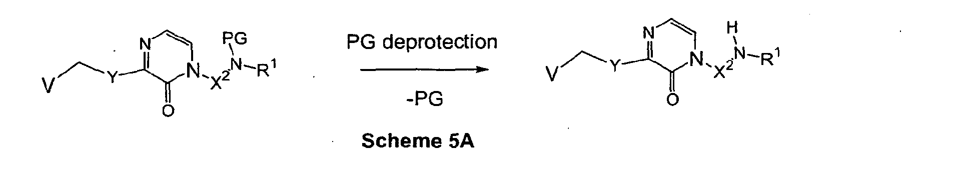

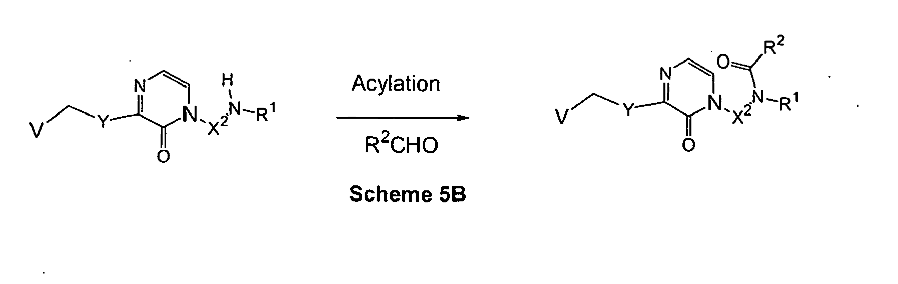

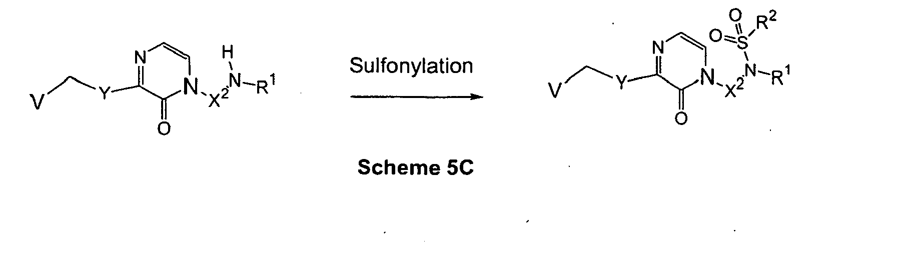

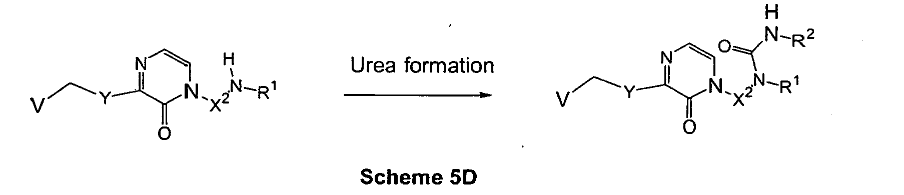

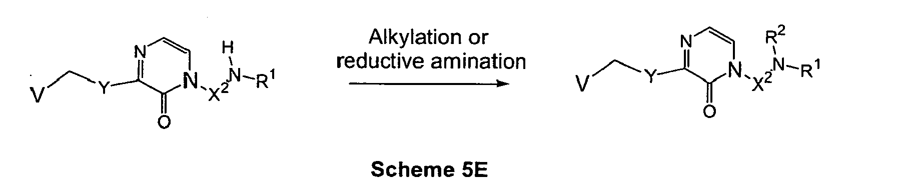

When amino group is protected with a protecting group, deprotection reaction may be carried out by synthetic methods well known to the person skilled in the art. Transformations of the amino group of Q², present in the intermediate and final compounds, into different amino derivatives of Q², present in other final compounds according to Formula (I) may be performed by synthetic methods well known by the person skilled in the art, such as acylation, sulfonylation, urea formation, alkylation or reductive amination reactions. Schemes 5A-E show a general overview of such chemical transformations. V, Y, X², R¹ and R² are defined as in Formula (I).

### Preparation of final compounds : Compounds substituted on carbon-6 of the pyrazinone-core.

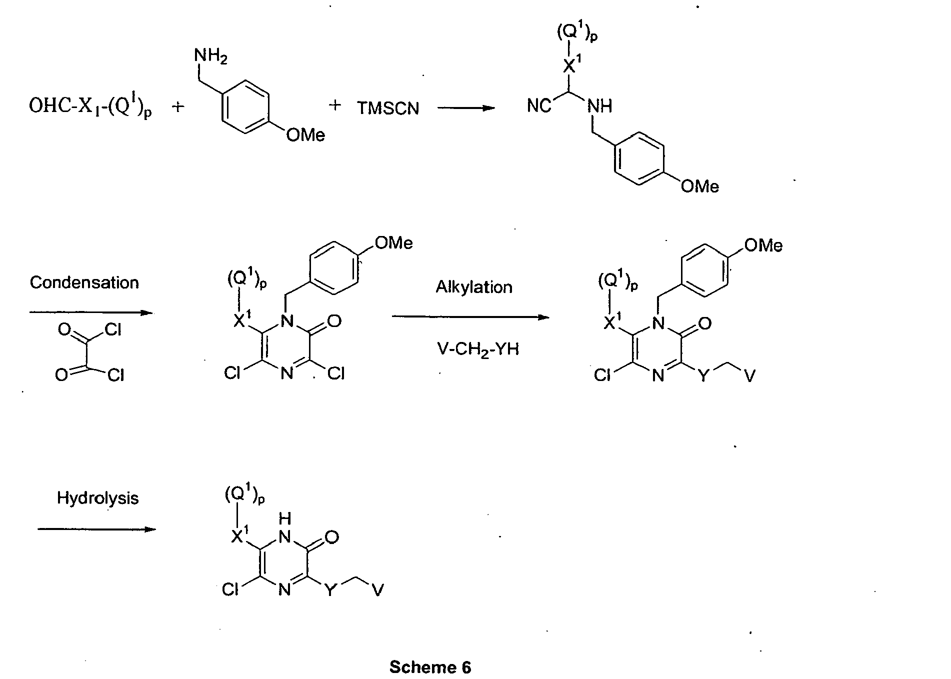

Reductive amination of the required starting material shown in the scheme was performed in the presence of trimethylsilyl cyanide (TMSCN), in an aprotic solvent, such as dichloromethane, and in the presence of a reducing agent such as Ti(iprO)₄, at a convenient temperature, for a period of time to ensure the completion of the reaction, typically 16 hours at room temperature.

Cyclization of the intermediates was achieved by reaction with oxalyl chloride in an aprotic solvent such as dichloroethane, at a convenient temperature, for a period of time to ensure the completion of the reaction, typically 60 hours at room temperature.

The alkylation reaction with intermediate V-CH₂-YH was performed in an aprotic solvent such as 1,2-dichloroethane, acetonitrile or DMF, in the presence of an inorganic base, such as K₂CO₃, Na₂CO₃, NaOH, KOH, at a convenient temperature, either by conventional heating or under microwave irradiation, for a period of time to ensure the completion of the reaction, typcially 30 minutes at 130 °C under microwave irradiation.

Hydrolysis was performed in acidic media, such as trifluoroacetic acid, at a convenient temperature, either by conventional heating or under microwave irradation, for a period of time to ensure the completion of the reaction, typically 15 minutes at 140 °C under microwave irradiation. V, Y, X¹ and p are defined as in Formula (I).

The transformations of different functional groups Q¹, present in the final compounds prepared by scheme 6, into different functional groups present in other final compounds according to Formula (I), can be performed by synthesis methods well known by the person skilled in the art.

### Pharmacology

The compounds according to the invention, in particular compounds according to Formula (I), the pharmaceutically acceptable acid or base addition salts thereof, a stereochemically isomeric form thereof, an *N-*oxide form thereof or a quaternary ammonium salt thereof, have surprisingly been shown to have a binding affinity towards α₂-adrenergic receptor, in particular towards α_{2C}-adrenergic receptor, in particular as an antagonist.

In view of their above mentioned potency, the compounds according to the invention are suitable for the prevention and/or treatment of diseases where antagonism of the α₂-adrenergic receptor, in particular antagonism of the α_{2C}-adrenergic receptor is of therapeutic use. In particular, the compounds according to the invention may be suitable for treatment and/or prophylaxis in the following diseases:
- Central nervous system disorders, including :
- Mood disorders, including particularly major depressive disorder, depression with or without psychotic features, catatonic features, melancholic features, atypical features of postpartum onset and, in the case of recurrent episodes, with or without seasonal pattern, dysthymic disorder, bipolar I disorder, bipolar II disorder, cyclothymic disorder, recurrent brief depressive disorder, mixed affective disorder, bipolar disorder not otherwise specified, mood disorder due to a general medical condition, substance-induced mood disorder, mood disorder not otherwise specified, seasonal affective disorder and premenstrual dysphoric disorders.
- Anxiety disorders, including panic attack, agoraphobia, panic disorder without agoraphobia, agoraphobia without history of panic disorder, specific phobia, social phobia, obsessive-compulsive disorder, posttraumatic stress disorder, acute stress disorder, generalized anxiety disorder, anxiety disorder due to a general medical condition, substance-induced anxiety disorder and anxiety disorder not otherwise specified.
- Stress-related disorders associated with depression and/or anxiety, including acute stress reaction, adjustment disorders (brief depressive reaction, prolonged depressive reaction, mixed anxiety and depressive reaction, adjustment disorder with predominant disturbance of other emotions, adjustment disorder with predominant disturbance of conduct, adjustment disorder with mixed disturbance of emotions and conduct, adjustment disorders with other specified predominant symptoms) and other reactions to severe stress.
- Dementia, amnesic disorders and cognitive disorders not otherwise specified, especially dementia caused by degenerative disorders, lesions, trauma, infections, vascular disorders, toxins, anoxia, vitamin deficiency or endocrinic disorders, or amnesic disorders caused by alcohol or other causes of thiamine deficiency, bilateral temporal lobe damage due to Herpes simplex encephalitis and other limbic encephalitis, neuronal loss secondary to anoxia / hypoglycaemia / severe convulsions and surgery, degenerative disorders, vascular disorders or pathology around ventricle III.
- Cognitive disorders, in particular due to cognitive impairment resulting from other medical conditions.
- Personality disorders, including paranoid personality disorder, schizoid personality disorder, schizotypical personality disorder, antisocial personality disorder, borderline personality disorder, histrionic personality disorder, narcissistic personality disorder, avoidant personality disorder, dependent personality disorder, obsessive-compulsive personality disorder and personality disorder not otherwise specified.
- Schizoaffective disorders resulting from various causes, including schizoaffective disorders of the manic type, of the depressive type, of mixed type, paranoid, disorganized, catatonic, undifferentiated and residual schizophrenia, schizophreniform disorder, schizoaffective disorder, delusional disorder, brief psychotic disorder, shared psychotic disorder, substance-induced psychotic disorder and psychotic disorder not otherwise specified.
- Akinesia, akinetic-rigid syndromes, dyskinesia and medication-induced parkinsonism, Gilles de la Tourette syndrome and its symptoms, tremor, chorea, myoclonus, tics and dystonia.
- Attention-deficit / hyperactivity disorder (ADHD).
- Parkinson's disease, drug-induced Parkinsonism, post-encephalitic Parkinsonism, progressive supranuclear palsy, multiple system atrophy, corticobasal degeneration, parkinsonism-ALS dementia complex and basal ganglia calcification.
- Dementia of the Alzheimer's type, with early or late onset, with depressed mood.
- Behavioural disturbances and conduct disorders in dementia and the mentally retarded, including restlessness and agitation.
- Extra-pyramidal movement disorders.
- Down's syndrome.
- Akathisia.
- Eating Disorders, including anorexia nervosa, atypical anorexia nervosa, bulimia nervosa, atypical bulimia nervosa, overeating associated with other psychological disturbances, vomiting associated with other psychological disturbances and non-specified eating disorders.
- AIDS-associated dementia.
- Chronic pain conditions, including neuropathic pain, inflammatory pain, cancer pain and post-operative pain following surgery, including dental surgery. These indications might also include acute pain, skeletal muscle pain, low back pain, upper extremity pain, fibromyalgia and myofascial pain syndromes, orofascial pain, abdominal pain, phantom pain, tic douloureux and atypical face pain, nerve root damage and arachnoiditis, geriatric pain, central pain and inflammatory pain.
- Neurodegenerative diseases, including Alzheimer's disease, Huntington's chorea, Creutzfeld-Jacob disease, Pick's disease, demyelinating disorders, such as multiple sclerosis and ALS, other neuropathies and neuralgia, multiple sclerosis, amyotropical lateral sclerosis, stroke and head trauma.
- Addiction disorders, including:
- Substance dependence or abuse with or without physiological dependence, particularly where the substance is alcohol, amphetamines, amphetamine-like substances, caffeine, cannabis, cocaine, hallucinogens, inhalants, nicotine, opioids, phencyclidine, phencyclidine-like compounds, sedative-hypnotics, benzodiazepines and/or other substances, particularly useful for treating withdrawal from the above substances and alcohol withdrawal delirium.
- Mood disorders induced particularly by alcohol, amphetamines, caffeine, cannabis, cocaine, hallucinogens, inhalants, nicotine, opioids, phencyclidine, sedatives, hypnotics, anxiolitics and other substances.
- Anxiety disorders induced particularly by alcohol, amphetamines, caffeine, cannabis, cocaine, hallucinogens, inhalants, nicotine, opioids, phencyclidine, sedatives, hypnotics, anxiolitics and other substances and adjustment disorders with anxiety.
- Smoking cessation.
- Body weight control, including obesity.
- Sleep disorders and disturbances, including:
- Dyssomnias and/or parasomnias as primary sleep disorders, sleep disorders related to another mental disorder, sleep disorder due to a general medical condition and substance-induced sleep disorder.
- Circadian rhythms disorders.
- Improving the quality of sleep.
- Sexual dysfunction, including sexual desire disorders, sexual arousal disorders, orgasmic disorders, sexual pain disorders, sexual dysfunction due to a general medical condition, substance-induced sexual dysfunction and sexual dysfunction not otherwise specified.

The invention therefore relates to a compound according to the general Formula (I), a pharmaceutically acceptable acid or base addition salt thereof, a stereochemically isomeric form thereof, an *N-*oxide form thereof or a quaternary ammonium salt thereof, for use as a medicine.

The invention also relates to the use of a compound according to the invention for the preparation of a medicament for the prevention and/or treatment of central nervous system disorders, mood disorders, anxiety disorders, stress-related disorders associated with depression and/or anxiety, cognitive disorders, personality disorders, schizoaffective disorders, Parkinson's disease, dementia of the Alzheimer's type, chronic pain conditions, neurodegenerative diseases, addiction disorders, mood disorders and sexual dysfunction.

The compounds according to the invention may be co-administered as add-on treatment and/or prophylaxis in the above listed diseases in combination with antidepressants, anxiolytics and/or antipsychotics which are currently available or in development or which will become available in the future, in particular to improve efficacy and/or onset of action. It will be appreciated that the compounds of the present invention and the other agents may be present as a combined preparation for simultaneous, separate or sequential use for the prevention and/or treatment of depression and/or anxiety. Such combined preparations may be, for example, in the form of a twin pack. It will also be appreciated that the compounds of the present invention and the other agents may be administered as separate pharmaceutical compositions, either simultaneously or sequentially.

The invention therefore relates to the use of the compounds according to the invention as an add-on treatment in combination with one or more other compounds selected from the group of antidepressants, anxiolytics and antipsychotics.

Suitable classes of antidepressant agents include norepinephrine reuptake inhibitors, selective serotonin reuptake inhibitors (SSRI's), monoamine oxidase inhibitors (MAOI's), reversible inhibitors of monoamine oxidase (RIMA's), serotonin and noradrenaline reuptake inhibitors (SNRI's), noradrenergic and specific serotonergic antidepressants (NaSSA's), corticotropin releasing factor (CRF) antagonists, α-adrenoreceptor antagonists and atypical antidepressants.

Suitable examples of norepinephrine reuptake inhibitors include amitriptyline, clomipramine, doxepin, imipramine, trimipramine, amoxapine, desipramine, maprotiline, nortriptyline, protriptyline, reboxetine and pharmaceutically acceptable salts thereof.

Suitable examples of selective serotonin reuptake inhibitors include fluoxetine, fluvoxamine, paroxetine, sertraline and pharmaceutically acceptable salts thereof.

Suitable examples of monoamine oxidase inhibitors include isocarboxazid, phenelzine, tranylcypromine, selegiline and pharmaceutically acceptable salts thereof.

Suitable examples of reversible inhibitors of monoamine oxidase include moclobemide and pharmaceutically acceptable salts thereof.

Suitable examples of serotonin and noradrenaline reuptake inhibitors include venlafaxine and pharmaceutically acceptable salts thereof.

Suitable atypical antidepressants include bupropion, lithium, nefazodone, trazodone, viloxazine, sibutramine and pharmaceutically acceptable salts thereof.

Other suitable antidepressants include adinazolam, alaproclate, amineptine, amitriptyline/chlordiazepoxide combination, atipamezole, azamianserin, bazinaprine, befuraline, bifemelane, binodaline, bipenamol, brofaromine, bupropion, caroxazone, cericlamine, cianopramine, cimoxatone, citalopram, clemeprol, clovoxamine, dazepinil, deanol, demexiptiline, dibenzepin, dothiepin, droxidopa, enefexine, estazolam, etoperidone, femoxetine, fengabine, fezolamine, fluotracen, idazoxan, indalpine, indeloxazine, iprindole, levoprotiline, litoxetine, lofepramine, medifoxamine, metapramine, metralindole, mianserin, milnacipran, minaprine, mirtazapine, monirelin, nebracetam, nefopam, nialamide, nomifensine, norfluoxetine, orotirelin, oxaflozane, pinazepam, pirlindone, pizotyline, ritanserin, rolipram, sercloremine, setiptiline, sibutramine, sulbutiamine, sulpiride, teniloxazine, thozalinone, thymoliberin, tianeptine, tiflucarbine, tofenacin, tofisopam, toloxatone, tomoxetine, veralipride, viqualine, zimelidine and zometapine and pharmaceutically acceptable salts thereof, and St. John's wort herb, or *Hypericum perforatum,* or extracts thereof.

Suitable classes of anti-anxiety agents include benzodiazepines and 5-HT_{1A} receptor agonists or antagonists, especially 5-HT_{1A} partial agonists, corticotropin releasing factor (CRF) antagonists, compounds having muscarinic cholinergic activity and compounds acting on ion channels. In addition to benzodiazepines, other suitable classes of anti-anxiety agents are nonbenzodiazepine sedative-hypnotic drugxs such as zolpidem; mood-stabilizing drugs such as clobazam, gabapentin, lamotrigine, loreclezole, oxcarbamazepine, stiripentol and vigabatrin; and barbiturates.

Suitable antipsychotic agents are selected from the group consisting of acetophenazine, in particular the maleate salt; alentemol, in particular the hydrobromide salt; alpertine; azaperone; batelapine, in particular the maleate salt; benperidol; benzindopyrine, in particular the hydrochloride salt; brofoxine; bromperidol; butaclamol, in particular the hydrochloride salt; butaperazine; carphenazine, in particular the maleate salt; carvotroline, in particular the hydrochloride salt; chlorpromazine; chlorprothixene; cinperene; cintriamide; clomacran, in particular the phosphate salt; clopenthixol; clopimozide; clopipazan, in particular the mesylate salt; cloroperone, in particular the hydrochloride salt; clothiapine; clothixamide, in particular the maleate salt; clozapine; cyclophenazine, in particular the hydrochloride salt; droperidol; etazolate, in particular the hydrochloride salt; fenimide; flucindole; flumezapine; fluphenazine, in particular the decanoate, enanthate and/or hydrochloride salts; fluspiperone; fluspirilene; flutroline; gevotroline, in particular the hydrochloride salt; halopemide; haloperidol; iloperidone; imidoline, in particular the hydrochloride salt; lenperone; loxapine; mazapertine, in particular the succinate salt; mesoridazine; metiapine; milenperone; milipertine; molindone, in particular the hydrochloride salt; naranol, in particular the hydrochloride salt; neflumozide, in particular the hydrochloride salt; ocaperidone; olanzapine; oxiperomide; penfluridol; pentiapine, in particular the maleate salt; perphenazine; pimozide; pinoxepin, in particular the hydrochloride salt; pipamperone; piperacetazine; pipotiazine, in particular the palmitate salt; piquindone, in particular the hydrochloride salt; prochlorperazine, in particular the edisylate salt; prochlorperazine, in particular the maleate salt; promazine, in particular the hydrochloride salt; quetiapine; remoxipride; risperidone; rimcazol, in particular the hydrochloride salt; seperidol, in particular the hydrochloride salt; sertindole; setoperone; spiperone; sulpiride; thioridazine; thiothixene; thorazine; tioperidone, in particular the hydrochloride salt; tiospirone, in particular the hydrochloride salt; trifluoperazine, in particular the hydrochloride salt; trifluperidol; triflupromazine; ziprasidone, in particular the hydrochloride salt; and mixtures thereof.

The compounds according to the invention surprisingly also show a high 5-HT-reuptake inhibition activity and are therefore very well suited for use in the treatment and/or prophylaxis of depression. It is thought that a 5-HT reuptake inhibitor with associated α₂-adrenoceptor antagonistic activity might be a new type of antidepressant, with a dual action on the central noradrenergic and serotonergic neuronal systems. The immediate effect on monoamine release of autoreceptor blockade may accelerate the onset of action of such a compound, compared to currently available drugs that require desensitization of the autoreceptors involved in the feedback mechanism in order to become fully effective. In addition, α_{2c}-adrenoceptor antagonism improves sexual function as shown by treatment with the α_{2c}-adrenoceptor antagonist yohimbine, thereby potentially reducing one of the side effects related to 5-HT uptake inhibition and enhancement of NEergic neurotransmission improves social function more effectively than SSRIs (J. Ignacio Andrés et al., J. Med. Chem. (2005), Vol. 48, 2054-2071).

### Pharmaceutical compositions

The invention also relates to a pharmaceutical composition comprising a pharmaceutically acceptable carrier or diluent and, as active ingredient, a therapeutically effective amount of a compound according to the invention, in particular a compound according to Formula (I), a pharmaceutically acceptable acid or base addition salt thereof, a stereochemically isomeric form thereof, an *N-*oxide form thereof or a quaternary ammonium salt thereof.

The compounds according to the invention, in particular the compounds according to Formula (I), the pharmaceutically acceptable acid or base addition salt thereof, a stereochemically isomeric form thereof, an *N-*oxide form thereof or a quaternary ammonium salt thereof , or any subgroup or combination thereof may be formulated into various pharmaceutical forms for administration purposes. As appropriate compositions there may be cited all compositions usually employed for systemically administering drugs.

To prepare the pharmaceutical compositions of this invention, an effective amount of the particular compound, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration orally, rectally, percutaneously, by parenteral injection or by inhalation. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules and tablets. Because of their ease in administration, tablets and capsules represent the most advantageous oral dosage unit forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations that are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a transdermal patch, as a spot-on, as an ointment.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier examples of such unit dosage forms are tablets (including scored or coated tablets), capsules, pills, powder packets, wafers, suppositories, injectable solutions or suspensions and the like, and segregated multiples thereof. Since the compounds according to the invention are potent orally administrable dopamine antagonists, pharmaceutical compositions comprising said compounds for administration orally are especially advantageous.

The invention also relates to a pharmaceutical composition comprising the compounds according to the invention and one or more other compounds selected from the group of antidepressants, anxiolytics and antipsychotics as well as to the use of such a composition for the manufacture of a medicament, in particular to improve efficacy and/or onset of action in the treatment of depression and/or anxiety.

The following examples are intended to illustrate but not to limit the scope of the present invention.

### Experimental part

Hereinafter, "THF" means tetrahydrofuran ; "DMF" means *N,N*-dimethylformamide ; "EtOAc" means ethyl acetate ; "DCE" means 1 ,2-dichloroethane ; "DMSO" means dimethylsulfoxide ; "TMSCN" means trimethylsilyl cyanide ; "Ti(iPrO)₄" means titanium(4+) salt 2-propanol ; "TFA" means trifluoro acetic acid ; "DCM" means dichloromethane ; "HATU" means O-(7-Azobenzotriazol-1-yl)-1,1,3,3-tetramethyluronium hexafluorophosphate ; "DIBAL" means hydro-bis(2-methylpropyl)aluminum ; "DIPEA" means diisopropylethylamine; "DIEA" means diethylamine ; "PS-TBD" is polymer-supported TBD and "PS-NCO" means polymer-supported isocyanate.

Microwave assisted reactions were performed in a single-mode reactor: Emrys™ Optimizer microwave reactor (Personal Chemistry A.B., currently Biotage). Description of the instrument can be found in www.personalchemistry.com. And in a multimode reactor: MicroSYNTH Labstation (Milestone, Inc.). Description of the instrument can be found in www.milestonesci.com.

### A. Preparation of the intermediate compounds

### A1. Preparation of intermediate compound 3

### a) Preparation of intermediate compound 1

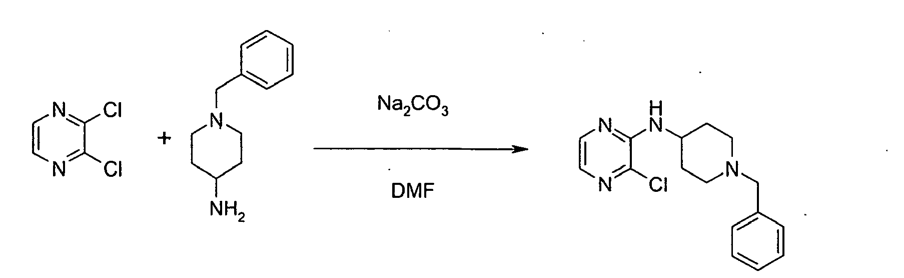

2,3-Dichloropyrazine (10 g, 62.12 mmol) and 1-(phenylmethyl)-4-piperidinamine (13.73 mL, 67.12 mmol) were dissolved in DMF (60 ml). Then Na₂CO₃ (10.09 g, 114.10 mmol) was added. The reaction was stirred at 130 °C for 16 hours. The solid was filtered off, washed with AcOEt and the solvent was evaporated till dryness. The product was dissolved in AcOEt, washed with H₂O and brine, dried with MgSO₄ and evaporated under vacuum. The product was used without any further purification yielding 15 g of the desired intermediate compound 1 (74 %).

### b) Preparation of intermediate compound 2

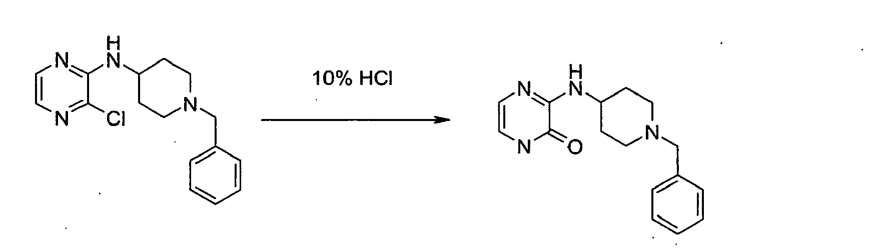

Intermediate compound 1 (7 g, 23.11 mmol) was dissolved in HCl (70 ml; 10 %) and heated in a sealed tube at 110 °C for 16 hours. A light brown solid was precipitated, it was filtered off, washed with water and dried under vacuum yielding 4.57 g of the desired intermediate compound 2 (70 %).

### c) Preparation of intermediate compound 3

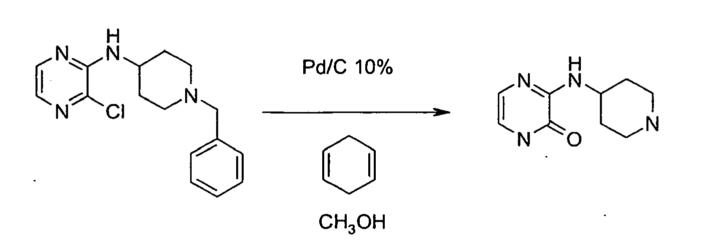

Intermediate compound 2 (4.17 g, 14.66 mmol) was dissolved in CH₃O (62 mL), then Pd/C (4.17 g; 10 %) and 1,4-cyclohexadiene (27.96 mL, 293.2 mmol) were added. The reaction was heated in a sealed tube at 65°C for 4 hours. The reaction was filtered over celite and the solvent was evaporated till dryness yielding 2.69 g of the desired intermediate compound 3 (94 %).

### A2. Preparation of intermediate compound 5

### a) Preparation of intermediate compound 4

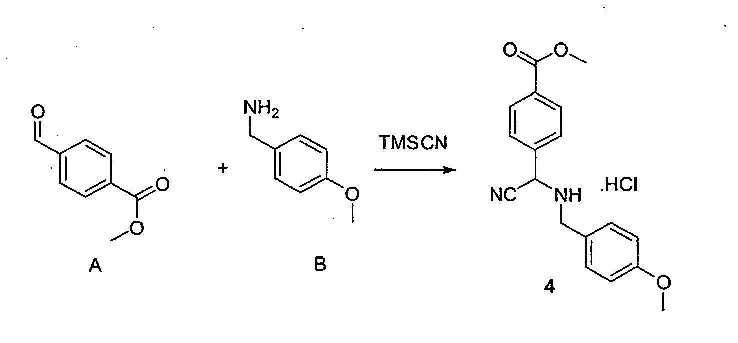

To a mixture of 4-formylbenzoic acid methyl ester (31.0 mmol) and 4-methoxybenzenemethanamine (34.1 mmol) in DCM (150 mL) was added Ti-(*i*-PrOH)₄ (3.1 mmol). The reaction was stirred for 2 hours at room temperature ; TMSCN (77.5 mmol) was added and the reaction was stirred 24 hours at room temperature. The solvent was evaporated and the oil was dissolved in Et₂O (100 mL) ; then a solution of *i*-PrOH/HCl 6 N (10 mL) was added. The precipitate was filtered off and washed with cold Et₂O to yield 5.2 g of intermediate compound 4 as a white solid (56 %).

### b) Preparation of intermediate compound 5

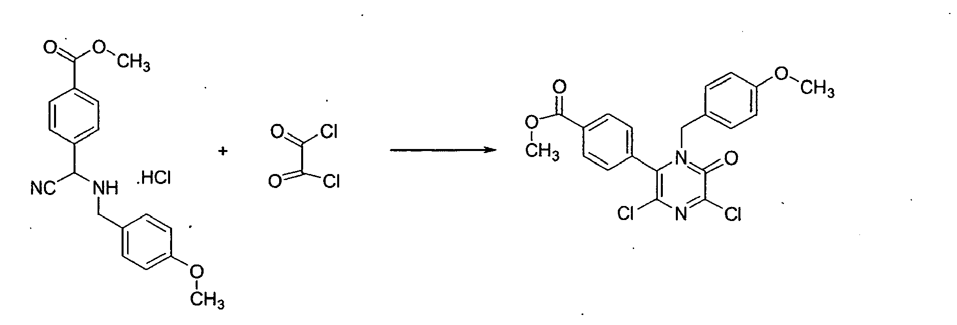

To a mixture of intermediate compound 4 (22 mmol) and DCE (200 mL), ethanedioyl dichloride (66.3 mmol) was added. The reaction was stirred for 4 days at room temperature. The crude was evaporated to dryness, to afford 6 g of intermediate compound 5 as a yellow oil (60 %). The crude was used in the next step without purification.

### A3. Preparation of intermediate compound 6

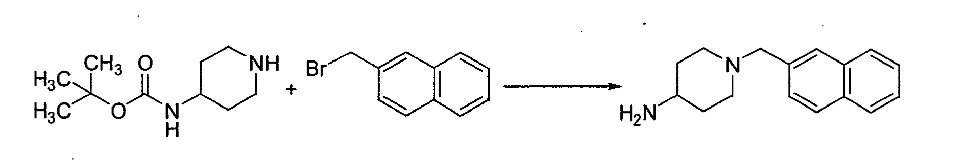

A mixture of 4-piperidinylcarbamic acid 1,1-dimethylethyl ester (25 mmol), 2-(bromomethyl)-naphtalene (25 mmol) and K₂CO₃ (52 mmol) in CH₃CN (50 ml) was heated to reflux for 3 hours. The organic phase was filtered and the solvent was evaporated to obtain a white solid. The solid was treated with a solution of DCM/TFA 8:2 and stirred for 1 hour. The organic phase was evaporated to obtained 5 g (83 %) of a white solid of intermediate compound 6.

### B. Preparation of final compounds

Final compounds were prepared according to the following three summary schemes.

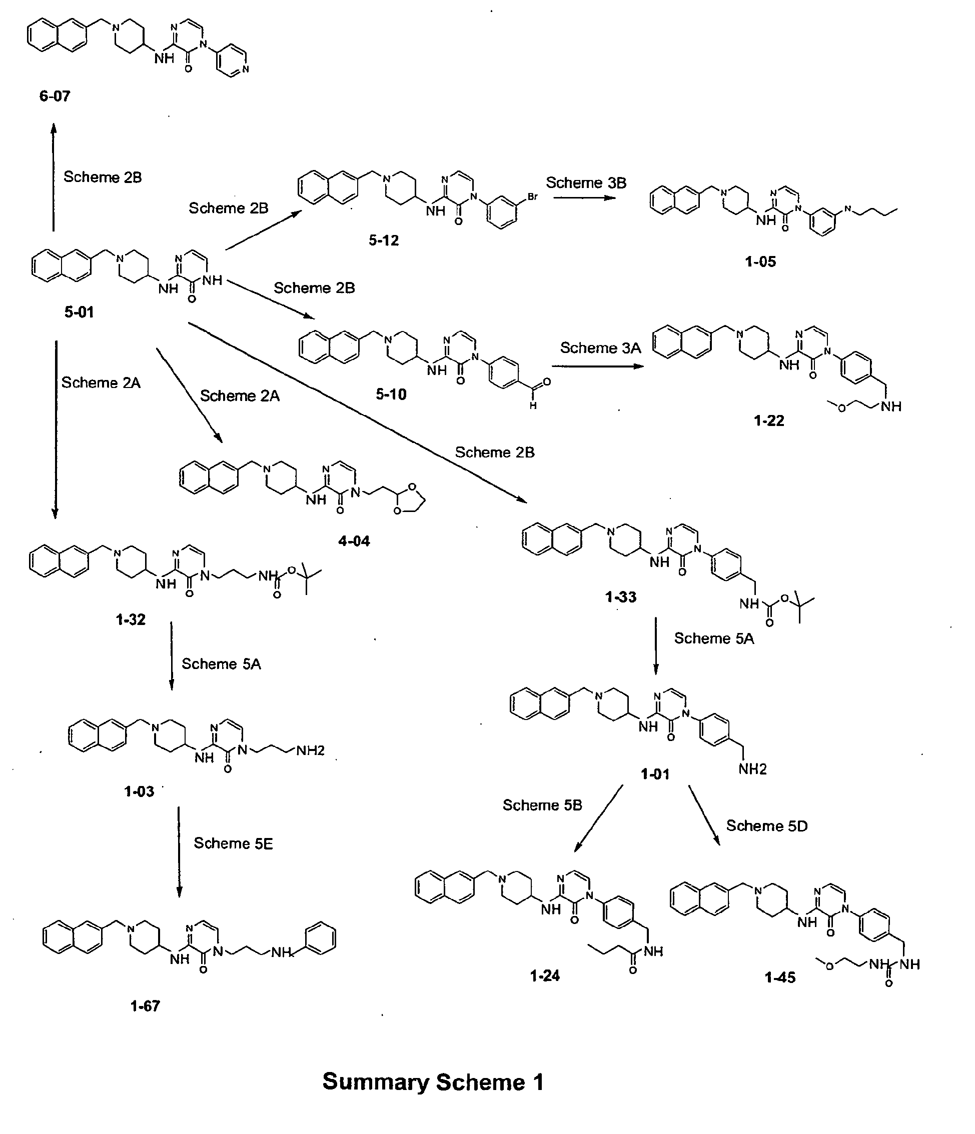

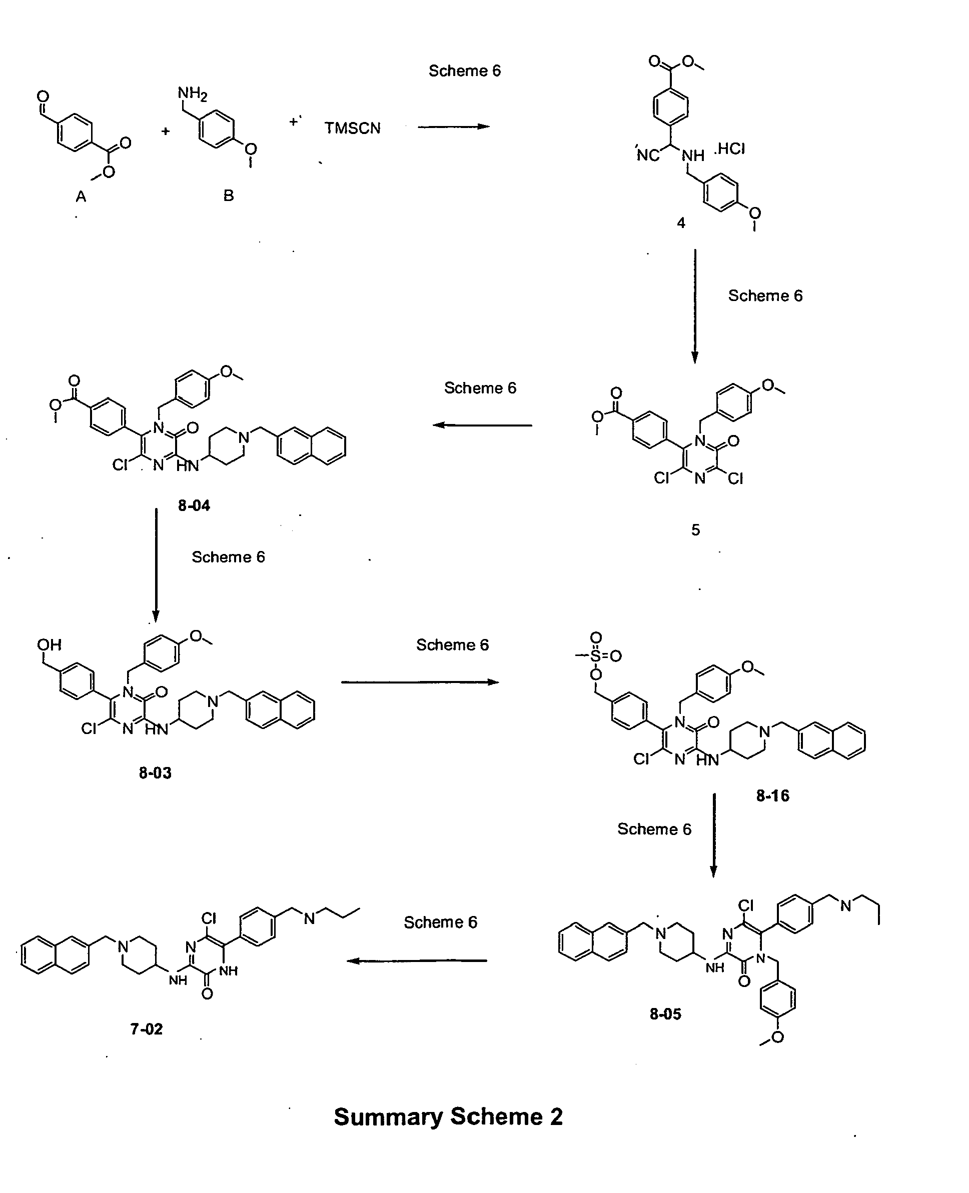

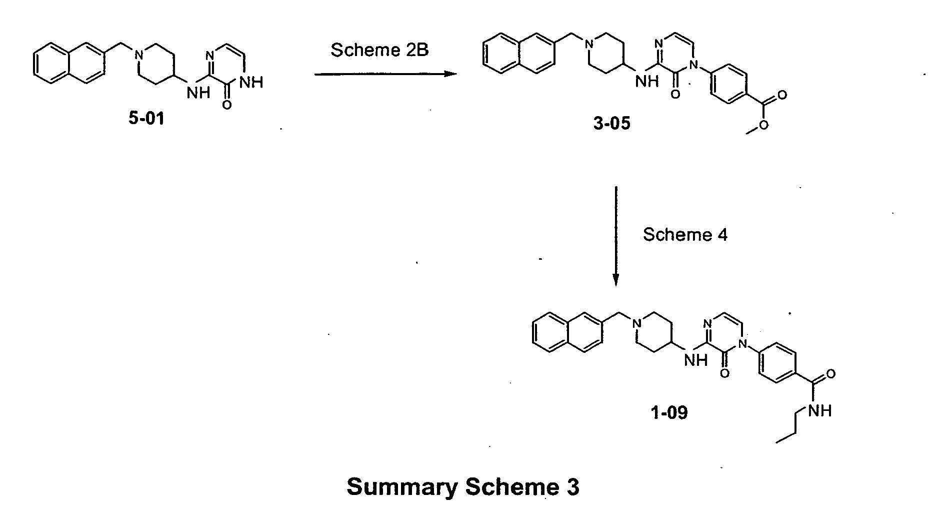

### B1. Preparation of final compound 5-01

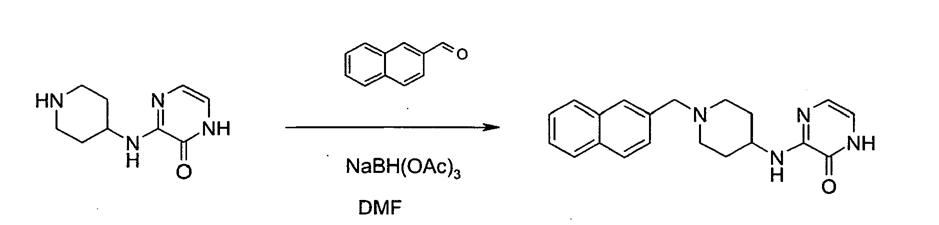

Intermediate compound 3 (2 g, 10.269 mmol) was dissolved in DMF (60 ml), then naphthalene-2-carbaldehyde (4.82 g, 30.87 mmol) and NaBH(OAc)₃ (3.2 g, 15.45 mmol) was added. The reaction was stirred at room temperature for 16 hours. The solvent was removed, the product was dissolved in EtOAc, washed with NaHCO₃ and brine and dried with MgSO₄. The solvent was evaporated till dryness and the product was purified by open chromatography using DCM/CH₃OH 9/1 as eluent, yielding 3 g of the desired final compound **5-01** (87%).

### B2. Preparation of final compound 1-67

### a) Preparation of final compound 1-32

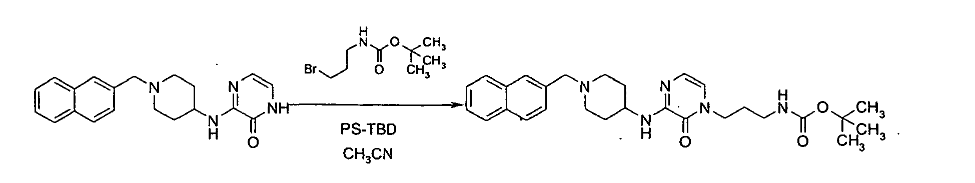

Final compound 5-01 (25 mg, 0.074 mmol), (3-bromopropyl)-carbamic acid 1,1-dimethylethyl ester (0.112 mmol), and PS-TBD (76 mg, 0.222 mmol) were suspended in CH₃CN (1 ml). The reaction was heated in the microwave at 130 °C for 20 minutes. The resin was filtered off, and the filtrate was concentrated under vacuum. The resulting crude was purified by HPLC yielding 0.029 g of the purified final compound **1-32** (80 %).

### b) Preparation of final compound 1-03

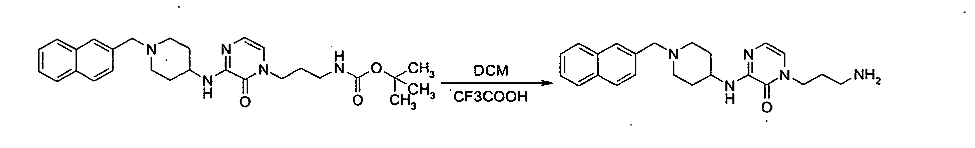

Final compound 1-32 (450 mg, 0.915 mmol) was dissolved in DCM (6 ml). Then TFA was added (6 ml). The reaction was stirred at room temperature for 2h. The solvent was concentrated under vacuum. The resulting crude was dissolved in EtOAc and washed with an aqueous solution of saturated K₂CO₃, then with NaCl (saturated), and dried with MgSO₄. The solvent was concentrated till dryness to yield 394 mg of the purified final compound **1-03** (97 %).

### c) Preparation of final compound 1-67

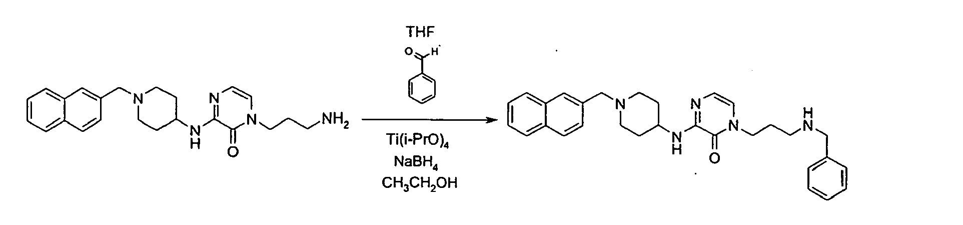

Final compound 1-03 (77 mg, 0.196 mmol) was dissolved in dry THF (3 ml). Benzaldehyde (30 µl, 0.295 mmol) and Ti(i-PrO)₄ (112 mg, 0.392 mmol) were added. The reaction was stirred at room temperature for 16 hours. Then NaBH₄ (23 mg, 0.59 mmol) and CH₃CH₂O (1 ml) were added, stirring at room temperature for 8 hours. Then NH₃ (aqueous solution) was added, a precipitate appears which was filtered over celite and washed with Et₂O. The organic layer was separated and the remaining aqueous layer was extracted with Et₂O. The combined organic layers were treated with HCl (2N). The aqueous phase was then treated with NaOH (2N) to PH 10-12, and washed with EtOAc (3 x 10ml). The organic layer was washed with brine, dried (MgSO₄) and evaporated till dryness. The resulting crude was purified by open flash chromatography using DCM/(CH₃OHlNH₃) 9:1 to yield 30 mg of the purified final compound **1-67** (32 %).

### B3. Preparation of final compound 4-04

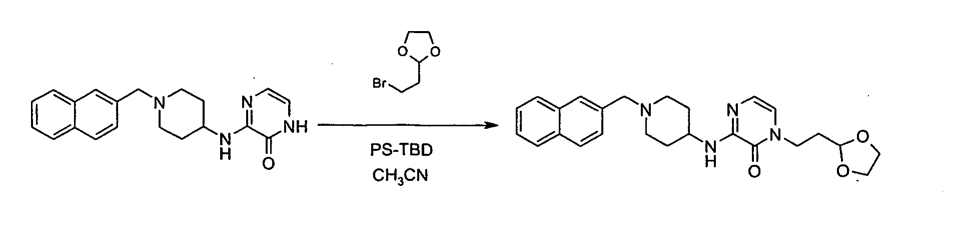

Final compound 5-01 (25 mg, 0.074 mmol) was dissolved in dry CH₃CN (1 ml), and then 2-(2-bromoethyl)-1,3-dioxolane (20.27 mg, 0.112 mmol) and PS-TBD (76 mg, 0.222 mmol) were added. The reaction mixture was heated in the microwave at 130°C for 20 minutes. The resin was filtered off, and the filtrate concentrated under vacuum. The resulting crude was purified by HPLC yielding 22 mg of the purified final compound **4-04** (68 %).

### B4. Preparation of final compounds 1-24 and 1-45

### a) Preparation of final compound 1-33

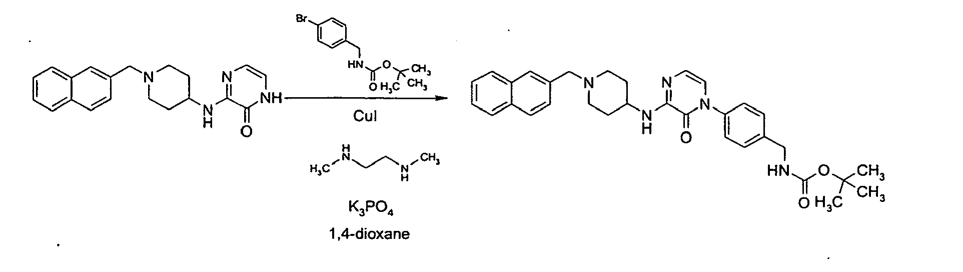

Final compound 5-01 (1g, 2.99 mmol) was dissolved in 1,4-dioxane (15 ml). [(4-bromophenyl)methyl]-carbamic acid 1,1-dimethylethyl ester (1.02 g, 3.59 mmol), and Cul (114 mg, 0.598 mmol) were added, and the mixture was strirred for 1 minute. Subsequently *N,N*'-dimethyl-1,2-ethanediamine (127 µl, 1.19 mmol) was added, and the mixture was stirred for 5 minutes. Finally K₃PO₄ (1.26 g, 5. 98 mmol) was added. The mixture was heated at 110°C in a seal tube for 16 hours. The reaction mixture was filtered over celite, washed with DCM and the solvent was evaporated till dryness. The crude product was dissolved in DCM, washed with NH₄Cl and brine and dried (MgSO₄). The solvent was evaporated under vacuum. The resulting crude was purified by open flash chromatography in silicagel inDCM/CH₃OH (9.5/0.5) to yield 1.53 g of the final compound **1-33** (95 %).

### b) Preparation of final compound 1-01

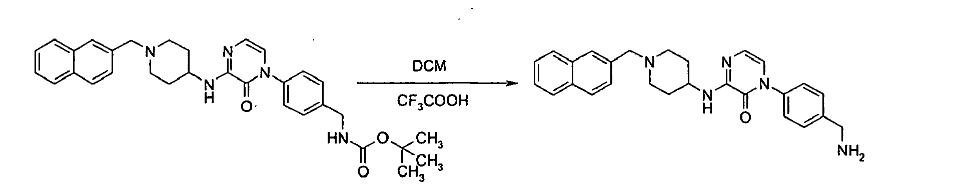

Final compound 1-33 (1.41g, 2.61 mmol) was dissolved in DCM (25 ml), then TFA (25 ml) was added. The mixture was stirred at room temperature for 16 hours. The solvent was removed and the resulting crude was dissolved in EtOAc and washed with K₂CO₃ (aqueous saturated) and brine, and was then dried (MgS04). The organic layer was evaporated under vacuum, and the crude residue used without any further purification, yielding 980 mg of the final compound **1-01** (86 %).

### c) Preparation of final compound 1-24

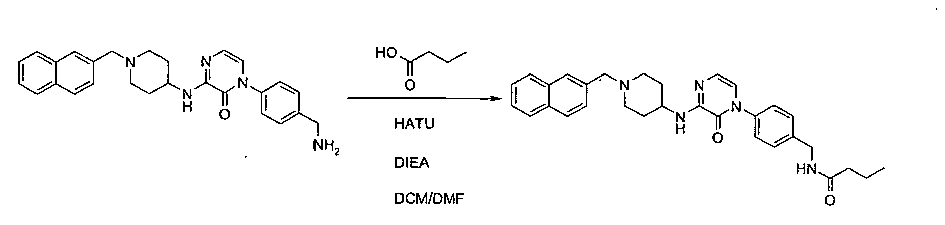

Butanoic acid (51,3 µl, 0.559 mmol), HATU (425 mg, 1,11 mmol) and DIEA (292.11 µl, 1.67 mmol) were dissolved in DCM/DMF (80 ml, 2/1). Then final compound 1-01 (270 mg, 0.615 mmol) was added. The reaction mixture was stirred at room temperature for 16 hours. The solvent was removed under vacuum. The crude was dissolved in DCM, washed with NH₄Cl, and brine, then dried (MgS04). The solvent was concentrated under reduced presure and the resulting crude purified by open chromatography in SiO₂ with DCM/(CH₃OH/NH₃) (9.5/0.5) to yield 95 mg of the final compound **1**-**24** (33%).

### d) Preparation of of final compound 1-45

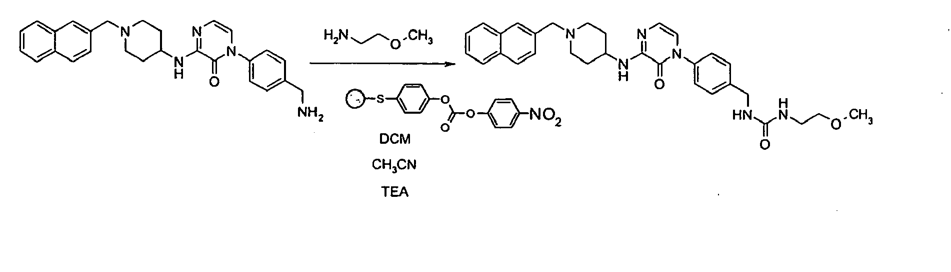

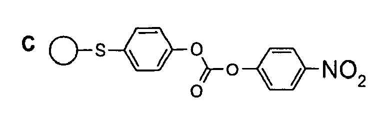

(111 mg, 0.135 mmol) was suspended in DCM (4 ml), and then 2-methoxyethanamine (101.39 mg, 1.35 mmol) was added. The reaction was stirred at room temperature for 16 hours. The resin was filtered off, and washed with DCM, CH₃OH THF and CH₃CN. The resin was suspended in CH₃CN (4 ml). Final compound 1-01 (50 mg, 0,11 mmol) and triethylamine (84 µl 0,605 mmol) were added. The reaction was heated at 65°C for 16 hours. The resin was filtered off, washed with CH₃CN, DCM and CH₃OH and the solvent was evaporated till dryness, to yield 58 mg of the final compound **1-45**(97%).

### B5. Preparation of final compound 1-22

### a) Preparation of final compound 5-10

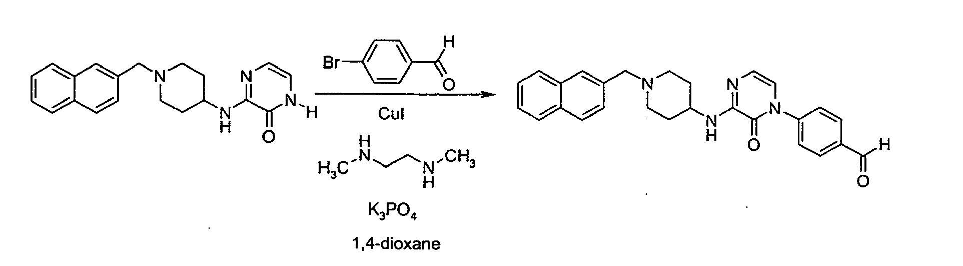

Final compound 5-01 (1 g, 2,99 mmol), 4-bromobenzaldehyde (664 mg, 3,58 mmol) and Cul (114 mg, 0,598 mmol) were suspended in 1,4-dioxane (10 ml). The reaction was stirred for 1 minute, and then *N,N*'-dimethyl-1,2-ethanediamine (131 µl, 1,2 mmol) was added while stirring for 5 minutes more. K₃PO₄ (1,26 g, 5,98 mmol) was added, while heating the mixture in a sealed tube at 110°C for 16 hours. The reaction was filtered over celite, washed with DCM and the solvent was evaporated till dryness. The crude compound was dissolved in EtOAc, washed with H₂O and brine, and dried (MgSO₄). The solvent was concentrated under vacuum, and the resulting crude purified by HPLC to yield 220 mg of the final compound **5-10** (34 %).

### b) Preparation of final compound 1-22

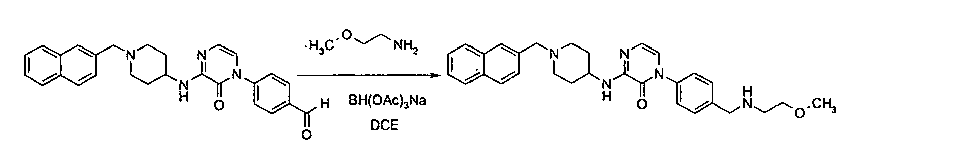

Final compound 5-10 (250 mg, 0,570 mmol), 2-methoxyethanamine (1,14 mmol) and BH(OAc)₃Na (181,20 mg, 0,855 mmol) were suspended in DCE (50 ml). The reaction was stirred at room temperature for 16 hours. Then NaHCO₃ was added. The organic layer was separated and washed with brine and dried (MgSO₄). The solvent was concentrated under vacuum. The resulting crude was purified by open chromatography in DCM/CH₃OH 9/1 yielding 115 mg of the final compound **1-22** (40 %).

### B6. Preparation of final compound 6-07

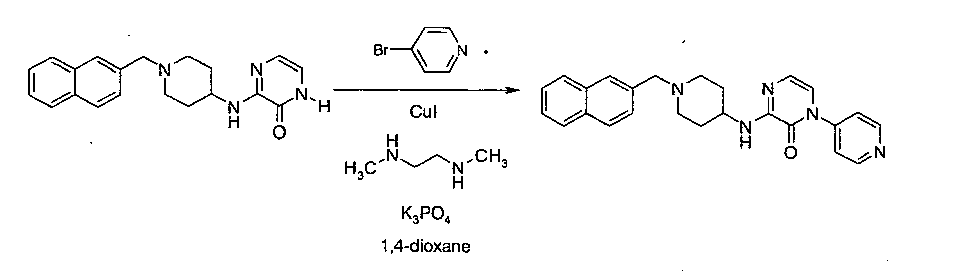

Final compound 5-01 (140 mg, 0.419 mmol), 4-bromopyridine (0.502 mmol) and Cul (16 mg, 0.083 mmol) were suspended in 1,4-dioxane (4 ml). The reaction was stirred for 1 minute at room temperature. Then *N,N*'-dimethyl-1,2-ethanediamine (18 µl, 0.167 mmol) was added and the mixture was stirred for 5 minutes more. Finally, K₃PO₄ (178 mg, 0,838 mmol) was added while heating the reaction mixture in a sealed tube at 110 °C for 16 hours. The mixture was filtered over celite, washed with DCM and the solvent evaporated till dryness. The crude was dissolved then in DCM, washed with H₂O and brine and dried (MgSO₄). The solvent was concentrated under reduced pressure. The resulting crude was purified by open chromatography in DCM/(CH₃OH/NH3) 9.5/0.5 to yield 120 mg the final compound **6-07** (70 %).

### B7. Preparation of final compound 1-09

### a) Preparation of final compound 3-05

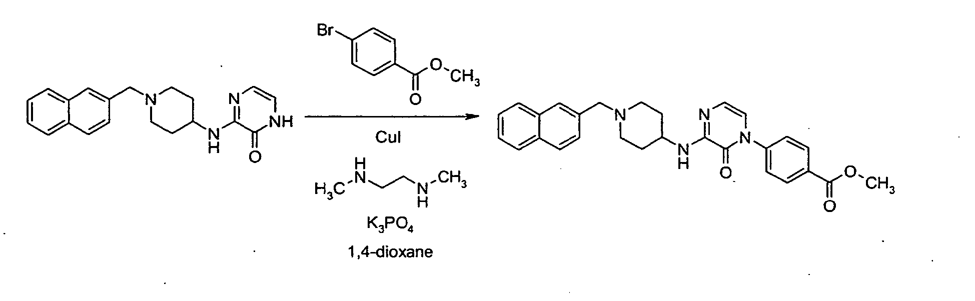

Final compound 5-01 (180 mg, 0.538 mmol), 4-bromobenzoic acid methyl ester (139 mg, 0.646 mmol) and Cul (21 mg, 0.17 mmol) were suspended in 1,4-dioxane (5 ml) and stirred at room temperature for 1 minute. Then *N,N*'-dimethyl-1,2-ethanediamine (23 µl, 0.215 mmol) was added and the mixture was stirred for 5 minutes more. Finally K₃PO₄ (228 mg, 1.07 mmol) was added, and the mixture was heated at 110°C for 16 hours in a sealed tube. The crude product was filtered over celite, washed with DCM and the solvent was concentrated under vacuum. The resulting crude was washed with H₂O, brine and dried (MgSO₄). The solvent was evaporated under reduced pressure and the resulting crude was purifed by open chromatography in DCM/(CH₃OH/NH3) 9.5/0.5 to yield 200 mg of the final compound **3-05** (80 %).

### b) Preparation of final compound 3-15

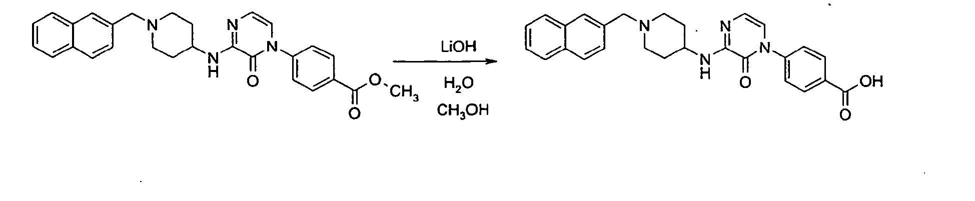

Final compound 3-05 (600 mg, 1.30 mmol), was suspended in CH₃OH (12 ml). Then LiOH (62.64 mg, 2.61 mmol) and H₂O (2.4 ml) were added and stirred for 16 hours at room temperature. The reaction was neutralized with HCl 10 %, the solvent was removed, and the product was triturated with Et₂O yielding 578 mg of the purified final compound **3-15** (quantitative).

### c) Preparation of final compound 1-09

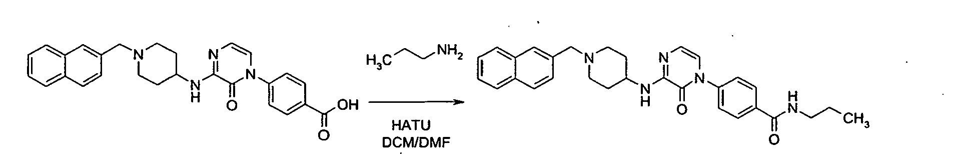

Final compound 3-15 (100 mg, 0.224 mmol) and HATU (100.83 mg, 0.265 mmol) were dissolved in DCM/DMF (11.5 ml, 2:1). Then 1-propanamine (12.06 mg, 0.204 mmol) was added. The reaction mixture was stirred at room temperature for 16 hours. The solvent was removed under vacuum. The crude was dissolved in DCM, washed with NH₄Cl, and brine, then dried (MgSO₄). The solvent was concentrated under reduced pressure and the resulting crude purified by open chromatography in SiO₂ with DCM/(CH₃OH/NH₃) 9.5/0.5 to yield 55 mg of final compound **1-09** (54 %).

### B8. Preparation of final compound 1-05

### a) Preparation of final compound 5-12

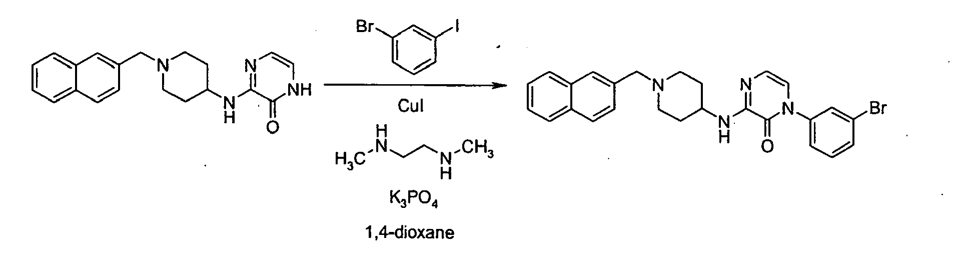

Final compound 5-01 (400 mg, 1.19 mmol), 1-bromo-3-iodobenzene (508 mg, 1.79 mmol) and Cul (45 mg, 0.24 mmol) were suspended in 1,4-dioxane (80 ml). The mixture was stirred at room temperature for 1 minute. Then *N,N*'-dimethyl-1,2-ethanediamine (54 µl, 0.480 mmol) was added and the mixture was stirred for 5 minutes more. K₃PO₄ (517 mg, 2.38 mmol) was added, and the mixture was heated at 110°C for 16 hours in a sealed tube. The crude product was filtered over celite, washed with DCM and the solvent was concentrated under vacuum. The resulting crude was washed with H₂O brine and dried (MgSO₄). The solvent was evaporated under reduced pressure and the resulting crude was purified by open chromatography in DCM/(CH₃OH/NH3) 9.5/0.5 to yield 120 mg of the final compound **5-12** (21 %).

### b) Preparation of final compound 1-05

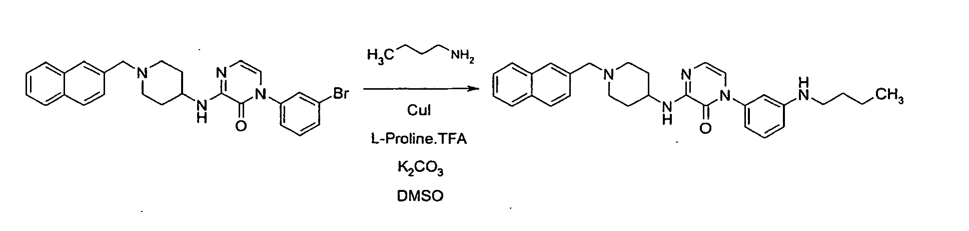

Final compound 5-12 (810 mg, 0.16 mmol), 1-butanamine (24 µl, 0.24 mmol), Cul (6 mg, 0,032 mmol) and L-proline.TFA (15 mg, 0.128 mmol) were suspended in DMSO (1 ml). Then K₂CO₃ (44 mg, 0.32 mmol) was added and the mixture was heated to 80 °C for 24 hours in a sealed tube. The crude product was filtered over celite, washed with DCM and the solvent was concentrated under vacuum. The resulting crude was washed with H₂O brine and dried (MgSO₄). The solvent was evaporated under reduced pressure and the resulting crude was purified by open chromatography in DCM/(CH₃OH/NH₃) 50/1 to yield 40 mg of the final compound **1-05** (52 %).

### B9. Preparation of final compound 8-05

### a) Preparation of final compound 8-04

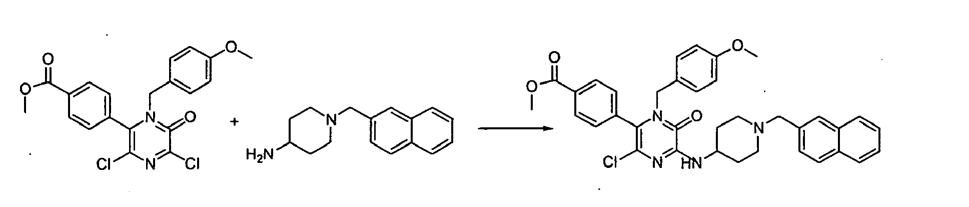

To a mixture of intermediate compound 5 (0.756 mmol) and intermediate compound 6 (0.787 mmol) in toluene/CH₃O (9/1) (2 ml), K₂CO₃ (105 mmol) was added. The reaction was heated under microwave irradiation at 130 °C for 30 minutes. The solvent was evaporated and the oil was purified by column chromatography (eluent DCM/CH₃OH 9/1); selected fractions were collected and their solvent evaporated yielding 0.39 g (88 %) of final compound **8-04** as a white solid.

### b) Preparation of final compound 8-03

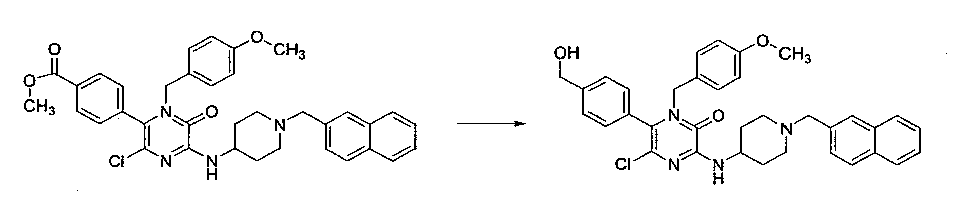

To a mixture of final compound 8-04 (0.32 mmol) in THF (20 mL), DIBAL/THF (3.2 mmol) was added at -78 °C. The reaction was stirred to room temperature for 24 hours. The crude was filtered over celite and the solvent was evaporated; the residue was treated with a solution of NaOH (4 mL) 4 N and DCM (20 mL). The crude were stirred for 10 minutes and the final compound was extracted with DCM (3x5 mL). The organic solvent was evaporated and the oil was purified by column chromatography (eluent DCM/CH₃OH 9/1); selected fractions were collected and their solvent evaporated, yielding 0.125 g (65 %) of final compound **8-03** as a white solid.

### c) Preparation of final compound 8-16

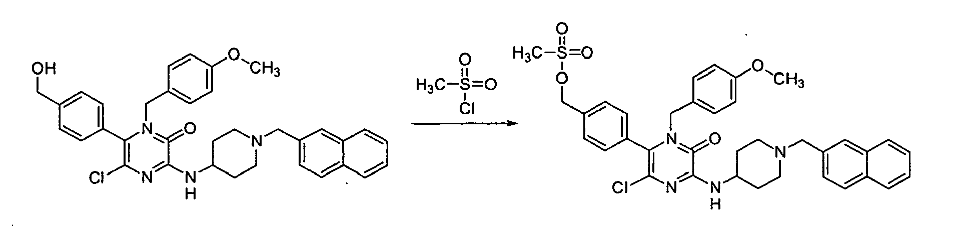

To a mixture of final compound 8-03 (0.336 mmol) in DCM (10 mL) methanesulfonyl chloride (0.37 mmol) and DiPEA (0.73 mmol) was added at 0 °C. The reaction mixture was stirred at room temperature for 3 hours. The solvent was evaporated and the residue was used for the next step without any purification. After the solvent was removed, the final compound **8-16** was obtained as a yellow oil.

### d) Preparation of final compound 8-05

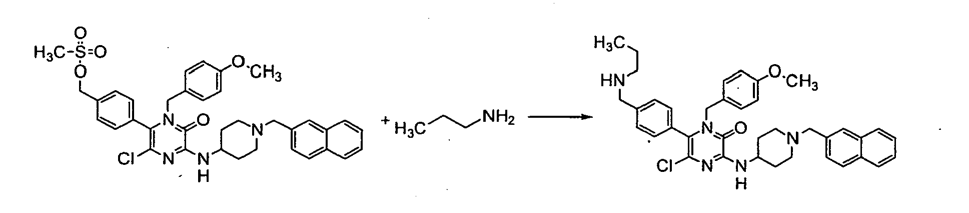

A mixture of final compound 8-16 (0.2971 mmol), propylamine (0.3268 mmol), and K₂CO₃ (0.653 mmol) in CH₃CN (2 mL) was heated under microwave irradiation at 130°C for 10 minutes. The organic solvent was evaporated and the oil was purified by column chromatography (eluent DCM/CH₃OH 9/1) ; selected fractions were collected and their solvent evaporated, yielding 0.135 g (71 %) of final compound **8-05** as an oil.

### B10. Preparation of final compound 7-02

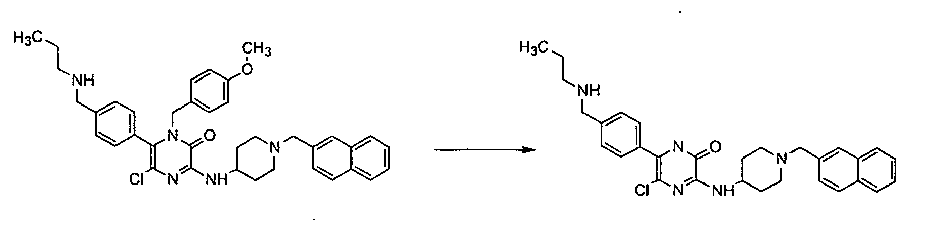

A mixture of final compound 8-05 (0.16 mmol) in TFA (2 mL) was heated under microwave irradiation at 130°C for 15 minutes. The organic solvent was evaporated and the oil was purified by column chromatography (eluent DCM/CH₃OH 9/1) ; selected fractions were collected and their solvent was evaporated, yielding 0.05 g (56 %) of final compound **7-02.**

The following compounds were prepared according to the above examples, schemes and procedures.

**Table 1: List of compounds for which Q² is a moiety of formula -NR¹R².**

| | | | | |
|---|---|---|---|---|
| 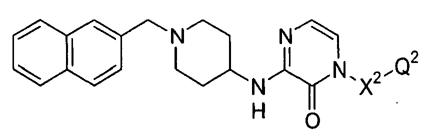 | | | | |

| **Co.Nr.** | **Scheme** | **--X²--** | **--Q²** | **Physico-chemical/ stereochemical data** |
|---|---|---|---|---|
| **1-01** | 5A | 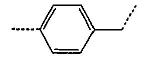 | -NH | |
| **1-02** | 5A | 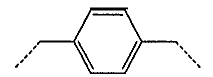 | -NH₂ | |
| **1-03** | 5A | 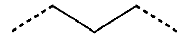 | -NH₂ | |
| **1-04** | 5A | 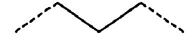 | 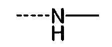 | |
| **1-05** | 3B | 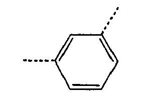 | 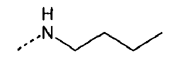 | |
| **1-06** | 3A | 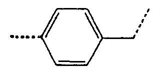 | 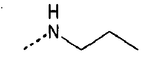 | |
| **1-07** | 3A | 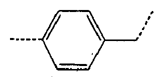 | 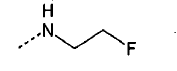 | |
| **1-08** | 3A | 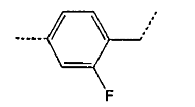 | 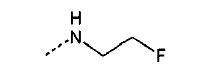 | |
| **1-09** | 4 | 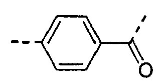 | 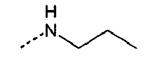 | |
| **1-10** | 4 | 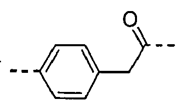 | 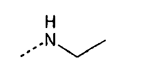 | |
| **1-11** | 4 | 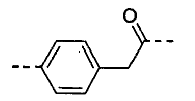 | 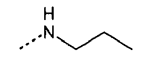 | |
| **1-12** | 2A | 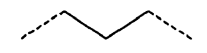 | 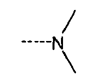 | |
| **1-13** | 4 | 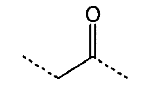 | 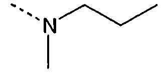 | |
| **1-14** | 3A | 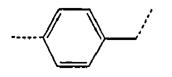 | 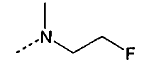 | |
| **1-15** | 2A | 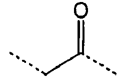 | 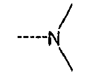 | |
| **1-16** | 4 | 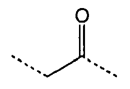 | 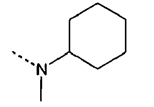 | |
| **1-17** | 3A | 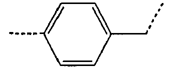 | 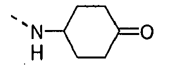 | |
| **1-18** | 4 | 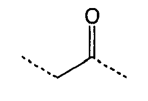 | 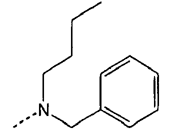 | |
| **1-19** | 4 | 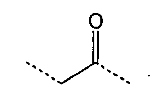 | 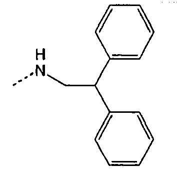 | |
| **1-20** | 4 | 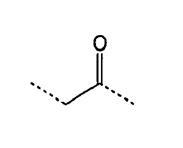 | 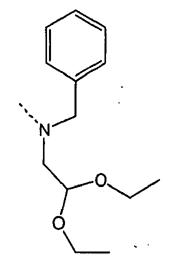 | |
| **1-104** | 4 | 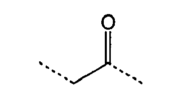 | 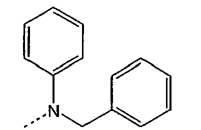 | |
| **1-21** | 3A | 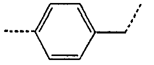 | 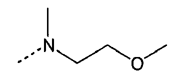 | |
| **1-22** | 3A | 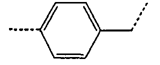 | 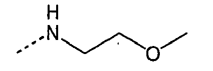 | 2 HCl |
| **1-23** | 5B | 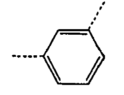 | 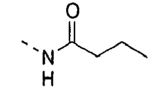 | |
| **1-24** | 5B | 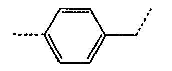 | 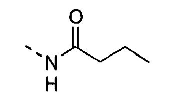 | |
| **1-25** | 5B | 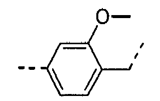 | 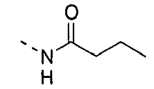 | |
| **1-26** | 5B | 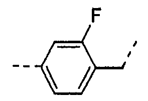 | 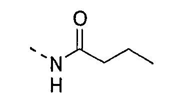 | |
| **1-27** | 5B | 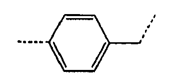 | 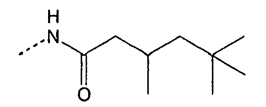 | |
| **1-28** | 5B | 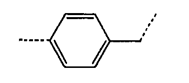 | 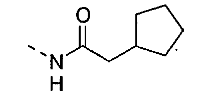 | |
| **1-29** | 5D | 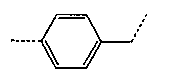 | 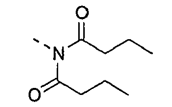 | |
| **1-30** | 5B | 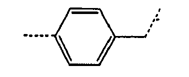 | 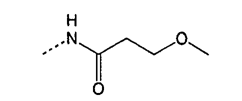 | |
| **1-31** | 5B | 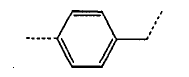 | 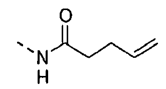 | |
| **1-32** | 2A | 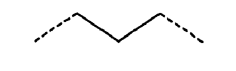 | 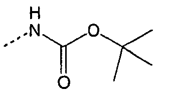 | |
| **1-33** | 2B | 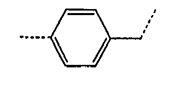 | 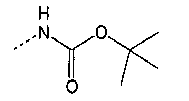 | |
| **1-34** | 2A | 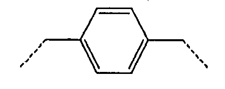 | 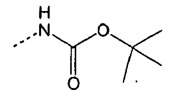 | |
| **1-35** | 2A | 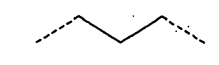 | 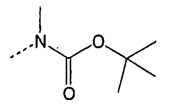 | |
| **1-36** | 5E | 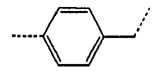 | 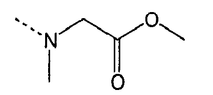 | |
| **1-37** | 5B | 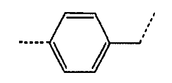 | 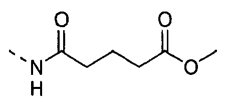 | |
| **1-38** | 4 | 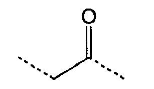 | 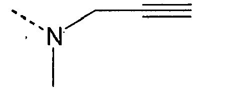 | |
| **1-39** | 3A | 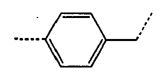 | 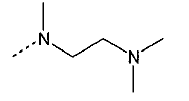 | |
| **1-40** | 5D | 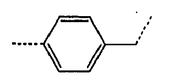 | 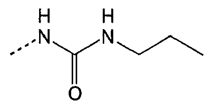 | |
| **1-41** | 5D | 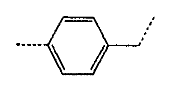 | 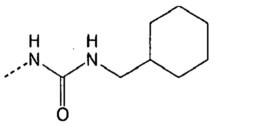 | |
| **1-42** | 5B | 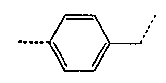 | 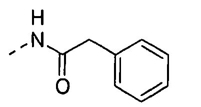 | |
| **1-43** | 5B | 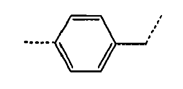 | 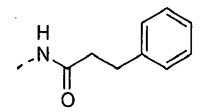 | |
| **1-44** | 5D | 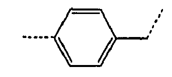 | 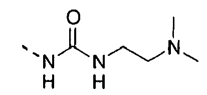 | |
| **1-45** | 5D | 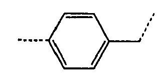 | 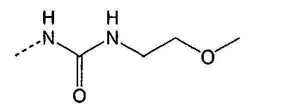 | |
| **1-46** | 5D | 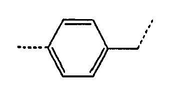 | 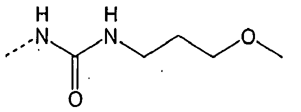 | |
| **1-47** | 5D | 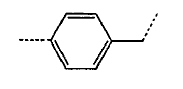 | 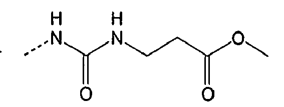 | |
| **1-48** | 5D | 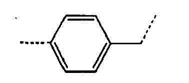 | 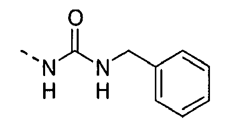 | |
| **1-49** | 5D | 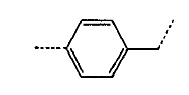 | 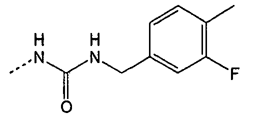 | |
| **1-50** | 5D | 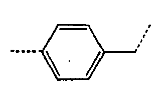 | 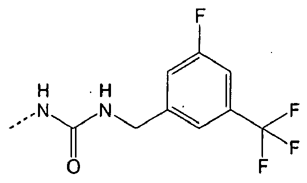 | |
| **1-51** | 5D | 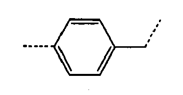 | 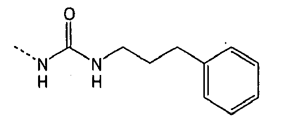 | |
| **1-52** | 5D | 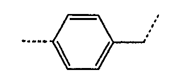 | 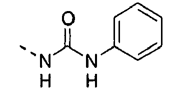 | |
| **1-53** | 5D | 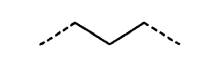 | 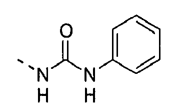 | |
| **1-54** | 5D | 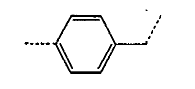 | 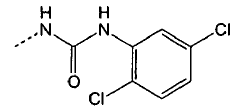 | |
| **1-55** | 5D | 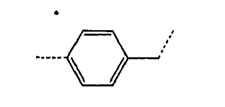 | 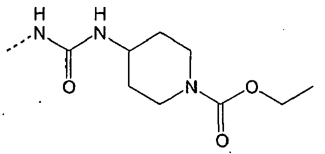 | |
| **1-56** | 3A | 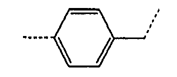 | 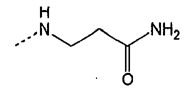 | |
| **1-57** | 3A | 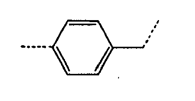 | 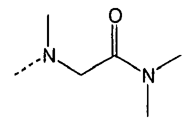 | |
| **1-58** | 5B | 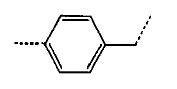 | 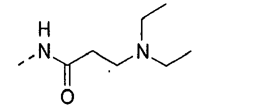 | |
| **1-59** | 5B | 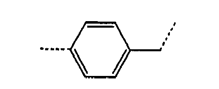 | 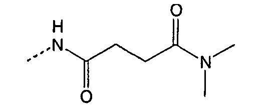 | |
| **1-60** | 3A | 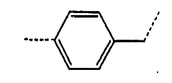 | 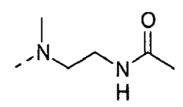 | |
| **1-61** | 3A | 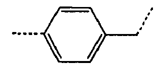 | 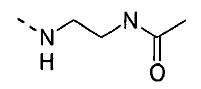 | |
| **1-62** | 5B | 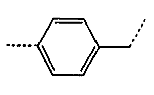 | 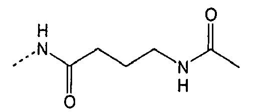 | |
| **1-63** | 4 | 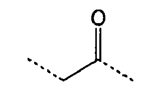 | 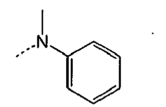 | |
| **1-64** | 4 | 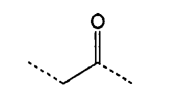 | 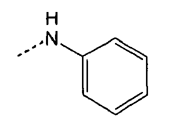 | |
| **1-65** | 4 | 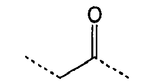 | 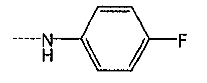 | |
| **1-66** | 4 | 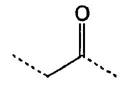 | 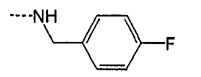 | |
| **1-67** | 5E | 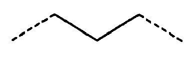 | 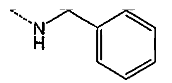 | |
| **1-68** | 4 | 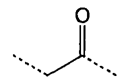 | 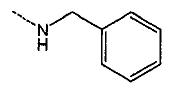 | |
| **1-69** | 4 | 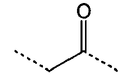 | 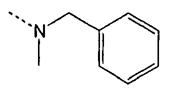 | |
| **1-70** | 3A | 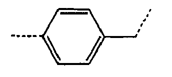 | 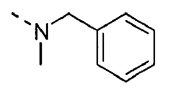 | |
| **1-71** | 4 | 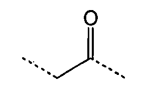 | 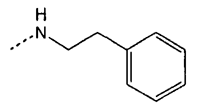 | |
| **1-72** | 4 | 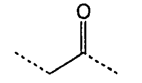 | 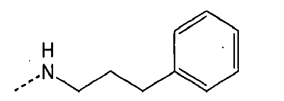 | |
| **1-73** | 4 | 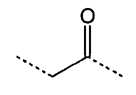 | 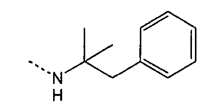 | |
| **1-74** | 5B | 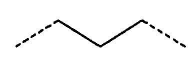 | 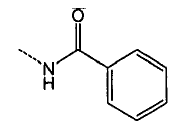 | |
| **1-75** | 5B | 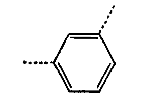 | 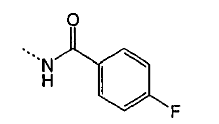 | |
| **1-76** | 5B | 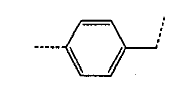 | 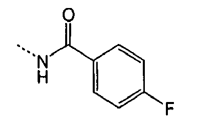 | |
| **1-77** | 2A | 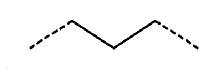 | 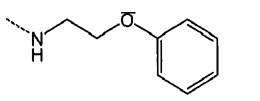 | |
| **1-78** | 3B | 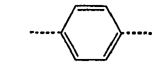 | 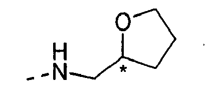 | (RS) |
| **1-79a** | 3A | 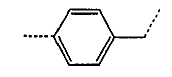 | 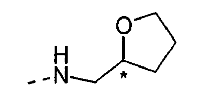 | (RS) |
| **1-79b** | 3A | 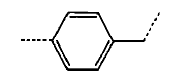 | 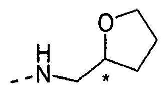 | .2HCl, (RS) |
| **1-80** | 3A | 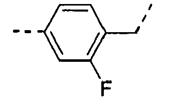 | 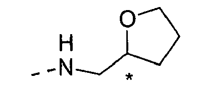 | (RS) |
| **1-81** | 4 | 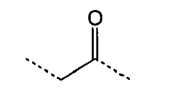 | 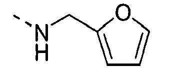 | |
| **1-82** | 3A | 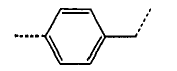 | 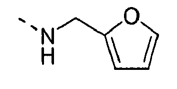 | |
| **1-83** | 3A | 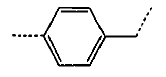 | 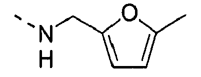 | 2HCl |
| **1-84** | 3A | 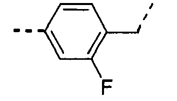 | 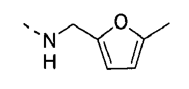 | |
| **1-85** | 3A | 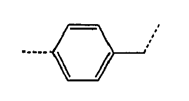 | 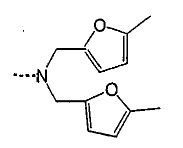 | |
| **1-86** | 4 | 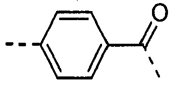 | 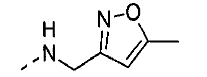 | |
| **1-87** | 3A | 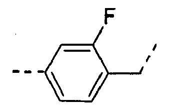 | 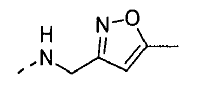 | |
| **1-88** | 4 | 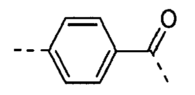 | 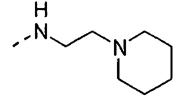 | |
| **1-89** | 3B | 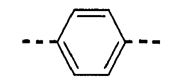 | 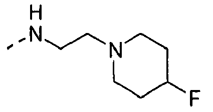 | |
| **1-90** | 3A | 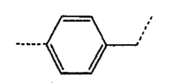 | 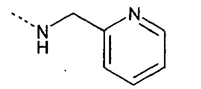 | |
| **1-91** | 4 | 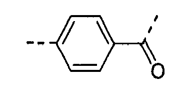 | 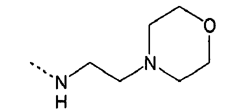 | |
| **1-92** | 3B | 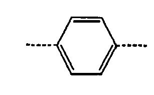 | 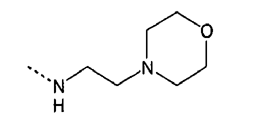 | |
| **1-93** | 3A | 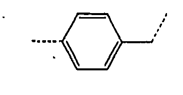 | 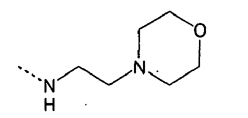 | |
| **1-94** | 4 | 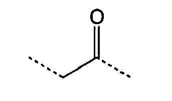 | 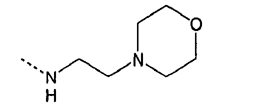 | |
| **1-95** | 5B | 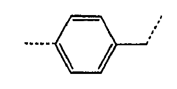 | 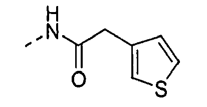 | |
| **1-96** | 5B | 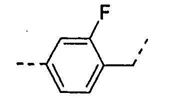 | 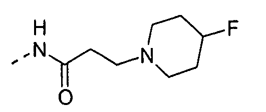 | .HCl |
| **1-97** | 5B | 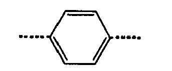 | 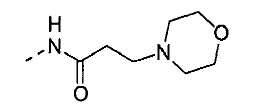 | |
| **1-98** | 5D | 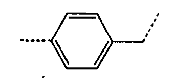 | 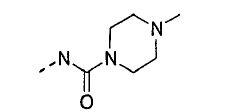 | |
| **1-99** | 5B | 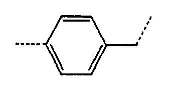 | 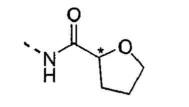 | (RS) |
| **1-100** | 5B | 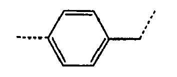 | 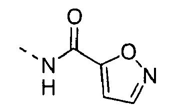 | |
| **1-101** | 5B | 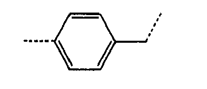 | 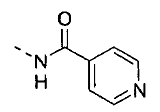 | |
| **1-102** | 5B | 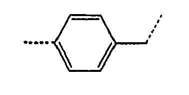 | 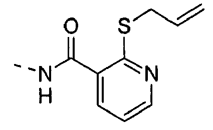 | |
| **1-103** | 5E | 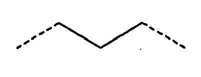 | 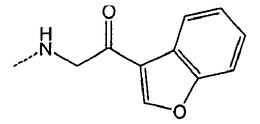 | |

**Table 2: List of compounds for which Q² is a moiety of formula -Pir.**

| | | | |
|---|---|---|---|
| 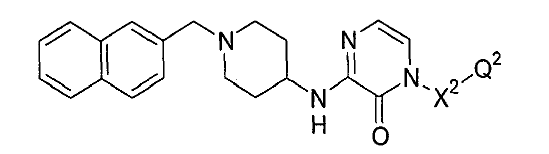 | | | |

| **Co.Nr.** | **Scheme** | **--X²--** | **--Q²** |
|---|---|---|---|
| **2-01** | 2B | 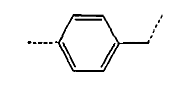 | 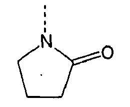 |
| **2-02** | 3A | 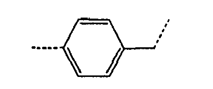 | 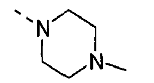 |
| **2-03** | 3B | 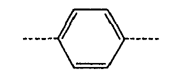 | 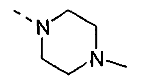 |
| **2-04** | 3A | 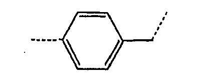 | 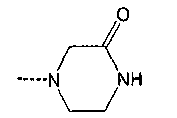 |
| **2-05** | 4 | 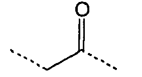 | 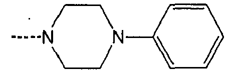 |
| **2-06** | 4 | 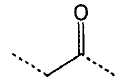 | 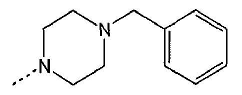 |
| **2-07** | 4 | 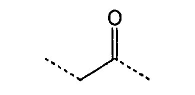 | 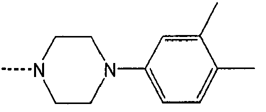 |
| **2-08** | 4 | 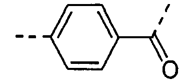 | 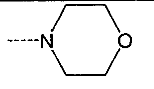 |
| **2-09** | 2A | 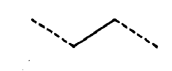 | 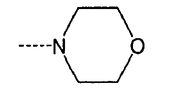 |
| **2-10** | 2A | 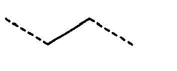 | 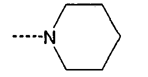 |
| **2-11** | 3A | 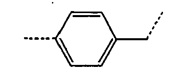 | 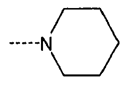 |
| **2-12** | 3A | 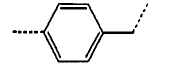 | 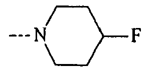 |
| **2-13** | 3A | 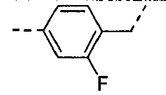 | 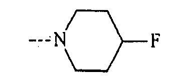 |
| **2-14** | 3A | 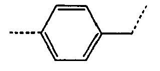 | 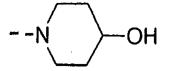 |
| **2-15** | 4 | 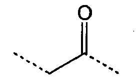 | 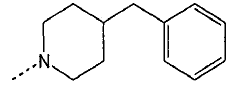 |
| **2-16** | 4 | 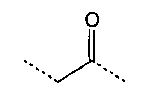 | 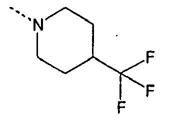 |
| **2-17** | 3A | 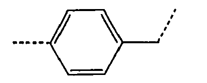 | 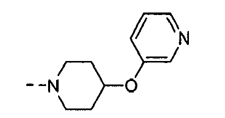 |
| **2-18** | 2A | 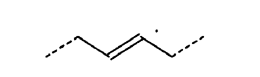 | 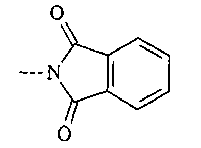 |
| **2-19** | 2A | 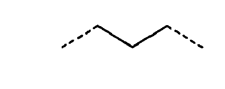 | 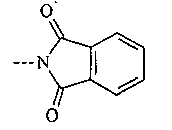 |
| **2-20** | 2A | 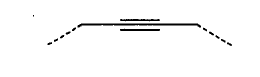 | 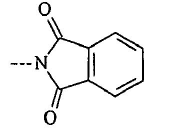 |

**Table 3: List of compounds for which Q² is a moiety of formula -OR³.**

| | | | | |
|---|---|---|---|---|
| 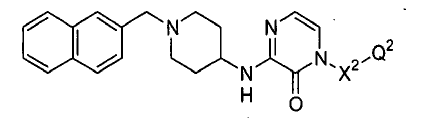 | | | | |

| **Co.Nr.** | **Scheme** | --X²-- | --Q² | **Physico-chemical/ stereo-chemical data** |
|---|---|---|---|---|
| **3-01** | 2A | 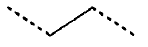 | -OH | |
| **3-02** | 2A | 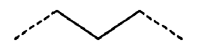 | -OH | |
| **3-03** | 2A | 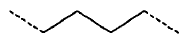 | -OH | |
| **3-15** | 2A | 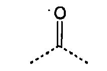 | -OH | |
| **3-04** | 2A | 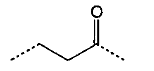 | ----O- | |
| **3-05** | 2B | 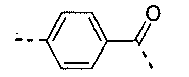 | ----O- | |
| **3-06** | 2A | 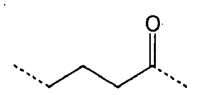 | 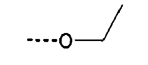 | |
| **3-07** | 2B | 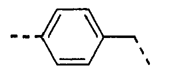 | 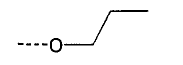 | |
| **3-08** | 2A | 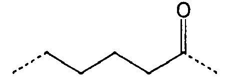 | 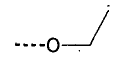 | |
| **3-09** | 2A | 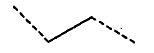 | 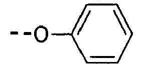 | |
| **3-10** | 2A | 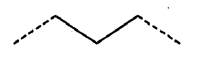 | 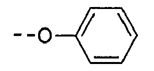 | |
| **3-11** | 2A | 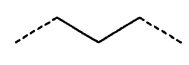 | 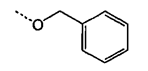 | |
| **3-12** | 2A | 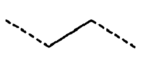 | 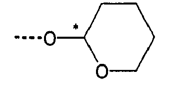 | (RS) |
| **3-13** | 2B | 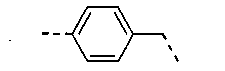 | 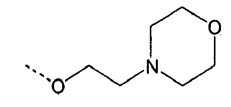 | |
| **3-14** | 2B | 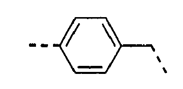 | 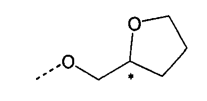 | (RS) |

**Table 4 : List of compounds for which there are two moieties Q² equal to -OR³, optionally taken together to form a moiety -OCH₂CH₂O-.**

| | | | | |
|---|---|---|---|---|
| 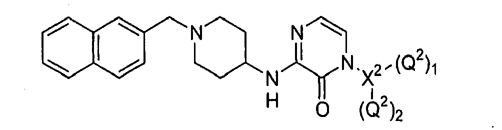 | | | | |

| **Co.Nr** | **Scheme** | **--X² --** | **--Q¹** | **--Q²** |
|---|---|---|---|---|
| **4-01** | 2A | 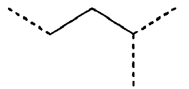 | 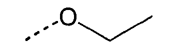 | 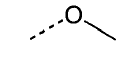 |
| **4-02** | 2A | 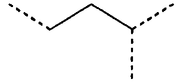 | 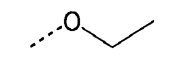 | 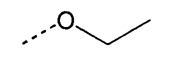 |
| **4-03** | 2A | 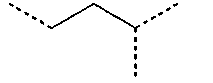 | 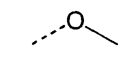 | 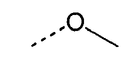 |
| **4-04** | 2A | 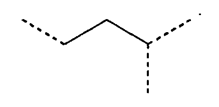 | 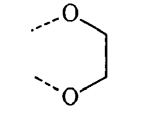 | |
| **4-05** | 2B | 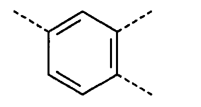 | 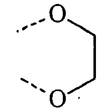 | |

**Table 5 : List of compounds for which Q² is -H.**

| | | | |
|---|---|---|---|
| 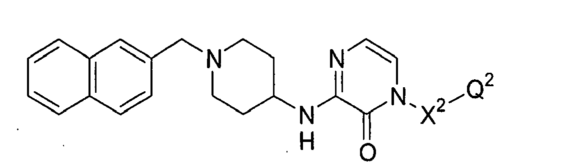 | | | |

| **Co.Nr.** | **Scheme** | **--X²--** | **--Q²** |
|---|---|---|---|
| **5-01** | 1B | c.b. | --H |
| **5-02** | 2A | 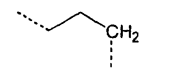 | --H |
| **5-03** | 2A | 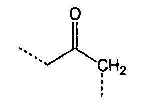 | --H |
| **5-04** | 2A | 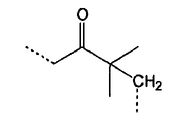 | --H |
| **5-05** | 2A | 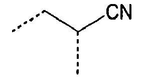 | --H |
| **5-06** | 2A | 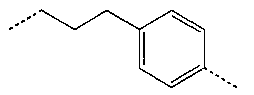 | --H |
| **5-07** | 2A | 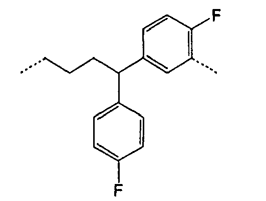 | --H |
| **5-08** | 2A | 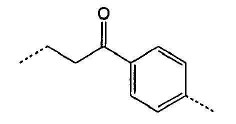 | --H |
| **5-09** | 2A | 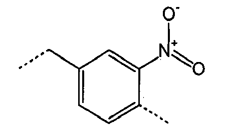 | --H |
| **5-10** | 2B | 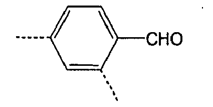 | --H |
| **5-11** | 2B | 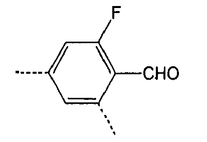 | --H |
| **5-12** | 2B | 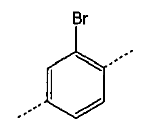 | --H |
| **5-13** | 2A | 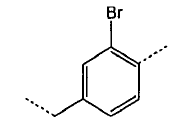 | --H |

**Table 6 : List of compounds for which Q² is -Het.**

| | | | |
|---|---|---|---|
| 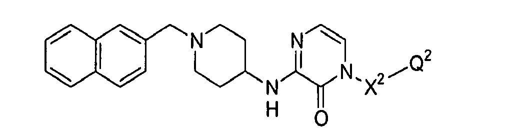 | | | |

| **Co.Nr.** | **Scheme** | **--X²--** | **--Q²** |
|---|---|---|---|
| **6-01** | 2B | c.b. | 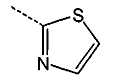 |
| **6-02** | 2B | c.b. | 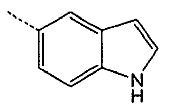 |
| **6-03** | 2B | c.b. | 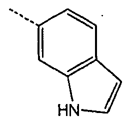 |
| **6-04** | 2B | c.b. | 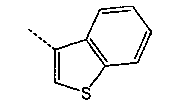 |
| **6-05** | 2B | c.b. | 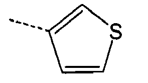 |
| **6-16** | 2B | c.b. | 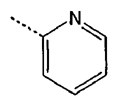 |
| **6-06** | 2B | c.b. | 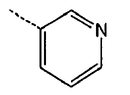 |
| **6-07** | 2B | c.b. | 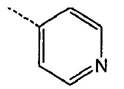 |
| **6-08** | 2B | c.b. | 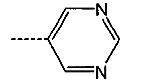 |
| **6-09** | 2B | c.b. | 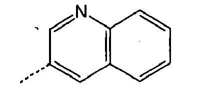 |
| **6-10** | 2A | ---CH₂--- | 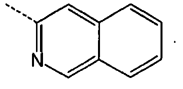 |
| **6-11** | 2A | 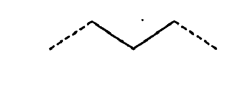 | 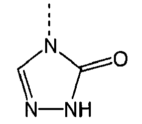 |
| **6-12** | 5E | c.b. | 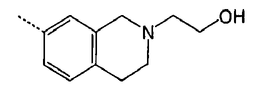 |
| **6-13** | 5B | c.b. | 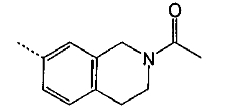 |
| **6-14** | 5E | c.b. | 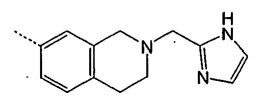 |
| **6-15** | 5E | c.b. | 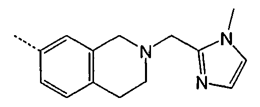 |

**Table 7 : List of compounds for which Q¹ is a moiety of formula -H, -NR¹R² or -Pir.**

| | | | | |
|---|---|---|---|---|
| 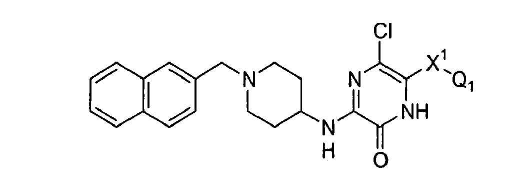 | | | | |

| **Co.Nr.** | **Scheme** | **--X¹--** | **--Q¹** | **Physico-chemical/ stereo-chemical data** |
|---|---|---|---|---|
| **7-01** | 6 | 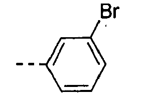 | --H | .CF₃COOH |
| **7-02** | 6 | 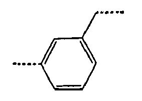 | 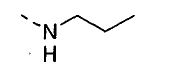 | .2CF₃COOH |
| **7-03** | 6 | 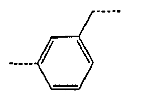 | 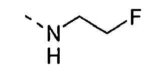 | .2CF₃COOH |
| **7-04** | 6 | 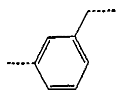 | 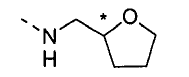 | .2CF₃COOH, (RS) .2CF₃COOH |
| **7-05** | 6 | 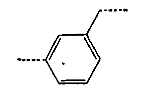 | 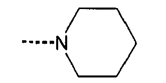 | |
| **7-06** | 6 | 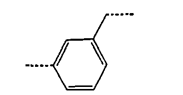 | 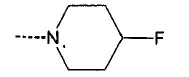 | 2CF₃COOH |
| **7-07** | 6 | 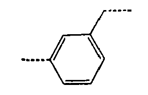 | 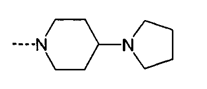 | .2CF₃COOH |
| **7-08** | 6 | 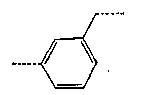 | 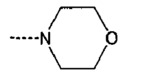 | .2CF₃COOH |
| **7-09** | 6 | 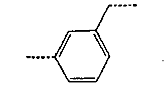 | 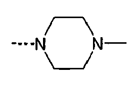 | .2CF₃COOH |
| **7-10** | 6 | 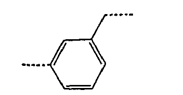 | 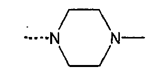 | .3CF₃COOH |

**Table 8 : List of compounds for which Q¹ is a moiety of formula -H, -OR³, -NR¹R² or -Pir.**

| | | | | |
|---|---|---|---|---|
| 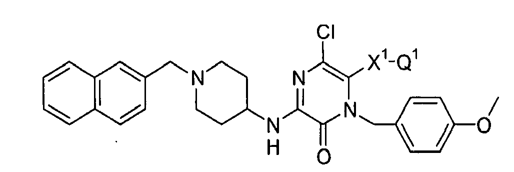 | | | | |

| **Co.Nr.** | **Scheme** | **--X¹--** | **--Q¹** | **Physico-chemical/ stereochemical data** |
|---|---|---|---|---|
| **8-01** | 6 | 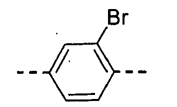 | --H | |
| **8-02** | 6 | 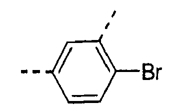 | --H | |
| **8-03** | 6 | 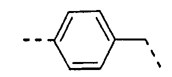 | --OH | |
| **8-04** | 6 | 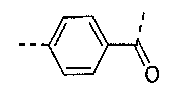 | 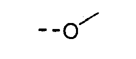 | |
| **8-16** | 6 | 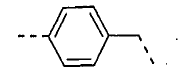 | --O-SO₂CH₃ | |
| **8-05** | 6 | 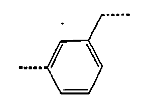 | 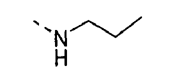 | |
| **8-06** | 6 | 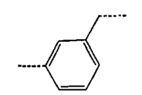 | 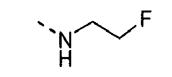 | |
| **8-07** | 6 | 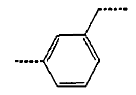 | 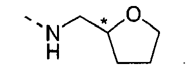 | (RS) |
| **8-08** | 6 | 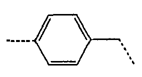 | 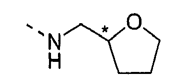 | (RS) |
| **8-09** | 6 | 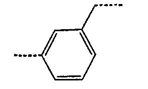 | 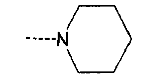 | |
| **8-10** | 6 | 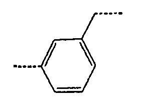 | 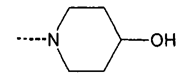 | |
| **8-11** | 6 | 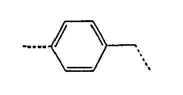 | 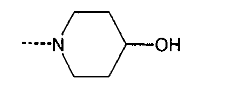 | |
| **8-12** | 6 | 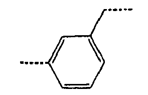 | 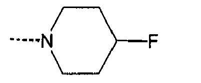 | |
| **8-13** | 6 | 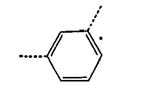 | 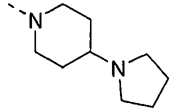 | |
| **8-14** | 6 | 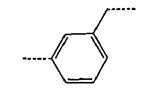 | 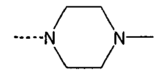 | |
| **8-15** | 6 | 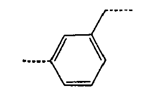 | 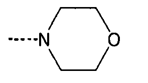 | |

**Table 9 : List of compounds where V is other than 2-naphthyl or Y is other than aminopiperidinyl.**

| | | | | | | |
|---|---|---|---|---|---|---|
| 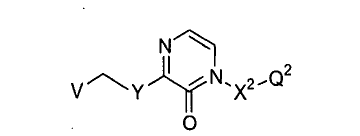 | | | | | | |

| **Co.Nr** | **Scheme** | **V--** | **--Y--** | **-X²--** | **--Q²** | **Physico-chemical/ stereochemical data** |
|---|---|---|---|---|---|---|
| **9-23** | 2A | 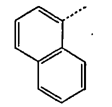 | 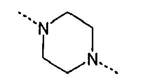 | 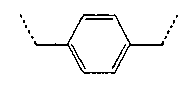 | -NH₂ | .2CF₃COOH |
| **9-24** | 2A | 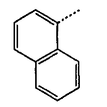 | 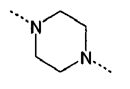 | 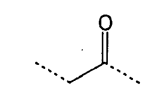 | 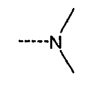 | |
| **9-25** | 2A | 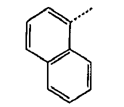 | 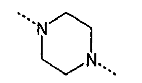 | 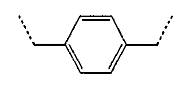 | 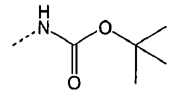 | |
| **9-26** | 2A | 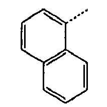 | 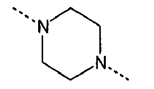 | 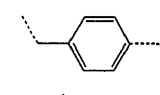 | --H | |
| **9-01** | 2A | 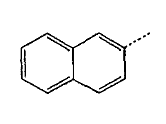 | 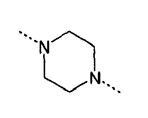 | 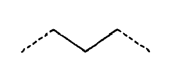 | 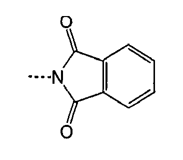 | .CF₃COOH |
| **9-02** | 2A | 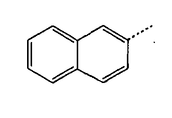 | 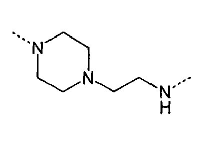 | 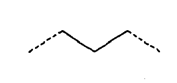 | 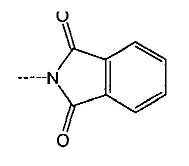 | .CF₃COOH |
| **9-03** | 2A | 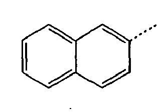 | 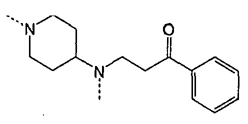 | 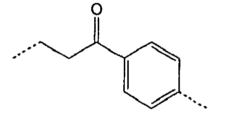 | --H | |
| **9-04** | 2A | 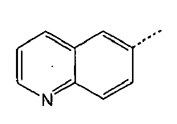 | 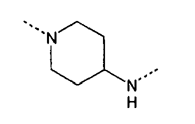 | 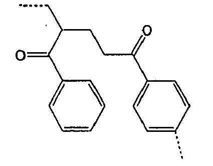 | --H | |
| **9-05** | 2A | 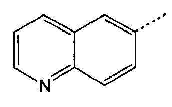 | 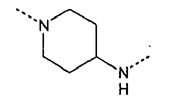 | 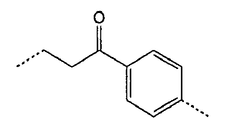 | --H | |
| **9-06** | 2A | 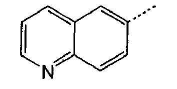 | 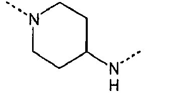 | 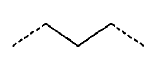 | 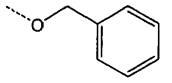 | |
| **9-07** | 2A | 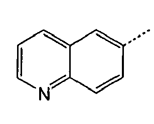 | 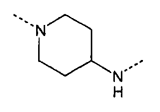 | 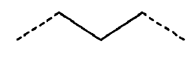 | 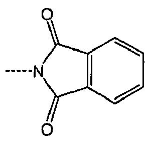 | .3CF₃COOH |
| **9-30** | 2A | 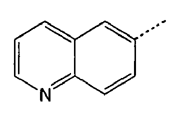 | 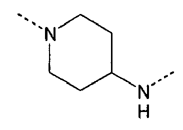 | 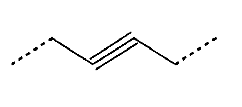 | 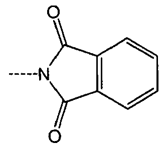 | .3CF₃COOH |
| **9-08** | 2A | 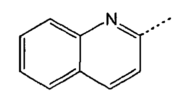 | 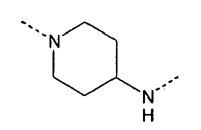 | 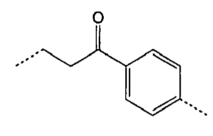 | --H | |
| **9-09** | 2A | 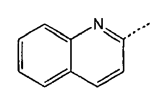 | 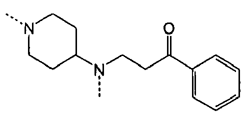 | 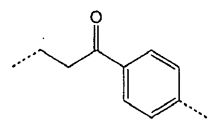 | --H | |
| **9-10** | 2A | 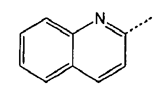 | 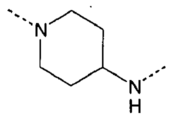 | 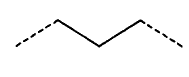 | 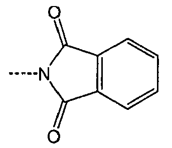 | .3CF₃COOH |
| **9-11** | 2A | 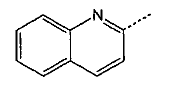 | 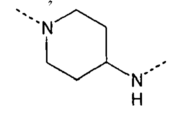 | 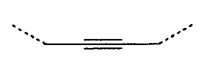 | 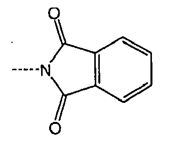 | .3CF₃COOH |
| **9-12** | 2A | 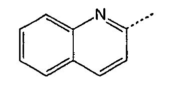 | 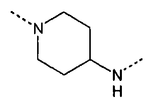 | 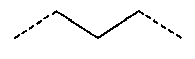 | 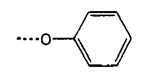 | |
| **9-13** | 1B | 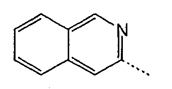 | 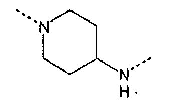 | c.b. | --H | |
| **9-14** | 2A | 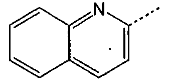 | 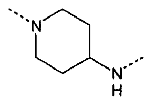 | 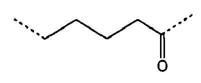 | 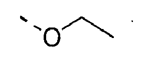 | |
| **9-15** | 2A | 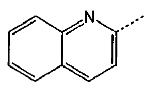 | 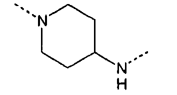 | 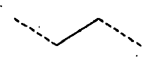 | 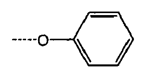 | |
| **9-16** | 2A | 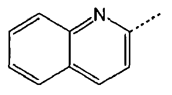 | 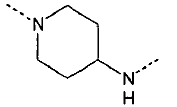 | 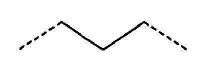 | 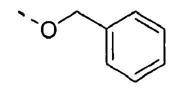 | |
| **9-17** | 2A | 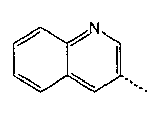 | 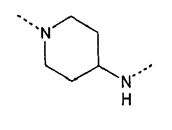 | 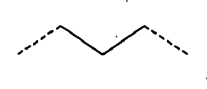 | 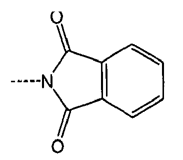 | |
| **9-18** | 2A | 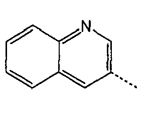 | 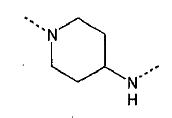 | 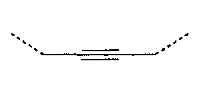 | 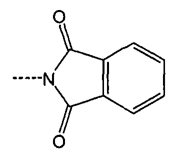 | .3CF₃COOH |
| **9-19** | 2A | 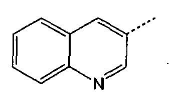 | 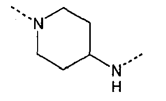 | 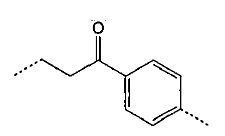 | H | |
| **9-20** | 2A | 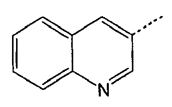 | 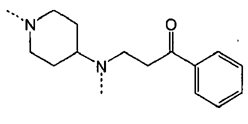 | 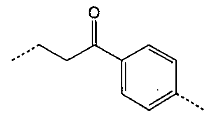 | H | |
| **9-21** | 2A | 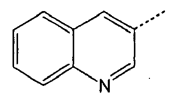 | 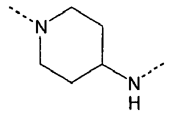 | 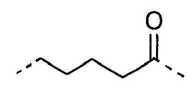 | 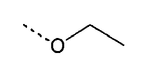 | |
| **9-22** | 2A | 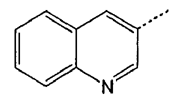 | 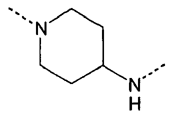 | 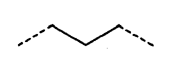 | 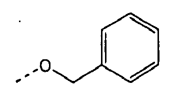 | |
| **9-27** | 2A | 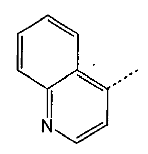 | 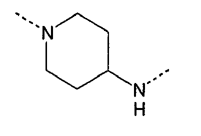 | 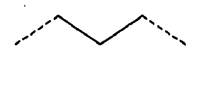 | 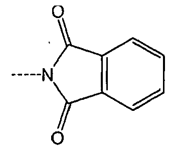 | |
| **9-28** | 2A | 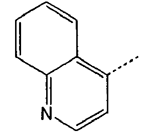 | 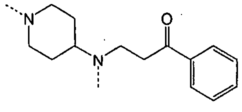 | 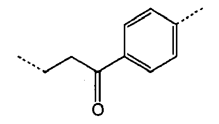 | H | |
| **9-29** | 2A | 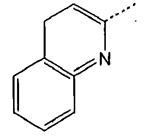 | 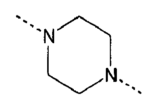 | 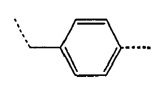 | H | |

### C. Pharmacological example

### General

The interaction of the compounds of Formula (I) with α_{2c}-adrenoceptor receptors was assessed in *in vitro* radioligand binding experiments. In general, a low concentration of a radioligand with a high binding affinity for a particular receptor or transporter is incubated with a sample of a tissue preparation enriched in a particular receptor or transporter or with a preparation of cells expressing cloned human receptors in a buffered medium. During the incubation, the radioligand binds to the receptor or transporter. When equilibrium of binding is reached, the receptor bound radioactivity is separated from the non-bound radioactivity, and the receptor- or transporter-bound activity is counted. The interaction of the test compounds with the receptor is assessed in competition binding experiments. Various concentrations of the test compound are added to the incubation mixture containing the receptor- or transporter preparation and the radioligand. The test compound in proportion to its binding affinity and its concentration inhibits binding of the radioligand. The radioligand used for hα_{2C}, hα_{2C} and hα_{2C} receptor binding was [³H]-raulwolscine.

### Example C.1 : Binding Experiment for α_{2c}-adrenoceptor

### Cell culture and membrane preparation.

CHO cells, stabile transfected with human adrenergic-α_{2A}, -α_{2B} or α_{2C} receptor cDNA, were cultured in Dulbecco's Modified Eagle's Medium (DMEM)/Nutrient mixture Ham's F12 (ratio 1:1)(Gibco, Gent-Belgium) supplemented with 10 % heat inactivated fetal calf serum (Life Technologies, Merelbeke-Belgium) and antibiotics (100 IU/ml penicillin G, 100 µg/ml streptomycin sulphate, 110 µg/ml pyruvic acid and 100 µg/ml L-glutamine). One day before collection, cells were induced with 5 mM sodiumbutyrate. Upon 80-90 % of confluence, cells were scraped in phosphate buffered saline without Ca²⁺ and Mg²⁺ and collected by centrifugation at 1500 x g for 10 minutes. The cells were homogenised in Tris-HCl 50 mM using an Ultraturrax homogenizer and centrifuged for 10 minutes at 23,500 x g. The pellet was washed once by resuspension and rehomogenization and the final pellet was resuspended in Tris-HCl , divided in 1 ml aliquots and stored at -70°C.

### Binding experiment for α₂-adrenergic receptor subtypes

Membranes were thawed and re-homogenized in incubation buffer (glycylglycine 25 mM, pH 8.0). In a total volume of 500 µl, 2-10 µg protein was incubated with [³H]raulwolscine (NET-722) (New England Nuclear, USA) (1 nM final concentration) with or without competitor for 60 minutes at 25°C followed by rapid filtration over GF/B filter using a Filtermate196 harvester (Packard, Meriden, CT). Filters were rinsed extensively with ice-cold rinsing buffer (Tris-HCl 50 mM pH 7.4). Filter-bound radioactivity was determined by scintillation counting in a Topcount (Packard, Meriden, CT) and results were expressed as counts per minute (cpm). Non-specific binding was determined in the presence of 1 µM oxymetazoline for hα_{2A}- and hα_{2B} receptors and 1 µM spiroxatrine for hα_{2C} receptors.

### Binding experiment for the 5HT-transporter

Human platelet membranes (Oceanix Biosciences Corporation, Hanover, MD, USA) were thawed, diluted in buffer (Tris-HCl 50 mM, 120 mM NaCl and 5 mM KCI) and quickly (max 3 s) homogenised with an Ultraturrax homogenizer. In a total volume of 250 µL, 50-100 µg protein was incubated with [³H]paroxetine (NET-869) (New England Nuclear, USA) (0.5 nM final concentration) with or without competitor for 60 min at 25 °C. Incubation was stopped by rapid filtration of the incubation mixture over GF/B filters, pre-wetted with 0.1 % polyethyleneamine, using a Filtermate196 harvester (Packard, Meriden, CT). Filters were rinsed extensively with ice-cold buffer and radioactivity on the filters was counted in a Topcount liquid scintillation counter (Packard, Meriden, CT). Data were expressed as cpm. Imipramine (at 1 µM final concentration) was used to determine the non-specific binding.

### Data analysis and results

Data from assays in the presence of compound were calculated as a percentage of total binding measured in the absence of test compound. Inhibition curves, plotting percent of total binding versus the log value of the concentration of the test compound, were automatically generated, and sigmoidal inhibition curves were fitted using non-linear regression. The pIC₅₀ values of test compounds were derived from individual curves.

All compounds according to Formula (I) produced an inhibition at least at the hα_{2C} site (but often also at the hα_{2A} and hα_{2B}- sites) of more than 50 % (pIC₅₀) at a test concentration ranging between 10⁻⁶ M and 10⁻⁹ M in a concentration-dependent manner.

For a selected number of compounds, covering most of the various embodiments of Formula (I), the results of the *in vitro* studies are given in Table 10.

**Table 10. Pharmacological data for the compounds according to the invention.**

| **Co_{.}Nr.** | **h-α2A pIC₅₀** | **h-α2C pIC₅₀** | **h-5HTT pIC₅₀** |
|---|---|---|---|
| **1-80** | 6.6 | 8.6 | 8.5 |
| **2-13** | 7.2 | 8.4 | 8.2 |
| **2-18** | 7.1 | 8.4 | 8.4 |
| **6-15** | 7.0 | 8.4 | 8.0 |
| **2-19** | 6.9 | 8.4 | 7.9 |
| **3-13** | 6.7 | 8.4 | 8.3 |
| **3-14** | 6.6 | 8.4 | 7.8 |
| **1-100** | 6.3 | 8.4 | 7.9 |
| **6-12** | 7.5 | 8.3 | 7.4 |
| **1-96** | 7.1 | 8.3 | 8.0 |
| **1-79a** | 6.8 | 8.3 | 8.5 |
| **6-14** | 7.1 | 8.2 | 7.8 |
| **1-07** | 6.9 | 8.2 | 8.1 |
| **1-79b** | 6.8 | 8.2 | 8.6 |
| **3-07** | 6.8 | 8.2 | 7.7 |
| **2-17** | 6.6 | 8.2 | 8.6 |
| **2-12** | 7.3 | 8.1 | 8.4 |
| **1-58** | 7.1 | 8.1 | 8.2 |
| **1-08** | 6.9 | 8.1 | 8.0 |
| **1-14** | 6.9 | 8.1 | 7.8 |
| **1-26** | 6.4 | 8.1 | 8.1 |
| **2-20** | 6.4 | 8.1 | 7.8 |
| **2-06** | 6.4 | 8.1 | 6.5 |
| **1-76** | 6.2 | 8.1 | 8.2 |
| **1-99** | 6.2 | 8.1 | 7.7 |
| **1-23** | 7.4 | 8.0 | 8.2 |
| **6-13** | 6.9 | 8.0 | 7.6 |
| **2-14** | 6.8 | 8.0 | 8.3 |
| **1-84** | 6.7 | 8.0 | 8.5 |
| **2-11** | 6.7 | 8.0 | 8.1 |
| **1-78** | 6.7 | 8.0 | 7.8 |
| **1-06** | 6.6 | 8.0 | 7.7 |
| **9-10** | 6.2 | 8.0 | 6.2 |
| **1-02** | 7.3 | 7.9 | 7.2 |
| **1-44** | 7.2 | 7.9 | 8.1 |
| **3-08** | 7.1 | 7.9 | 8.0 |
| **1-17** | 7.1 | 7.9 | 7.9 |
| **1-82** | 6.8 | 7.9 | 8.3 |
| **1-61** | 6.7 | 7.9 | 8.0 |
| **1-89** | 6.7 | 7.9 | 7.8 |
| **1-32** | 6.7 | 7.9 | 7.5 |
| **1-90** | 6.6 | 7.9 | 8.1 |
| **1-87** | 6.6 | 7.9 | 7.9 |
| **1-97** | 6.5 | 7.9 | 7.8 |
| **1-66** | 6.5 | 7.9 | 7.2 |
| **9-11** | 6.5 | 7.9 | 6.7 |
| **1-05** | 7.4 | 7.8 | 7.7 |
| **1-39** | 7.1 | 7.8 | 7.2 |
| **2-02** | 6.9 | 7.8 | 8.3 |
| **1-98** | 6.8 | 7.8 | 8.3 |
| **1-22** | 6.7 | 7.8 | 7.8 |
| **1-21** | 6.6 | 7.8 | 7.8 |
| **1-57** | 6.6 | 7.8 | 7.3 |
| **1-92** | 6.5 | 7.8 | 7.9 |
| **1-01** | 6.7 | 7.7 | 7.7 |
| **1-69** | 6.7 | 7.7 | 7.3 |
| **1-104** | 6.7 | 7.7 | 6.9 |
| **1-68** | 6.7 | 7.7 | 6.8 |
| **1-93** | 6.5 | 7.7 | 7.8 |
| **1-71** | 6.5 | 7.7 | 6.9 |
| **1-95** | 6.4 | 7.7 | 8.2 |
| **1-11** | 6.3 | 7.7 | 7.7 |
| **1-53** | 6.2 | 7.7 | 8.1 |
| **6-16** | 7.2 | 7.6 | 6.8 |
| **1-88** | 7.1 | 7.6 | 8.5 |
| **2-03** | 7.0 | 7.6 | 7.9 |
| **2-08** | 7.0 | 7.6 | 7.2 |
| **2-04** | 6.9 | 7.6 | 7.5 |
| **1-63** | 6.8 | 7.6 | 7.2 |
| **1-83** | 6.7 | 7.6 | 8.3 |
| **1-60** | 6.7 | 7.6 | 8.0 |
| **2-10** | 6.7 | 7.6 | 6.8 |
| **1-56** | 6.6 | 7.6 | 7.8 |
| **3-11** | 6.6 | 7.6 | 7.7 |
| **1-86** | 6.6 | 7.6 | 7.6 |
| **3-01** | 6.6 | 7.6 | 7.0 |
| **1-04** | 6.6 | 7.6 | 6.6 |
| **1-24** | 6.5 | 7.6 | 8.2 |
| **1-35** | 6.5 | 7.6 | 7.4 |
| **1-16** | 6.5 | 7.6 | 6.6 |
| **1-77** | 6.4 | 7.6 | 7.2 |
| **1-20** | 6.4 | 7.6 | 7.0 |
| **1-101** | 6.3 | 7.6 | 8.2 |
| **9-16** | 6.3 | 7.6 | 6.0 |
| **1-67** | 6.2 | 7.6 | 6.8 |
| **4-05** | 7.7 | 7.5 | 8.0 |
| **6-02** | 6.9 | 7.5 | 7.6 |
| **1-64** | 6.8 | 7.5 | 6.9 |
| **1-81** | 6.8 | 7.5 | 6.2 |
| **1-72** | 6.7 | 7.5 | 7.2 |
| **1-12** | 6.7 | 7.5 | 6.6 |
| **2-01** | 6.6 | 7.5 | 7.7 |
| **9-02** | 6.6 | 7.5 | 7.3 |
| **2-15** | 6.6 | 7.5 | 6.7 |
| **1-31** | 6.5 | 7.5 | 8.1 |
| **1-70** | 6.5 | 7.5 | 8.0 |
| **1-30** | 6.5 | 7.5 | 7.8 |
| **1-10** | 6.4 | 7.5 | 7.8 |
| **1-45** | 6.4 | 7.5 | 7.6 |
| **1-42** | 6.3 | 7.5 | 8.3 |
| **1-28** | 6.3 | 7.5 | 8.1 |
| **1-47** | 6.3 | 7.5 | 7.6 |
| **1-103** | 6.2 | 7.5 | 6.8 |
| **1-74** | 6.1 | 7.5 | 6.9 |
| **9-30** | 5.7 | 7.5 | 8.1 |
| **6-03** | 7.3 | 7.4 | 7.3 |
| **8-11** | 6.9 | 7.4 | 7.5 |
| **6-05** | 6.9 | 7.4 | 7.4 |
| **9-08** | 6.9 | 7.4 | 6.2 |
| **1-65** | 6.8 | 7.4 | 6.8 |
| **1-09** | 6.7 | 7.4 | 7.7 |
| **1-18** | 6.7 | 7.4 | 7.1 |
| **5-04** | 6.7 | 7.4 | 6.4 |
| **1-43** | 6.6 | 7.4 | 8.2 |
| **9-03** | 6.6 | 7.4 | 7.4 |
| **2-05** | 6.6 | 7.4 | 6.7 |
| **1-48** | 6.5 | 7.4 | 8.0 |
| **1-59** | 6.5 | 7.4 | 7.5 |
| **1-29** | 6.4 | 7.4 | 7.7 |
| **1-73** | 6.4 | 7.4 | 6.8 |
| **1-49** | 6.3 | 7.4 | 7.4 |
| **1-46** | 6.3 | 7.4 | 7.2 |
| **1-37** | 6.2 | 7.4 | 7.9 |
| **1-25** | 6.2 | 7.4 | 7.9 |
| **5-08** | 7.0 | 7.3 | 7.8 |
| **8-09** | 7.0 | 7.3 | 7.3 |
| **3-10** | 6.9 | 7.3 | 7.8 |
| **1-03** | 6.7 | 7.3 | 6.7 |
| **6-04** | 6.6 | 7.3 | 7.5 |
| **9-09** | 6.5 | 7.3 | 6.9 |
| **1-62** | 6.4 | 7.3 | 7.5 |
| **6-06** | 6.4 | 7.3 | 7.0 |
| **1-36** | 6.3 | 7.3 | 6.8 |
| **1-94** | 6.2 | 7.3 | 5.7 |
| **1-75** | 7.0 | 7.2 | 8.4 |
| **5-02** | 6.8 | 7.2 | 7.0 |
| **3-06** | 6.7 | 7.2 | 7.1 |
| **6-07** | 6.7 | 7.2 | 7.0 |
| **6-01** | 6.6 | 7.2 | 7.7 |
| **6-10** | 6.6 | 7.2 | 6.9 |
| **1-102** | 6.5 | 7.2 | 7.8 |
| **1-91** | 6.5 | 7.2 | 7.8 |
| **5-10** | 6.5 | 7.2 | 7.0 |
| **5-11** | 6.4 | 7.2 | 7.4 |
| **1-19** | 6.4 | 7.2 | 7.1 |
| **3-03** | 6.4 | 7.2 | 6.8 |
| **1-38** | 6.4 | 7.2 | 5.8 |
| **1-52** | 6.3 | 7.2 | 8.1 |
| **9-12** | 6.3 | 7.2 | 6.1 |
| **1-13** | 6.3 | 7.2 | 5.7 |
| **1-27** | 6.2 | 7.2 | 7.7 |
| **1-41** | 6.2 | 7.2 | 7.2 |
| **9-07** | 5.8 | 7.2 | 7.3 |
| **9-18** | 5.7 | 7.2 | 6.0 |
| **3-05** | 7.0 | 7.1 | 7.4 |
| **8-12** | 7.0 | 7.1 | 6.9 |
| **8-10** | 6.8 | 7.1 | 7.5 |
| **5-09** | 6.7 | 7.1 | 7.3 |
| **1-51** | 6.4 | 7.1 | 7.8 |
| **2-07** | 6.4 | 7.1 | 7.2 |
| **1-33** | 6.3 | 7.1 | 7.6 |
| **4-04** | 6.3 | 7.1 | 6.8 |
| **9-01** | 6.2 | 7.1 | 7.5 |
| **3-12** | 6.2 | 7.1 | 7.1 |
| **1-85** | 6.1 | 7.1 | 7.5 |
| **4-02** | 6.1 | 7.1 | 6.6 |
| **3-04** | 6.0 | 7.1 | 6.4 |
| **5-03** | 6.7 | 7.0 | 6.0 |
| **1-34** | 6.6 | 7.0 | 7.1 |
| **3-09** | 6.5 | 7.0 | 7.9 |
| **1-55** | 6.2 | 7.0 | 7.2 |
| **3-02** | 6.2 | 7.0 | 6.7 |
| **7-10** | 6.2 | 7.0 | 6.7 |
| **6-11** | 6.1 | 7.0 | 6.9 |
| **4-03** | 6.0 | 7.0 | 6.5 |
| **1-40** | 5.9 | 7.0 | 7.3 |
| **4-01** | 5.9 | 7.0 | 6.7 |
| **6-09** | 6.7 | 6.9 | 7.3 |
| **5-13** | 6.7 | 6.9 | 6.9 |
| **7-09** | 6.5 | 6.9 | 6.8 |
| **1-15** | 6.4 | 6.9 | 5.5 |
| **8-14** | 6.3 | 6.9 | 7.3 |
| **8-13** | 6.3 | 6.9 | 6.9 |
| **5-01** | 6.3 | 6.9 | 5.9 |
| **2-16** | 6.3 | 6.9 | 5.9 |
| **7-04** | 6.2 | 6.9 | 6.4 |
| **9-14** | 6.2 | 6.9 | 5.4 |
| **7-05** | 6.1 | 6.9 | 6.2 |
| **9-17** | 5.4 | 6.9 | 5.6 |
| **8-15** | 6.5 | 6.8 | 6.9 |
| **5-07** | 6.5 | 6.8 | 6.8 |
| **8-06** | 6.3 | 6.8 | 7.1 |
| **8-07** | 6.3 | 6.8 | 7.0 |
| **7-02** | 6.3 | 6.8 | 6.6 |
| **9-15** | 6.2 | 6.8 | 6.2 |
| **2-09** | 6.0 | 6.8 | 6.3 |
| **9-04** | 5.9 | 6.8 | 7.5 |
| **8-08** | 6.5 | 6.7 | 7.2 |
| **5-05** | 6.4 | 6.7 | 6.5 |
| **6-08** | 6.2 | 6.7 | 6.7 |
| **7-07** | 6.2 | 6.7 | 6.6 |
| **7-03** | 6.1 | 6.7 | 6.1 |
| **1-54** | 6.0 | 6.7 | 6.8 |
| **1-50** | 5.9 | 6.7 | 7.2 |
| **9-21** | 5.9 | 6.7 | 5.7 |
| **8-03** | 6.7 | 6.6 | 6.9 |
| **5-06** | 6.6 | 6.6 | 6.6 |
| **8-05** | 6.3 | 6.6 | 6.9 |
| **7-06** | 5.9 | 6.6 | 6.0 |
| **9-06** | 5.9 | 6.5 | 7.4 |
| **9-25** | 6.2 | 6.4 | 5.9 |
| **9-20** | 5.5 | 6.4 | 6.4 |
| **9-13** | 5.5 | 6.4 | < 5.0 |
| **9-24** | 5.3 | 6.4 | < 5.0 |
| **9-29** | 6.2 | 6.3 | < 5.0 |
| **7-08** | 5.8 | 6.3 | 5.9 |
| **9-19** | 5.4 | 6.2 | 5.8 |
| **9-28** | < 5.0 | 6.2 | 6.7 |
| **9-05** | 5.8 | 6.1 | 7.6 |
| **7-01** | 5.8 | 6.1 | 6.3 |
| **9-22** | 5.4 | 6.1 | 5.3 |
| **9-27** | < 5.0 | 6.1 | 6.2 |
| **9-23** | 6.4 | 6.0 | 5.9 |
| **9-26** | 6.3 | 6.0 | 5.6 |
| **8-04** | 5.9 | 5.9 | 6.1 |
| **8-01** | 5.5 | 5.8 | 6.2 |
| **8-02** | 5.9 | 5.7 | 5.7 |

### D. Composition examples

"Active ingredient" (a.i.) as used throughout these examples relates to a compound of formula (i), the pharmaceutically acceptable acid or base addition salts thereof, the stereochemically isomeric forms thereof, the *N-*oxide form thereof, a quaternary ammonium salt thereof and prodrugs thereof.

### Example D.1 : oral drops

500 Grams of the a.i. is dissolved in 0.5 l of 2-hydroxypropanoic acid and 1.5 l of the polyethylene glycol at 60-80 °C. After cooling to 30-40 °C there are added 35 l of polyethylene glycol and the mixture is stirred well. Then there is added a solution of 1750 grams of sodium saccharin in 2.5 l of purified water and while stirring there are added 2.5 l of cocoa flavor and polyethylene glycol q.s. to a volume of 50 l, providing an oral drop solution comprising 10 mg/ml of a.i. The resulting solution is filled into suitable containers.

### Example D.2 : oral solution

9 Grams of methyl 4-hydroxybenzoate and 1 gram of propyl 4-hydroxybenzoate are dissolved in 4 l of boiling purified water. In 3 l of this solution are dissolved first 10 grams of 2,3-dihydroxybutanedioic acid and thereafter 20 grams of the a.i. The latter solution is combined with the remaining part of the former solution and 12 l 1,2,3-propanetriol and 3 l of sorbitol 70 % solution are added thereto. 40 Grams of sodium saccharin are dissolved in 0.5 l of water and 2 ml of raspberry and 2 ml of gooseberry essence are added. The latter solution is combined with the former, water is added q.s. to a volume of 20 l providing an oral solution comprising 5 mg of the active ingredient per teaspoonful (5 ml). The resulting solution is filled in suitable containers.

### Example D.3: film-coated tablets

### Preparation of tablet core

A mixture of 100 grams of the a.i., 570 grams lactose and 200 grams starch is mixed well and thereafter humidified with a solution of 5 grams sodium dodecyl sulfate and 10 grams polyvinylpyrrolidone in about 200 ml of water. The wet powder mixture is sieved, dried and sieved again. Then there is added 100 grams microcrystalline cellulose and 15 grams hydrogenated vegetable oil. The whole is mixed well and compressed into tablets, giving 10,000 tablets, each containing 10 mg of the active ingredient.

### Coating

To a solution of 10 grams methyl cellulose in 75 ml of denaturated ethanol there is added a solution of 5 grams of ethyl cellulose in 150 ml of dichloromethane. Then there are added 75 ml of dichloromethane and 2.5 ml 1,2,3-propanetriol. 10 grams of polyethylene glycol is molten and dissolved in 75 ml of dichloromethane. The latter solution is added to the former and then there are added 2.5 grams of magnesium octadecanoate, 5 grams of polyvinylpyrrolidone and 30 ml of concentrated color suspension and the whole is homogenated. The tablet cores are coated with the thus obtained mixture in a coating apparatus.

### Example D.4 : injectable solution

1.8 grams methyl 4-hydroxybenzoate and 0.2 grams propyl 4-hydroxybenzoate are dissolved in about 0.5 l of boiling water for injection. After cooling to about 50 °C there are added while stirring 4 grams lactic acid, 0.05 grams propylene glycol and 4 grams of the a.i.. The solution is cooled to room temperature and supplemented with water for injection q.s. ad 1 l, giving a solution comprising 4 mg/ml of a.i.. The solution is sterilized by filtration and filled in sterile containers.

### Physico-chemical data

### General procedure

The HPLC gradient was supplied by a HP 1100 from Agilent Technologies comprising a quaternary pump with degasser, an autosampler, a column oven (set at 40 °C) and diode-array detector (DAD). Flow from the column was split to a MS detector. The MS detector was configured with an electrospray ionization source. Nitrogen was used as the nebulizer gas. The source temperature was maintained at 140 °C. Data acquisition was performed with Mass-Lynx-Openlynx software.

### E.1 LCMS - PROCEDURE 1

In addition to the general procedure: Reversed phase HPLC was carried out on an XDB-C18 cartridge (3.5 µm, 4.6 x 30 mm) from Agilent, with a flow rate of 1 ml/min. The gradient conditions used are: 80 % A (0.5 g/l ammonium acetate solution), 10 % B (acetonitrile), 10 % C (methanol) to 50 % B and 50 % C in 6.0 minutes, to 100 % B at 6.5 minutes, kept till 7.0 minutes and equilibrated to initial conditions at 7.6 minutes until 9.0 minutes. Injection volume 5 µl. High-resolution mass spectra (Time of Flight, TOF) were acquired by scanning from 100 to 750, for example in 1.0 second using a dwell time of 1.0 second. The capillary needle voltage was 2.5 kV for positive ionization mode and 2.9 kV for negative ionization mode. The cone voltage was 20 V for both positive and negative ionization modes. Leucine-enkephaline was the standard substance used for the lock mass calibration.

### E.2 LCMS - PROCEDURE 2

In addition to the general procedure: Reversed phase HPLC was carried out on an XDB-C18 cartridge (3.5 µm, 4.6 x 30 mm) from Agilent, with a flow rate of 1 ml/min. The gradient conditions used are: 80 % A (0.5 g/l ammonium acetate solution), 10 % B (acetonitrile), 10 % C (methanol) to 50 % B and 50 % C in 6.0 minutes, to 100 % B at 6.5 minutes, kept till 7.0 minutes and equilibrated to initial conditions at 7.6 minutes until 9.0 minutes. Injection volume 5 µl. High-resolution mass spectra (Time of Flight, TOF) were acquired only in positive ionization mode by scanning from 100 to 750 in 0.5 seconds using a dwell time of 0.1 seconds. The capillary needle voltage was 2.5 kV and the cone voltage was 20 V. Leucine-enkephaline was the standard substance used for the lock mass calibration.

### E.3 LCMS - PROCEDURE 3

In addition to the general procedure: Reversed phase HPLC was carried out on an XDB-C18 cartridge (3.5 µm, 4.6 x 30 mm) from Agilent, with a flow rate of 1 ml/min. The gradient conditions used are: 80 % A (0.5 g/l ammonium acetate solution), 10 % B (acetonitrile), 10 % C (methanol) to 50 % B and 50 % C in 6.0 minutes, to 100 % B at 6.5 minutes, kept till 7.0 minutes and equilibrated to initial conditions at 7.6 minutes until 9.0 minutes. Injection volume 5 µl. High-resolution mass spectra (Time of Flight, TOF) were acquired by scanning from 100 to 750 in 0.5 seconds using a dwell time of 0.3 seconds. The capillary needle voltage was 2.5 kV for positive ionization mode and 2.9 kV for negative ionization mode. The cone voltage was 20 V for both positive and negative ionization modes. Leucine-enkephaline was the standard substance used for the lock mass calibration.

### E.4 LCMS- PROCEDURE 4

In addition to the general procedure: Reversed phase HPLC was carried out on an ACE-C18 column (3.0 µm, 4.6 x 30 mm) from Advanced Chromatography Technologies, with a flow rate of 1.5 ml/min. The gradient conditions used are: 80 % A (0.5 g/l ammonium acetate solution), 10 % B (acetonitrile), 10 % C (methanol) to 50 % B and 50 % C in 6.5 minutes, to 100 % B at 7 minutes and equilibrated to initial conditions at 7.5 minutes until 9.0 minutes. Injection volume 5 µl. High-resolution mass spectra (Time of Flight, TOF) were acquired only in positive ionization mode by scanning from 100 to 750 in 0.5 seconds using a dwell time of 0.1 seconds. The capillary needle voltage was 2.5 kV for positive ionization mode and the cone voltage was 20 V. Leucine-enkephaline was the standard substance used for the lock mass calibration.

### E.5 LCMS - PROCEDURE 5

In addition to the general procedure: Same as procedure 4 but an injection volume of 10 µL was used.

### E.6 LCMS - PROCEDURE 6

In addition to the general procedure: Reversed phase HPLC was carried out on an XDB-C8 cartridge (3.5 µm, 4.6 x 30 mm) from Agilent, with a flow rate of 1 ml/min. The gradient conditions used are: 80 % A (0.5 g/l ammonium acetate solution), 10 % B (acetonitrile), 10 % C (methanol) to 50 % B and 50 % C in 6.0 minutes, to 100 % B at 6.5 minutes, kept till 7.0 minutes and equilibrated to initial conditions at 7.6 minutes until 9.0 minutes. Injection volume 5 µl. High-resolution mass spectra (Time of Flight, TOF) were acquired by scanning from 100 to 750 in 0.5 seconds using a dwell time of 0.3 seconds. The capillary needle voltage was 2.5 kV for positive ionization mode and 2.9 kV for negative ionization mode. The cone voltage was 20 V for both positive and negative ionization modes. Leucine-enkephaline was the standard substance used for the lock mass calibration.

### E.7 LCMS - PROCEDURE 7

In addition to the general procedure: Same as procedure 2 but an injection volume of 10 µL was used.

### E.8 LCMS - PROCEDURE 8

In addition to the general procedure: Reversed phase HPLC was carried out on an XDB-C18 cartridge (3.5 µm, 4.6 x 30 mm) from Agilent, with a flow rate of 1 ml/min. The gradient conditions used are: 80 % A (0.5 g/l ammonium acetate solution), 10 % B (acetonitrile), 10 % C (methanol) to 50 % B and 50 % C in 6.0 minutes, to 100 % B at 6.5 minutes, kept till 7.0 minutes and equilibrated to initial conditions at 7.6 minutes until 9.0 minutes. Injection volume 5 µl. Low-resolution mass spectra (ZQ detector, quadrupole) were acquired by scanning from 100 to 1000 in 1.0 second using a dwell time of 0.3 seconds. The capillary needle voltage was 3 kV. The cone voltage was 20 V and 50 V for positive ionization mode and 20 V for negative ionization mode.

### E.9 LCMS - PROCEDURE 9

In addition to the general procedure: Reversed phase HPLC was carried out on an XT-C18 column (3.5 µm, 4.6 x 30 mm) from Waters, with a flow rate of 1 ml/min. The gradient conditions used are: 80 % A (1 g/l ammonium bicarbonate solution), 10 % B (acetonitrile), 10 % C (methanol) to 50 % B and 50 % C in 6.0 minutes, to 100 % B at 6.5 minutes, kept till 7.0 minutes and equilibrated to initial conditions at 7.6 minutes until 9.0 minutes. Injection volume 5 µl.
High-resolution mass spectra (Time of Flight, TOF) were acquired only in positive ionization mode by scanning from 100 to 750 in 0.5 seconds using a dwell time of 0.1 seconds. The capillary needle voltage was 2.5 kV and the cone voltage was 20 V. Leucine-enkephaline was the standard substance used for the lock mass calibration.

### E.10 Melting points

For a number of compounds, melting points were determined in open capillary tubes on a Mettler FP62 apparatus. Melting points were measured with a temperature gradient of 3 or 10 °C/minute. Maximum temperature was 300 °C. The melting point was read from a digital display.

**Table 11: Analytical data**

| **Co. Nr.** | **Rₜ** | **(MH)⁺** | **Procedure** | **Melting point (°C)** |
|---|---|---|---|---|
| **1-01** | 4.15 | 440 | 2 | |
| **1-02** | 3.80 | 454 | 1 | |
| **1-03** | 3.03 | 392 | 1 | |
| **1-04** | 3.26 | 406 | 6 | |
| **1-05** | 5.86 | 482 | 4 | 117.5 |
| **1-06** | 4.61 | 482 | 2 | |
| **1-07** | 4.53 | 486 | 4 | |
| **1-08** | 4.79 | 504 | 4 | |
| **1-09** | 5.15 | 496 | 2 | 210.1 |
| **1-10** | 4.39 | 496 | 4 | |
| **1-11** | 4.72 | 510 | 4 | |
| **1-12** | 3.51 | 420 | 6 | |
| **1-13** | 4.52 | 448 | 1 | |
| **1-14** | 5.15 | 500 | 4 | |
| **1-15** | 3.63 | 420 | 1 | |
| **1-16** | 5.27 | 488 | 1 | |
| **1-17** | 5.40 | 522 | 7 | |
| **1-18** | 5.90 | 538 | 1 | |
| **1-19** | 5.73 | 572 | 1 | |
| **1-20** | 5.96 | 598 | 1 | |
| **1-21** | 5.57 | 512 | 2 | |
| **1-22** | 4.96 | 498 | 2 | |
| **1-23** | 4.90 | 496 | 4 | |
| **1-24** | 5.14 | 510 | 2 | |
| **1-25** | 4.79 | 540 | 4 | |
| **1-26** | 4.84 | 528 | 4 | 95 |
| **1-27** | 6.35 | 580 | 2 | |
| **1-28** | 5.75 | 550 | 2 | |
| **1-29** | 5.93 | 580 | 4 | |
| **1-30** | 4.73 | 526 | 2 | |
| **1-31** | 5.23 | 522 | 2 | |
| **1-32** | 5.91 | 492 | 1 | |
| **1-33** | 5.85 | 540 | 2 | |
| **1-34** | 5.71 | 554 | 1 | |
| **1-35** | 5.79 | 506 | 8 | |
| **1-36** | 5.63 | 526 | 7 | |
| **1-37** | 4.98 | 568 | 2 | |
| **1-38** | 4.20 | 444 | 1 | |
| **1-39** | 5.63 | 525 | 9 | |
| **1-40** | 5.05 | 525 | 2 | |
| **1-41** | 5.95 | 579 | 2 | 104 |
| **1-42** | 5.48 | 558 | 2 | |
| **1-43** | 5.65 | 572 | 2 | |
| **1-44** | 4.57 | 554 | 2 | |
| **1-45** | 4.68 | 541 | 2 | |
| **1-46** | 4.81 | 555 | 2 | |
| **1-47** | 4.77 | 569 | 2 | 185.9 |
| **1-48** | 5.39 | 573 | 2 | |
| **1-49** | 5.71 | 605 | 2 | |
| **1-50** | 5.92 | 659 | 2 | |
| **1-51** | 5.80 | 601 | 2 | 151.2 |
| **1-52** | 5.46 | 559 | 7 | |
| **1-53** | 5.02 | 511 | 7 | |
| **1-54** | 6.37 | 627 | 7 | |
| **1-55** | 5.22 | 638 | 2 | |
| **1-56** | 4.92 | 511 | 9 | |
| **1-57** | 5.24 | 539 | 2 | |
| **1-58** | 4.45 | 567 | 2 | |
| **1-59** | 4.59 | 567 | 2 | |
| **1-60** | 5.01 | 539 | 7 | |
| **1-61** | 4.50 | 525 | 7 | |
| **1-62** | 4.38 | 567 | 2 | |
| **1-63** | 5.10 | 482 | 1 | |
| **1-64** | 4.81 | 468 | 1 | |
| **1-65** | 4.91 | 486 | 1 | |
| **1-66** | 4.88 | 500 | 1 | |
| **1-67** | 4.83 | 482 | 2 | |
| **1-68** | 4.73 | 482 | 1 | |
| **1-69** | 5.02 | 496 | 1 | |
| **1-70** | 6.69 | 544 | 2 | |
| **1-71** | 5.03 | 496 | 1 | |
| **1-72** | 5.26 | 510 | 1 | |
| **1-73** | 5.64 | 524 | 1 | |
| **1-74** | 4.97 | 496 | 2 | |
| **1-75** | 5.26 | 548 | 4 | |
| **1-76** | 5.03 | 562 | 4 | |
| **1-77** | 5.20 | 512 | 7 | |
| **1-78** | 5.12 | 510 | 4 | > 300 |
| **1-79a** | 5.13 | 524 | 2 | > 300 |
| **1-79b** | 5.28 | 524 | 2 | |
| **1-80** | 5.00 | 542 | 4 | |
| **1-81** | 4.42 | 472 | 1 | |
| **1-82** | 5.64 | 520 | 2 | |
| **1-83** | 5.92 | 534 | 2 | |
| **1-84** | 5.56 | 552 | 4 | |
| **1-85** | 6.98 | 628 | 2 | |
| **1-86** | 4.49 | 549 | 5 | |
| **1-87** | 5.09 | 553 | 4 | |
| **1-88** | 5.74 | 565 | 9 | 172.3 |
| **1-89** | 5.32 | 555 | 5 | |
| **1-90** | 5.23 | 531 | 7 | |
| **1-91** | 4.73 | 567 | 7 | 271 |
| **1-92** | 4.96 | 539 | 5 | 195.3 |
| **1-93** | 4.68 | 553 | 2 | |
| **1-94** | 3.89 | 505 | 1 | |
| **1-95** | 5.36 | 564 | 2 | |
| **1-96** | 4.94 | 615 | 4 | |
| **1-97** | 4.58 | 567 | 5 | 183.4 |
| **1-98** | 4.67 | 566 | 2 | |
| **1-99** | 4.52 | 538 | 4 | 84 |
| **1-100** | 4.48 | 535 | 4 | |
| **1-101** | 4.86 | 545 | 2 | |
| **1-102** | 5.76 | 617 | 2 | 117.7 |
| **1-103** | 5.61 | 550 | 7 | |
| **1-104** | 5.84 | 558 | 1 | |
| **2-01** | 5.04 | 508 | 2 | |
| **2-02** | 5.60 | 523 | 9 | |
| **2-03** | 5.49 | 509 | 2 | |
| **2-04** | 4.69 | 523 | 7 | |
| **2-05** | 5.20 | 537 | 1 | |
| **2-06** | 5.28 | 551 | 1 | |
| **2-07** | 5.72 | 565 | 1 | |
| **2-08** | 4.73 | 524 | 2 | 271.4 |
| **2-09** | 4.28 | 448 | 1 | |
| **2-10** | 5.12 | 446 | 1 | 161.2 |
| **2-11** | 5.73 | 508 | 2 | |
| **2-12** | 5.95 | 526 | 7 | |
| **2-13** | 5.54 | 544 | 4 | |
| **2-14** | 4.89 | 524 | 7 | |
| **2-15** | 5.77 | 550 | 1 | |
| **2-16** | 4.98 | 528 | 1 | |
| **2-17** | 5.36 | 601 | 4 | |
| **2-18** | 5.73 | 534 | 2 | 172.8 |
| **2-19** | 5.45 | 522 | 1 | 76.5 |
| **2-20** | 5.45 | 532 | 2 | 96.3 |
| **3-01** | 3.87 | 379 | 3 | |
| **3-02** | 4.02 | 393 | 8 | |
| **3-03** | 4.24 | 407 | 3 | |
| **3-04** | 4.63 | 421 | 8 | |
| **3-05** | 5.57 | 469 | 2 | 196.5 |
| **3-06** | 5.17 | 449 | 8 | |
| **3-07** | 5.64 | 483 | 4 | 159.8 |
| **3-08** | 5.26 | 463 | 1 | |
| **3-09** | 5.75 | 455 | 8 | |
| **3-10** | 5.91 | 469 | 8 | |
| **3-11** | 5.96 | 483 | 8 | |
| **3-12** | 5.42 | 463 | 3 | |
| **3-13** | 4.80 | 554 | 4 | 153.9 |
| **3-14** | 5.22 | 525 | 4 | |
| **3-15** | 3.24 | 455 | 2 | |
| **4-01** | 5.28 | 451 | 3 | |
| **4-02** | 5.53 | 465 | 3 | |
| **4-03** | 4.93 | 437 | 3 | |
| **4-04** | 4.67 | 435 | 8 | |
| **4-05** | 5.49 | 469 | 2 | |
| **5-01** | 3.69 | 335 | 1 | |
| **5-02** | 4.97 | 377 | 1 | |
| **5-03** | 3.95 | 391 | 1 | |
| **5-04** | 5.01 | 433 | 1 | |
| **5-05** | 4.22 | 388 | 8 | |
| **5-06** | 6.11 | 439 | 1 | |
| **5-07** | 6.54 | 579 | 8 | |
| **5-08** | 5.55 | 467 | 3 | |
| **5-09** | 5.29 | 470 | 1 | |
| **5-10** | 5.13 | 439 | 2 | |
| **5-11** | 4.82 | 457 | 4 | |
| **5-13** | 5.84 | 503 | 1 | |
| **6-01** | 5.89 | 418 | 2 | |
| **6-02** | 5.42 | 450 | 2 | |
| **6-03** | 5.42 | 450 | 2 | |
| **6-04** | 5.94 | 467 | 2 | |
| **6-05** | 5.38 | 417 | 2 | 152.8 |
| **6-06** | 4.47 | 412 | 2 | > 300 |
| **6-07** | 4.58 | 412 | 2 | 125.1 |
| **6-08** | 4.31 | 413 | 2 | |
| **6-09** | 5.35 | 462 | 2 | |
| **6-10** | 5.41 | 476 | 1 | |
| **6-11** | 3.33 | 460 | 4 | |
| **6-12** | 4.56 | 510 | 4 | |
| **6-13** | 4.6 | 508 | 5 | |
| **6-14** | 4.57 | 546 | 5 | 134.7 |
| **6-15** | 4.97 | 560 | 4 | |
| **6-16** | 5.00 | 412 | 2 | |
| **7-01** | 6.56 | 523 | 2 | |
| **7-02** | 4.98 | 516 | 4 | |
| **7-03** | 5.38 | 520 | 4 | |
| **7-04** | 5.46 | 558 | 4 | |
| **7-05** | 5.96 | 542 | 4 | |
| **7-06** | 6.06 | 560 | 4 | |
| **7-07** | 5.82 | 597 | 2 | |
| **7-08** | 5.70 | 544 | 4 | |
| **7-09** | 6.07 | 543 | 2 | |
| **7-10** | 5.41 | 557 | 4 | |
| **8-01** | 6.98 | 643 | 2 | |
| **8-02** | 7.04 | 643 | 2 | |
| **8-03** | 6.12 | 595 | 2 | |
| **8-04** | 6.72 | 623 | 2 | |
| **8-05** | 5.83 | 636 | 4 | |
| **8-06** | 6.01 | 640 | 4 | |
| **8-07** | 6.14 | 678 | 4 | |
| **8-08** | 6.52 | 678 | 2 | |
| **8-09** | 6.66 | 662 | 4 | |
| **8-10** | 5.91 | 678 | 4 | |
| **8-11** | 5.89 | 678 | 4 | |
| **8-12** | 6.56 | 680 | 4 | |
| **8-13** | 6.72 | 717 | 2 | |
| **8-14** | 6.15 | 677 | 4 | |
| **8-15** | 6.3 | 664 | 4 | |
| **9-01** | 5.63 | 508 | 2 | |
| **9-02** | 5.19 | 551 | 2 | |
| **9-03** | 6.22 | 599 | 3 | |
| **9-04** | 5.36 | 600 | 2 | |
| **9-05** | 4.38 | 468 | 2 | |
| **9-06** | 4.93 | 484 | 2 | |
| **9-07** | 4.23 | 523 | 2 | |
| **9-08** | 4.85 | 468 | 2 | |
| **9-09** | 5.72 | 600 | 2 | |
| **9-10** | 4.64 | 523 | 2 | |
| **9-11** | 4.75 | 533 | 2 | |
| **9-12** | 5.30 | 470 | 2 | |
| **9-14** | 4.67 | 464 | 2 | |
| **9-15** | 5.10 | 456 | 2 | |
| **9-16** | 5.34 | 484 | 2 | |
| **9-17** | 4.59 | 523 | 2 | |
| **9-18** | 4.63 | 533 | 2 | |
| **9-19** | 4.74 | 468 | 2 | |
| **9-20** | 5.60 | 600 | 2 | |
| **9-21** | 4.54 | 464 | 2 | |
| **9-22** | 5.23 | 484 | 2 | |
| **9-23** | 4.45 | 440 | 1 | |
| **9-24** | 4.46 | 406 | 1 | |
| **9-25** | 6.03 | 540 | 1 | |
| **9-26** | 5.93 | 411 | 1 | |
| **9-27** | 4.78 | 523 | 2 | |
| **9-28** | 5.76 | 600 | 2 | |
| **9-29** | 4.87 | 412 | 1 | |
| **9-30** | 4.37 | 533 | 2 | |

## Claims

1. Compound according to the general Formula (I) 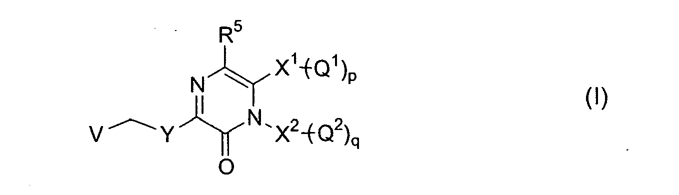 a pharmaceutically acceptable acid or base addition salt thereof, a stereochemically isomeric form thereof, an *N*-oxide form thereof or a quaternary ammonium salt thereof, wherein :
V is a naphthyl-radical, wherein one CH-unit in the napthyl-moiety may optionally be replaced by a *N*-atom;
Y is a bivalent radical of Formula (II) 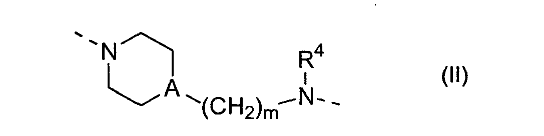
wherein
A is a nitrogen or a carbon-atom ;
m is an integer equal to zero, 1 or 2 ;
R⁴ is selected from the group of hydrogen ; alkyl and phenylcarboxylalkyl ;
R⁵ is selected from the group of hydrogen and halo ;
X¹, X² are each, independently from each other, a covalent bond, a saturated or an unsaturated (C₁₋₈)-hydrocarbon radical, wherein one or more bivalent -CH₂-units and/or one or more monovalent CH₃-units may optionally be replaced by a respective bivalent or monovalent phenyl-unit ; and wherein one or more hydrogen atoms may be replaced by a radical selected from the group of oxo ; (C₁₋₃)alkyloxy ; halo ; cyano ; nitro ; formyl ; hydroxy ; amino ; trifluoromethyl ; mono- and di((C₁₋₃)alkyl)amino ; carboxy; and thio ;
p, q are each, independently from each other, an integer equal to 1 or 2;
Q¹, Q² are each, independently from each other, a radical selected from the group of hydrogen ; -NR¹R²; Pir ; -OR³ and Het; wherein two radicals -OR³ may be taken together to form a bivalent radical -O-(CH₂)ᵣ-O- wherein r is an integer equal to 1, 2 or 3;
R¹ and R² are each, independently from each other, a radical selected from the group of hydrogen ; alkyl ; alkenyl ; alkynyl ; aryl ; arylalkyl ; alkylcarbonyl ; alkenylcarbonyl ; alkyloxy ; alkyloxyalkyl ; alkyloxycarbonyl ; alkyloxyalkylcarbonyl ; alkyloxycarbonylalkyl ; alkyloxycarbonylalkylcarbonyl; alkylsulfonyl ; arylcarbonyl ; aryloxyalkyl ; arylalkylcarbonyl ; arylsulfonyl ; Het ; Het-alkyl ; Het-alkylcarbonyl ; Het-carbonyl ; Het-carbonylalkyl ; alkyl-NR^{a}R^{b} ; carbonyl-NR^{a}R^{b} ; carbonylalkyl-NR^{a}R^{b} ; alkylcarbonyl-NR^{a}R^{b} ; and alkylcarbonylalkyl-NR^{a}R^{b} ; wherein R^{a} and R^{b} are each independently selected from the group of hydrogen, alkyl, alkylcarbonyl, alkyloxyalkyl, alkyloxycarbonylalkyl, aryl, arylalkyl, Het and alkyl-NR^{c}R^{d} , wherein R^{c} and R^{d} are each independently from each other hydrogen or alkyl ;
Pir is a radical containing at least one N, by which it is attached to the X-radical, selected from the group of pyrrolidinyl ; imidazolidinyl ; pyrazolidinyl ; piperidinyl ; piperazinyl ; pyrrolyl ; pyrrolinyl ; imidazolinyl ; pyrrazolinyl ; pyrrolyl ; imidazolyl ; pyrazolyl ; triazolyl ; azepyl ; diazepyl ; morpholinyl ; thiomorpholinyl ; indolyl ; isoindolyl; indolinyl ; indazolyl ; benzimidazolyl ; and 1,2,3,4-tetrahydro-isoquinolinyl ; wherein each Pir-radical is optionally substituted by 1, 2 or 3 radicals selected from the group of hydroxy ; halo ; oxo ; (C₁₋₃)alkyl; trifluoromethyl ; phenyl; benzyl ; pyrrolidinyl ; and pyridinyloxy ;
R³ is a radical selected from the group of hydrogen ; alkyl ; aryl ; arylalkyl ; Het ; and Het-alkyl ;
Het is a heterocyclic radical selected from the group of pyrrolidinyl ; imidazolidinyl ; pyrazolidinyl ; piperidinyl ; piperazinyl ; pyrrolyl ; pyrrolinyl ; imidazolinyl ; pyrrazolinyl ; pyrrolyl ; imidazolyl ; pyrazolyl ; triazolyl ; pyridinyl ; pyridazinyl ; pyrimidinyl ; pyrazinyl ; triazinyl ; azepyl ; diazepyl ; morpholinyl ; thiomorpholinyl ; indolyl ; isoindolyl ; indolinyl ; indazolyl ; benzimidazolyl ; 1,2,3,4-tetrahydro-isoquinolinyl ; furyl ; thienyl ; oxazolyl ; isoxazolyl ; thiazolyl ; thiadiazolyl ; isothiazolyl ; dioxolyl ; dithianyl ; tetrahydrofuryl ; tetrahydropyranyl ; quinolinyl; isoquinolinyl ; quinoxalinyl ; benzoxazolyl ; benzisoxazolyl; benzothiazolyl ; benzisothiazolyl ; benzofuranyl ; benzothienyl ; benzopiperidinyl ; chromenyl ; and imidazo[1,2-a]pyridinyl ; wherein each Het-radical is optionally substituted by one or more radicals selected from the group of halo ; oxo ; (C₁₋₃)alkyl; (C₁₋₃)alkylcarbonyl ; (C₁₋₃)alkenylthio ; imidazolyl-(C₁₋₃)alkyl ; and (C₁₋₃)alkyloxycarbonyl ;
aryl is naphthalenyl or phenyl, each optionally substituted with 1, 2 or 3 substituents, each independently from each other, selected from the group of oxo ; (C₁₋₃)alkyl; (C₁₋₃)alkyloxy ; halo ; cyano ; nitro ; formyl; hydroxy; amino ; trifluoromethyl ; mono- and di((C₁₋₃)alkyl)amino ; carboxy ; and thio ;
alkyl is a straight or branched saturated hydrocarbon radical having from 1 to 8 carbon atoms ; or is a cyclic saturated hydrocarbon radical having from 3 to 7 carbon atoms ; or is a cyclic saturated hydrocarbon radical having from 3 to 7 carbon atoms attached to a straight or branched saturated hydrocarbon radical having from 1 to 8 carbon atoms ; wherein each radical is optionally substituted on one or more carbon atoms with one or more radicals selected from the group of oxo ; (C₁₋₃)alkyloxy ; halo ; cyano ; nitro ; formyl ; hydroxy ; amino ; carboxy ; and thio ;
alkenyl is an alkyl radical as defined above, further having one or more double bonds ;
alkynyl is an alkyl radical as defined above, further having one or more triple bonds ; and
arylalkyl is an alkyl radical as defined above, further having one or more CH₃-groups replaced by phenyl.

2. Compound according to claim 1, **characterized in that** V is selected from the group of radicals (z-1), (z-2), (z-3), (z-4), (z-5) and (z-6).

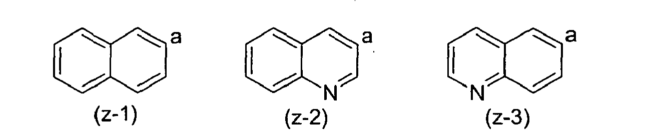

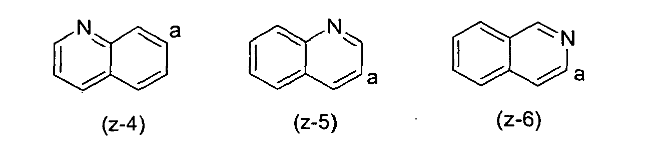

3. Compound according to any one of claims 1 and 2, **characterized in that** A is a carbon atom, m is zero and R⁴ is hydrogen.

4. Compound according to any one of claims 1 to 3, **characterized in that** the moiety -CH₂-Y- is attached to V by the atom, denoted by "a".

5. Compound according to any one of claims 1 to 4, **characterized in that** R⁵ is chloro.

6. Compound according to any one of claims 1 to 5, **characterized in that** each of X¹ and X², independently from each other, are selected from the group of a covalent bond; -CH₂-; -CH₂CH₂- ; -CH₂CH₂CH₂- ; -CH₂CH₂CH₂CH₂- ; -CH₂CH=CHCH₂-; -CH₂C≡CCH₂- ; -CH(CH₃)CH(CH₃)- ; -C(=O)CH₂- ; -C(=O)CH₂CH₂- ; -C(=O)CH₂CH₂CH₂- ; -CH₂C(=O)- ; -CH₂CH₂C(=O)- ; -CH₂CH₂CH₂C(=O)- ; -CH₂CH₂CH₂CH₂C(=O)- ; -CH₂CH₂C(=O)CH₂- ; -C₆H₄- ; -CH₂C₆H₄- ; -CH₂CH₂C₆H₄- ; -CH₂CH₂CH₂C₆H₄- ; -C₆H₄CH₂- ; -C₆H₄CH₂CH₂- ; -C₆H₄CH₂CH₂CH₂- ; -CH₂C₆H₄CH₂- ; -CH₂CH₂C₆H₄CH₂CH₂- ; -C₆H₄C(=O)- ; -C₆H₄CH₂C(=O)- ; -C₆H₄CH₂CH₂C(=O)-; and -CH₂CH₂C(=O)C₆H₄-.

7. Compound according to any one of claims 1 to 6, **characterized in that** each of X¹ and X², independently from each other, are selected from the group of a covalent bound; -CH₂- ; -CH₂CH₂- ; -CH₂CH₂CH₂- ; -CH₂CH₂CH₂CH₂- ; -CH₂CH=CHCH₂-; -CH₂C=CCH₂- ; -CH₂C(=O)- ; -CH₂CH₂C(=O)- ; -CH₂CH₂CH₂C(=O)- ; -CH₂CH₂CH₂CH₂C(=O)- ; -C₆H₄- ; -CH₂C₆H₄- ; -CH₂CH₂CH₂C₆H₄- ; -C₆H₄CH₂- ; -CH₂C₆H₄CH₂- ; -C₆H₄C(=O)- ; -C₆H₄CH₂C(=O)- ; -C₆H₄CH₂CH₂C(=O)- ; and -CH₂CH₂C(=O)C₆H₄-.

8. Compound according to any one of claims 1 to 7, **characterized in that** one or more hydrogen atoms in each of X¹ and X² are optionally replaced by a radical selected from the group of oxo ; (C₁₋₃)alkyloxy ; halo ; cyano ; nitro ; and formyl.

9. Compound according to any one of claims 1 to 8, **characterized in that** X¹ is a covalent bond, Q¹ is hydrogen and p is 1.

10. Compound according to any one of claims 1 to 9, **characterized in that** R¹ and R² are each, independently from each other, a radical selected from the group of hydrogen ; alkyl ; alkenyl ; alkynyl ; aryl ; arylalkyl ; alkylcarbonyl ; alkenylcarbonyl ; alkyloxyalkyl ; alkyloxycarbonyl ; alkyloxyalkylcarbonyl ; alkyloxycarbonylalkyl ; alkyloxycarbonylalkylcarbonyl ; arylcarbonyl ; aryloxyalkyl ; arylalkylcarbonyl ; Het-alkyl ; Het-alkylcarbonyl; Het-carbonyl ; Het-carbonylalkyl ; alkyl-NR^{a}R^{b} ; carbonyl-NR^{a}R^{b} ; carbonylalkyl-NR^{a}R^{b} ; alkylcarbonyl-NR^{a}R^{b} ; and alkylcarbonylalkyl-NR^{a}R^{b}; wherein each of R^{a} and R^{b} independently are selected from the group of hydrogen, alkyl, alkylcarbonyl, alkyloxyalkyl, alkyloxycarbonylalkyl, aryl, arylalkyl, Het and alkyl-NR^{c}R^{d}, wherein R^{c} and R^{d} are each independently from each other hydrogen or alkyl.

11. Compound according to any one of claims 1 to 10, **characterized in that** Pir is a radical containing at least one N, by which it is attached to the X-radical, selected from the group of pyrrolidinyl ; piperidinyl ; piperazinyl ; imidazolyl; morpholinyl ; isoindolyl ; wherein each Pir-radical is optionally substituted by 1, 2 or 3 radicals selected from the group of hydroxy ; halo ; oxo ; (C₁₋₃)alkyl; trifluoromethyl ; phenyl ; benzyl ; pyrrolidinyl ; and pyridinyloxy.

12. Compound according to any one of claims 1 to 11, **characterized in that** Het is a heterocyclic radical selected from the group of piperidinyl ; piperazinyl ; triazolyl; pyridinyl ; pyrimidinyl ; morpholinyl ; indolyl ; furyl ; thienyl ; isoxazolyl; thiazolyl ; tetrahydrofuryl ; tetrahydropyranyl ; quinolinyl ; isoquinolinyl ; benzofuranyl ; benzothienyl ; and benzopiperidinyl ; wherein each Het-radical is optionally substituted by one or more radicals selected from the group of oxo ; (C₁₋₃)alkyl; (C₁₋₃)alkylcarbonyl ; and imidazolyl-(C₁₋₃)alkyl.

13. Compound according to any one of claims 1 to 12, **characterized in that** aryl is phenyl, optionally substituted with 1, 2 or 3 substituents, each independently from each other, selected from the group of (C₁₋₃)alkyl; halo; and trifluoromethyl.

14. Compound according to claim 1, **characterized in that**:
V is selected from the group of radicals (z-1), (z-2), (z-3), (z-5) and (z-6) ; wherein the moiety -CH₂-Y- is attached to V by the atom, denoted by "a" ;
Y is a bivalent radical of Formula (II) wherein A is a nitrogen or a carbon-atom ; m is an integer equal to zero or 2 ; and R⁴ is selected from the group of hydrogen ; alkyl and phenylcarboxylalkyl ;
R⁵ is selected from the group of hydrogen and halo ;
X¹, X² are each, independently from each other, are selected from the group of a covalent bond ; --CH₂-; -CH₂CH₂- ; -CH₂CH₂CH₂- ; -CH₂CH₂CH₂CH₂- ; -CH₂CH=CHCH₂-; -CH₂C≡CCH₂- ; -CH₂C(=O)- ; -CH₂CH₂C(=O)- ; -CH₂CH₂CH₂C(=O)- ; -CH₂CH₂CH₂CH₂C(=O)- ; -C₆H₄- ; -CH₂C₆H₄- ; -CH₂CH₂CH₂C₆H₄-; -C₆H₄CH₂- ; -CH₂C₆H₄CH₂- ; -C₆H₄C(=O)- ; -C₆H₄CH₂C(=O)- ; -C₆H₄CH₂CH₂C(=O)- ; and -CH₂CH₂C(=O)C₆H₄- ; and wherein one or more hydrogen atoms may be replaced by a radical selected from the group of oxo ; (C₁₋₃)alkyloxy ; halo ; cyano ; nitro; and formyl ;
p, q are each, independently from each other, an integer equal to 1 or 2;
Q¹, Q² are each, independently from each other, a radical selected from the group of hydrogen ; -NR¹R² ; Pir ; -OR³ and Het; wherein two radicals -OR³ may be taken together to form a bivalent radical -O-(CH₂)ᵣ-O- wherein r is an integer equal to 1, 2 or 3 ;
R¹ and R² are each, independently from each other, a radical selected from the group of hydrogen ; alkyl ; alkenyl ; alkynyl ; aryl ; arylalkyl ; alkylcarbonyl ; alkenylcarbonyl ; alkyloxyalkyl ; alkyloxycarbonyl ; alkyloxyalkylcarbonyl ; alkyloxycarbonylalkyl ; alkyloxycarbonylalkylcarbonyl; arylcarbonyl ; aryloxyalkyl ; arylalkylcarbonyl ; Het-alkyl ; Het-alkylcarbonyl ; Het-carbonyl ; Het-carbonylalkyl ; alkyl-NR^{a}R^{b} ; carbonyl-NR^{a}R^{b} ; carbonylalkyl-NR^{a}R^{b} ; alkylcarbonyl-NR^{a}R^{b} ; and alkylcarbonylalkyl-NR^{a}R^{b} ; wherein R^{a} and R^{b} are each independently selected from the group of hydrogen, alkyl, alkylcarbonyl, alkyloxyalkyl, alkyloxycarbonylalkyl, aryl, arylalkyl, Het and alkyl-NR^{c}R^{d} , wherein R^{c} and R^{d} are each independently from each other hydrogen or alkyl ;
Pir is a radical containing at least one N, by which it is attached to the X-radical, selected from the group of pyrrolidinyl ; piperidinyl ; piperazinyl ; imidazolyl; morpholinyl ; isoindolyl ; wherein each Pir-radical is optionally substituted by 1, 2 or 3 radicals selected from the group of hydroxy; halo ; oxo ; (C₁₋₃)alkyl; trifluoromethyl ; phenyl ; benzyl ; pyrrolidinyl ; and pyridinyloxy ;
R³ is a radical selected from the group of hydrogen ; alkyl ; aryl ; arylalkyl; Het ; and Het-alkyl ;
Het is a heterocyclic radical selected from the group of piperidinyl ; piperazinyl ; triazolyl; pyridinyl ; pyrimidinyl ; morpholinyl ; indolyl ; furyl ; thienyl ; isoxazolyl; thiazolyl ; tetrahydrofuryl ; tetrahydropyranyl ; quinolinyl ; isoquinolinyl ; benzofuranyl ; benzothienyl ; and benzopiperidinyl ; wherein each Het-radical is optionally substituted by one or more radicals selected from the group of oxo ; (C₁₋₃)alkyl; (C₁₋₃)alkylcarbonyl ; and imidazolyl-(C₁₋₃)alkyl ;
aryl is phenyl, optionally substituted with 1, 2 or 3 substituents, each independently from each other, selected from the group of (C₁₋₃)alkyl; halo ; and trifluoromethyl ;
alkyl is a straight or branched saturated hydrocarbon radical having from 1 to 8 carbon atoms ; or is a cyclic saturated hydrocarbon radical having from 3 to 7 carbon atoms ; or is a cyclic saturated hydrocarbon radical having from 3 to 7 carbon atoms attached to a straight or branched saturated hydrocarbon radical having from 1 to 8 carbon atoms ; wherein each radical is optionally substituted on one or more carbon atoms with one or more radicals selected from the group of oxo ; (C₁₋₃)alkyloxy ; halo ; cyano ; nitro ; formyl ; hydroxy ; amino ; carboxy ; and thio ;
alkenyl is an alkyl radical as defined above, further having one or more double bonds;
alkynyl is an alkyl radical as defined above, further having one or more triple bonds ; and
arylalkyl is an alkyl radical as defined above, further having one or more CH₃-groups replaced by phenyl.

15. Compound according to any one of claims 1 to 14 for use as a medicine.

16. Pharmaceutical composition comprising a pharmaceutically acceptable carrier or diluent and, as active ingredient, a therapeutically effective amount of a compound according to any one of claims 1 to 14.

17. Pharmaceutical composition according to claim 16, **characterized in that** is comprises further one or more other compounds selected from the group of antidepressants, anxiolytics and antipsychotics.

18. Pharmaceutical composition according to any of of claims 16 and 17,
**characterized in that** it is in a form suitable to be orally administered.

19. Process for the preparation of a pharmaceutical composition as claimed in claim 16, **characterized in that** a pharmaceutically acceptable carrier is intimately mixed with a therapeutically effective amount of a compound as claimed in any one of claims 1 to 14.

20. Process for the preparation of a pharmaceutical composition as claimed in claim 17, **characterized in that** a pharmaceutically acceptable carrier is intimately mixed with a therapeutically effective amount of a compound as claimed in any one of claims 1 to 14 and one or more other compounds selected from the group of antidepressants, anxiolytics and antipsychotics.

21. Use of a compound according to any one of claims 1 to 14 for the preparation of a medicament for the prevention and/or treatment of diseases where antagonism of the α₂-adrenergic receptor, in particular antagonism of the α_{2C}-adrenergic receptor is of therapeutic use.

22. Use of a compound according to any one of claims 1 to 14 for the preparation of a medicament for the prevention and/or treatment of central nervous system disorders, mood disorders, anxiety disorders, stress-related disorders associated with depression and/or anxiety, cognitive disorders, personality disorders, schizoaffective disorders, Parkinson's disease, dementia of the Alzheimer's type, chronic pain conditions, neurodegenerative diseases, addiction disorders, mood disorders and sexual dysfunction.

23. Use of a compound according to any one of claims 1 to 14 in combination with one or more other compounds selected from the group of antidepressants, anxiolytics and antipsychotics for the preparation of a medicament for the prevention and/or treatment of central nervous system disorders, mood disorders, anxiety disorders, stress-related disorders associated with depression and/or anxiety, cognitive disorders, personality disorders, schizoaffective disorders, Parkinson's disease, dementia of the Alzheimer's type, chronic pain conditions, neurodegenerative diseases, addiction disorders, mood disorders and sexual dysfunction.

## Patentansprüche

1. Verbindungen der allgemeinen Formel (I) 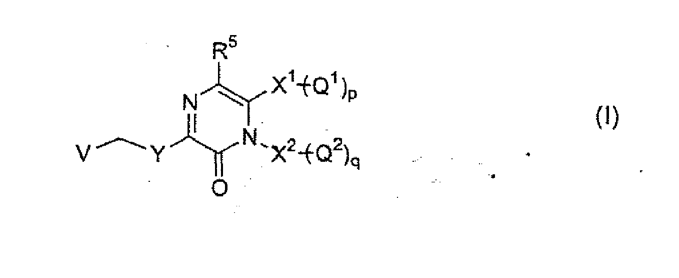 deren pharmazeutisch unbedenkliche Säure- oder Basenadditionssalze, deren stereochemisch isomere Formen, deren N-Oxidformen und deren quaternäre Ammoniumsalze, wobei:
V für einen Naphthylrest steht, wobei eine CH-Einheit in der Naphthylgruppe gegebenenfalls durch ein N-Atom ersetzt sein kann;
Y für einen zweiwertigen Rest der Formel (II) 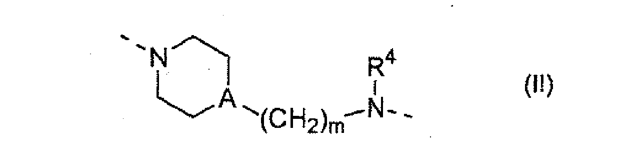 steht;
wobei
A für ein Stickstoff- oder ein Kohlenstoffatom steht;
m für eine ganze Zahl gleich null, 1 oder 2 steht;
R⁴ aus der aus Wasserstoff, Alkyl und Phenylcarboxylalkyl bestehenden Gruppe ausgewählt ist;
R⁵ aus der aus Wasserstoff und Halogen bestehenden Gruppe ausgewählt ist;
X¹, X² jeweils unabhängig voneinander für eine kovalente Bindung, einen gesättigten oder einen ungesättigten (C₁₋₈)Kohlenwasserstoffrest, wobei eine oder mehrere zweiwertige -CH₂-Einheiten und/oder eine oder mehrere einwertige CH₃-Einheiten gegebenenfalls durch eine zweiwertige beziehungsweise einwertige Phenyleinheit ersetzt sein können, und wobei ein oder mehrere Wasserstoffatome durch einen aus der aus Oxo, (C₁₋₃)Alkyloxy, Halogen, Cyano, Nitro, Formyl, Hydroxy, Amino, Trifluormethyl, Mono- und Di ((C₁₋₃)-Alkyl) amino, Carboxy und Thio bestehenden Gruppe ausgewählten Rest ersetzt sein können, stehen;
p, q jeweils unabhängig voneinander für eine
Q¹, Q² ganze Zahl gleich 1 oder 2 stehen; jeweils unabhängig voneinander für einen aus der aus Wasserstoff, -NR¹R², Pir,OR³ und Het bestehenden Gruppe ausgewählten Rest stehen; wobei zwei Reste -OR³ zusammen einen zweiwertigen Rest -O-(CH₂)ᵣ-O- bilden können, wobei r für eine ganze Zahl gleich 1, 2 oder 3 steht;
R¹ und R² jeweils unabhängig voneinander für einen aus der aus Wasserstoff, Alkyl, Alkenyl, Alkinyl, Aryl, Arylalkyl, Alkylcarbonyl, Alkenylcarbonyl, Alkyloxy, Alkyloxyalkyl, Alkyloxycarbonyl, Alkyloxyalkylcarbonyl, Alkyloxycarbonylalkyl, Alkyloxycarbonylalkylcarbonyl, Alkylsulfonyl, Arylcarbonyl, Aryloxyalkyl, Arylalkylcarbonyl, Arylsulfonyl, Het, Het-Alkyl, Het-Alkylcarbonyl, Het-Carbonyl, Het-Carbonylalkyl, Alkyl-NR^{a}R^{b}, Carbonyl-NR^{a}R^{b}, Carbonylalkyl-NR^{a}R^{b}, Alkylcarbonyl-NR^{a}R^{b} und Alkylcarbonylalkyl-NR^{a}R^{b} bestehenden Gruppe ausgewählten Rest stehen; wobei R^{a} und R^{b} jeweils unabhängig aus der aus Wasserstoff, Alkyl, Alkylcarbonyl, Alkyloxyalkyl, Alkyloxycarbonylalkyl, Aryl, Arylalkyl, Het und Alkyl-NR^{c}R^{d} bestehenden Gruppe ausgewählt sind, wobei R^{c} und R^{d} jeweils unabhängig voneinander für Wasserstoff oder Alkyl stehen;
Pir für einen Rest mit wenigstens einem N, über das er an den X-Rest gebunden ist, steht, ausgewählt aus der aus Pyrrolidinyl, Imidazolidinyl, Pyrazolidinyl, Piperidinyl, Piperazinyl, Pyrrolyl, Pyrrolinyl, Imidazolinyl, Pyrrazolinyl, Pyrrolyl, Imidazolyl, Pyrazolyl, Triazolyl, Azepyl, Diazepyl, Morpholinyl, Thiomorpholinyl, Indolyl, Isoindolyl, Indolinyl, Indazolyl, Benzimidazolyl und 1,2,3,4-Tetrahydroisochinolinyl bestehenden Gruppe; wobei die Pir-Reste jeweils gegebenenfalls durch 1, 2 oder 3 Reste ausgewählt aus der aus Hydroxy, Halogen, Oxo, (C₁₋₃)-Alkyl, Trifluormethyl, Phenyl, Benzyl, Pyrrolidinyl und Pyridinyloxy bestehenden Gruppe substituiert sind;
R³ für einen Rest ausgewählt aus der aus Wasserstoff, Alkyl, Aryl, Arylalkyl, Het und Het-Alkyl bestehenden Gruppe steht;
Het für einen heterocyclischen Rest ausgewählt aus der aus Pyrrolidinyl, Imidazolidinyl, Pyrazolidinyl, Piperidinyl, Piperazinyl, Pyrrolyl, Pyrrolinyl, Imidazolinyl, Pyrrazolinyl, Pyrrolyl, Imidazolyl, Pyrazolyl, Triazolyl, Pyridinyl, Pyridazinyl, Pyrimidinyl, Pyrazinyl, Triazinyl, Azepyl, Diazepyl, Morpholinyl, Thiomorpholinyl, Indolyl, Isoindolyl, Indolinyl, Indazolyl, Benzimidazolyl, 1,2,3,4-Tetrahydroisochinolinyl, Furyl, Thienyl, Oxazolyl, Isoxazolyl, Thiazolyl, Thiadiazolyl, Isothiazolyl, Dioxolyl, Dithianyl, Tetrahydrofuryl, Tetrahydropyranyl, Chinolinyl, Isochinolinyl, Chinoxalinyl, Benzoxazolyl, Benzisoxazolyl, Benzothiazolyl, Benzisothiazolyl, Benzofuranyl, Benzothienyl, Benzopiperidinyl, Chromenyl und Imidazo[1,2-a]pyridinyl bestehenden Gruppe steht; wobei die Het-Reste jeweils gegebenenfalls durch einen oder mehrere Reste ausgewählt aus der aus Halogen, Oxo, (C₁₋₃)-Alkyl, (C₁₋₃)-Alkylcarbonyl, (C₁₋₃)-Alkenylthio, Imidazolyl-(C₁₋₃)-alkyl und (C₁₋₃)-Alkyloxycarbonyl bestehenden Gruppe substituiert sind;
Aryl für Naphthalinyl oder Phenyl steht, jeweils gegebenenfalls substituiert durch 1, 2 oder 3 Substituenten jeweils unabhängig voneinander ausgewählt aus der aus Oxo, (C₁₋₃)-Alkyl, (C₁₋₃)-Alkyloxy, Halogen, Cyano, Nitro, Formyl, Hydroxy, Amino, Trifluormethyl, Mono- und Di((C₁₋₃ )-alkyl) amino, Carboxy und Thio bestehenden Gruppe;
Alkyl für einen geradkettigen oder verzweigten gesättigten Kohlenwasserstoffrest mit 1 bis 8 Kohlenstoffatomen oder einen cyclischen gesättigten Kohlenwasserstoffrest mit 3 bis 7 Kohlenstoffatomen oder einen cyclischen gesättigten Kohlenwasserstoffrest mit 3 bis 7 Kohlenstoffatomen gebunden an einen geradkettigen oder verzweigten gesättigten Kohlenwasserstoffrest mit 1 bis 8 Kohlenstoffatomen steht; wobei die Reste jeweils gegebenenfalls an einem oder mehreren Kohlenstoffatomen durch einen oder mehrere Reste ausgewählt aus der aus Oxo, (C₁₋₃)-Alkyloxy, Halogen, Cyano, Nitro, Formyl, Hydroxy, Amino, Carboxy und Thio bestehenden Gruppe substituiert sind;
Alkenyl für einen wie oben definierten Alkylrest steht, der weiterhin eine oder mehrere Doppelbindungen aufweist;
Alkinyl für einen wie oben definierten Alkylrest steht, der weiterhin eine oder mehrere Dreifachbindungen aufweist; und
Arylalkyl für einen wie oben definierten Alkylrest steht, bei dem weiterhin eine oder mehrere CH₃-Gruppen durch Phenyl ersetzt sind.

2. Verbindungen nach Anspruch 1, **dadurch gekennzeichnet, dass** V aus der Gruppe der Reste (z-1), (z-2), (z-3), (z-4), (z-5) und (z-6) ausgewählt ist.

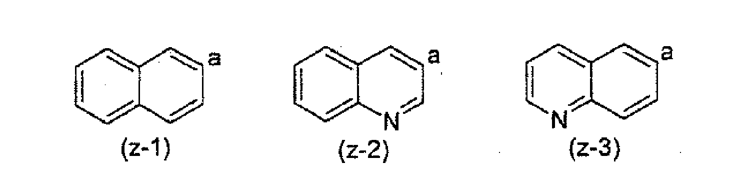

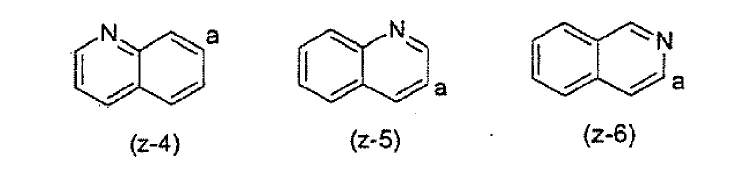

3. Verbindungen nach Anspruch 1 oder 2, **dadurch gekennzeichnet, dass** A für ein Kohlenstoffatom steht, m für null steht und R⁴ für Wasserstoff steht.

4. Verbindungen nach einem der Ansprüche 1 bis 3, **dadurch gekennzeichnet, dass** die Gruppe -CH₂-Y-über das durch "a" gekennzeichnete Atom an V gebunden ist.

5. Verbindungen nach einem der Ansprüche 1 bis 4, **dadurch gekennzeichnet, dass** R⁵ für Chlor steht.

6. Verbindungen nach einem der Ansprüche 1 bis 5, **dadurch gekennzeichnet, dass** X¹ und X² jeweils unabhängig voneinander aus der aus einer kovalenten Bindung, -CH₂-, -CH₂CH₂-, -CH₂CH₂CH₂-, - CH₂CH₂CH₂CH₂-, -CH₂CH=CHCH₂-, -CH₂C=CCH₂-, - CH(CH₃)CH(CH₃)-, -C(=O)CH₂-, -C(=O)CH₂CH₂-, - C(=O)CH₂CH₂CH₂-, -CH₂C(=O)-, -CH₂CH₂C(-O)-, - CH₂CH₂CH₂C(=O)-, -CH₂CH₂CH₂CH₂C (=O) -, - CH₂CH₂C(=O)CH₂-, -C₆H₄-, -CH₂C₆H₄-, -CH₂CH₂C₆H₄-, - CH₂CH₂CH₂C₆H₄-, -C₆H₄CH₂-, -C₆H₄CH₂CH₂-, - C₆H₄CH₂CH₂CH₂-, -CH₂C₆H₄CH₂-, -CH₂CH₂C₆H₄CH₂CH₂-, - C₆H₄C (=0) -, -C₆H₄CH₂C (=0) -, -C₆H₄CH₂CH₂C (=O) - und - CH₂CH₂C(=O)C₆H₄- bestehenden Gruppe ausgewählt sind.

7. Verbindungen nach einem der Ansprüche 1 bis 6, **dadurch gekennzeichnet, dass** X¹ und X² jeweils unabhängig voneinander aus der aus einer kovalenten Bindung, -CH₂-, -CH₂CH₂-, -CH₂CH₂CH₂-, - CH₂CH₂CH₂CH₂-, -CH₂CH=CHCH₂-, -CH₂C≡CCH₂-, - CH₂C(=O) -, -CH₂CH₂C (=O) -, -CH₂CH₂CH₂C (=O) -, - CH₂CH₂CH₂CH₂C (=0) -, -C₆H₄-, -CH₂C₆H₄-, -CH₂CH₂CH₂C₆H₄-, -C₆H₄CH₂-, -CH₂C₆H₄CH₂-, -C₆H₄C (=0) -, -C₆H₄CH₂C (=0) -, -C₆H₄CH₂CH₂C (=0) - und -CH₂CH₂C (=O) C₆H₄- bestehenden Gruppe ausgewählt sind.

8. Verbindungen nach einem der Ansprüche 1 bis 7, **dadurch gekennzeichnet, dass** ein oder mehrere Wasserstoffatome in X¹ und X² jeweils gegebenenfalls durch einen aus der aus Oxo, (C₁₋₃)-Alkyloxy, Halogen, Cyano, Nitro und Formyl bestehenden Gruppe ausgewählten Rest substituiert sind.

9. Verbindungen nach einem der Ansprüche 1 bis 8, **dadurch gekennzeichnet, dass** X¹ für eine kovalente Bindung steht, Q¹ für Wasserstoff steht und p für 1 steht.

10. Verbindungen nach einem der Ansprüche 1 bis 9, **dadurch gekennzeichnet, dass** R¹ und R² jeweils unabhängig voneinander für einen aus der aus Wasserstoff, Alkyl, Alkenyl, Alkinyl, Aryl, Arylalkyl, Alkylcarbonyl, Alkenylcarbonyl, Alkyloxyalkyl, Alkyloxycarbonyl, Alkyloxyalkylcarbonyl, Alkyloxycarbonylalkyl, Alkyloxycarbonylalkylcarbonyl, Arylcarbonyl, Aryloxyalkyl, Arylalkylcarbonyl, Het-Alkyl, Het-Alkylcarbonyl, Het-Carbonyl, Het-Carbonylalkyl, Alkyl-NR^{a}R^{b}, Carbonyl-NR^{a}R^{b}, Carbonylalkyl-NR^{a}R^{b}, Alkylcarbonyl-NR^{a}R^{b} und Alkylcarbonylalkyl-NR^{a}R^{b} bestehenden Gruppe ausgewählten Rest stehen; wobei R^{a} und R^{b} jeweils unabhängig aus der aus Wasserstoff, Alkyl, Alkylcarbonyl, Alkyloxyalkyl, Alkyloxycarbonylalkyl, Aryl, Arylalkyl, Het und Alkyl-NR^{c}R^{d} bestehenden Gruppe ausgewählt sind, wobei R^{c} und R^{d} jeweils unabhängig voneinander für Wasserstoff oder Alkyl stehen.

11. Verbindungen nach einem der Ansprüche 1 bis 10, **dadurch gekennzeichnet, dass** Pir für einen Rest steht, der wenigstens ein N enthält, über das er an den X-Rest gebunden ist, ausgewählt aus der aus Pyrrolidinyl, Piperidinyl, Piperazinyl, Imidazolyl, Morpholinyl und Isoindolyl bestehenden Gruppe; wobei die Pir-Reste jeweils gegebenenfalls durch 1, 2 oder 3 Reste ausgewählt aus der aus Hydroxy, Halogen, Oxo, (C₁₋₃)-Alkyl, Trifluormethyl, Phenyl, Benzyl, Pyrrolidinyl und Pyridinyloxy bestehenden Gruppe substituiert sind.

12. Verbindungen nach einem der Ansprüche 1 bis 11, **dadurch gekennzeichnet, dass** Het für einen heterocyclischen Rest ausgewählt aus der aus Piperidinyl, Piperazinyl, Triazolyl, Pyridinyl, Pyrimidinyl, Morpholinyl, Indolyl, Furyl, Thienyl, Isoxazolyl, Thiazolyl, Tetrahydrofuryl, Tetrahydropyranyl, Chinolinyl, Isochinolinyl, Benzofuranyl, Benzothienyl und Benzopiperidinyl bestehenden Gruppe steht; wobei die Het-Reste jeweils gegebenenfalls durch einen oder mehrere aus der aus Oxo, (C₁₋₃)-Alkyl, (C₁₋₃)-Alkylcarbonyl und Imidazolyl-(C₁₋₃)-alkyl bestehenden Gruppe ausgewählte Reste substituiert sind.

13. Verbindungen nach einem der Ansprüche 1 bis 12, **dadurch gekennzeichnet, dass** Aryl für Phenyl steht, das gegebenenfalls durch 1, 2 oder 3 Substituenten, die jeweils unabhängig voneinander aus der aus (C₁₋₃)-Alkyl, Halogen und Trifluormethyl bestehenden Gruppe ausgewählt sind, substituiert ist.

14. Verbindungen nach Anspruch 1, **dadurch gekennzeichnet, dass**:
V aus der aus den Resten (z-1), (z-2), (z-3), (z-5) und (z-6) bestehenden Gruppe ausgewählt ist; wobei die Gruppe -CH₂-Y- über das durch "a" gekennzeichnete Atom an V gebunden ist.
Y für einen zweiwertigen Rest der Formel (II) steht, wobei A für ein Stickstoff- oder ein Kohlenstoffatom steht, m für eine ganze Zahl gleich null oder 2 steht und R⁴ aus der aus Wasserstoff, Alkyl und Phenylcarboxylalkyl bestehenden Gruppe ausgewählt ist;
R⁵ aus der aus Wasserstoff und Halogen bestehenden Gruppe ausgewählt ist;
X¹, X² jeweils unabhängig voneinander aus der aus einer kovalenten Bindung, -CH₂-, -CH₂CH₂-,CH₂CH₂CH₂-, -CH₂CH₂CH₂CH₂-, -CH₂CH=CHCH₂-,CH₂C≡CCH₂-, -CH₂C(=O)-, -CH₂CH₂C(=O)-,CH₂CH₂CH₂C(=O)-, -CH₂CH₂CH₂CH₂C(=O)-, -C₆H₄-,CH₂C₆H₄-, -CH₂CH₂CH₂C₆H₄-, -C₆H₄CH₂-,CH₂C₆H₄CH₂-, -C₆H₄C(=0) -, -C₆H₄CH₂C (=0) -,C₆H₄CH₂CH₂C(=O)- und -CH₂CH₂C(=O)C₆H₄-bestehenden Gruppe ausgewählt sind; und wobei ein oder mehrere Wasserstoffatome durch einen aus der aus Oxo, (C₁₋₃)-Alkyloxy, Halogen, Cyano, Nitro und Formyl bestehenden Gruppe ausgewählten Rest ersetzt sein können;
p, q jeweils unabhängig voneinander für eine ganze Zahl gleich 1 oder 2 stehen;
Q¹, Q² jeweils unabhängig voneinander für einen aus der aus Wasserstoff, -NR¹R², Pir, -OR³ und Het bestehenden Gruppe ausgewählten Rest stehen; wobei zwei ResteOR³ zusammen einen zweiwertigen Rest -O-(CH₂)ᵣ-O- bilden können, wobei r für eine ganze Zahl gleich 1, 2 oder 3 steht;
R¹ und R² jeweils unabhängig voneinander für einen aus der aus Wasserstoff, Alkyl, Alkenyl, Alkinyl, Aryl, Arylalkyl, Alkylcarbonyl, Alkenylcarbonyl, Alkyloxyalkyl, Alkyloxycarbonyl, Alkyloxyalkylcarbonyl, Alkyloxycarbonylalkyl, Alkyloxycarbonylalkylcarbonyl, Arylcarbonyl, Aryloxyalkyl, Arylalkylcarbonyl, Het-Alkyl, Het-Alkylcarbonyl, Het-Carbonyl, Het-Carbonylalkyl, Alkyl-NR^{a}R^{b}, Carbonyl-NR^{a}R^{b}, Carbonylalkyl-NR^{a}R^{b}, Alkylcarbonyl-NR^{a}R^{b} und Alkylcarbonylalkyl-NR^{a}R^{b} bestehenden Gruppe ausgewählten Rest stehen; wobei R^{a} und R^{b} jeweils unabhängig aus der aus Wasserstoff, Alkyl, Alkylcarbonyl, Alkyloxyalkyl, Alkyloxycarbonylalkyl, Aryl, Arylalkyl, Het und Alkyl-NR^{c}R^{d} bestehenden Gruppe ausgewählt sind, wobei R^{c} und R^{d} jeweils unabhängig voneinander für Wasserstoff oder Alkyl stehen;
Pir für einen Rest mit wenigstens einem N, über das er an den X-Rest gebunden ist, steht, ausgewählt aus der aus Pyrrolidinyl, Piperidinyl, Piperazinyl, Imidazolinyl, Morpholinyl, Isoindolyl bestehenden Gruppe; wobei die Pir-Reste jeweils gegebenenfalls durch 1, 2 oder 3 Reste ausgewählt aus der aus Hydroxy, Halogen, Oxo, (C₁₋₃)-Alkyl, Trifluormethyl, Phenyl, Benzyl, Pyrrolidinyl und Pyridinyloxy bestehenden Gruppe substituiert sind;
R³ für einen Rest ausgewählt aus der aus Wasserstoff, Alkyl, Aryl, Arylalkyl, Het und Het-Alkyl bestehenden Gruppe steht;
Het für einen heterocyclischen Rest ausgewählt aus der aus Piperidinyl, Piperazinyl, Triazolyl, Pyridinyl, Pyrimidinyl, Morpholinyl, Indolyl, Furyl, Thienyl, Isoxazolyl, Thiazolyl, Tetrahydrofuryl, Tetrahydropyranyl, Chinolinyl, Isochinolinyl, Benzofuranyl, Benzothienyl und Benzopiperidinyl, bestehenden Gruppe steht; wobei die Het-Reste jeweils gegebenenfalls durch einen oder mehrere Reste ausgewählt aus der aus Oxo, (C₁₋₃)-Alkyl, (C₁₋₃)-Alkylcarbonyl und Imidazolyl-(C₁₋₃)-alkyl bestehenden Gruppe substituiert sind;
Aryl für Phenyl steht, gegebenenfalls substituiert durch 1, 2 oder 3 Substituenten jeweils unabhängig voneinander ausgewählt aus der aus (C₁₋₃)-Alkyl, Halogen und Trifluormethyl bestehenden Gruppe;
Alkyl für einen geradkettigen oder verzweigten gesättigten Kohlenwasserstoffrest mit 1 bis 8 Kohlenstoffatomen oder einen cyclischen gesättigten Kohlenwasserstoffrest mit 3 bis 7 Kohlenstoffatomen oder einen cyclischen gesättigten Kohlenwasserstoffrest mit 3 bis 7 Kohlenstoffatomen, der an einen geradkettigen oder verzweigten gesättigten Kohlenwasserstoffrest mit 1 bis 8 Kohlenstoffatomen gebunden ist, steht; wobei die Reste jeweils gegebenenfalls an einem oder mehreren Kohlenstoffatomen durch einen oder mehrere aus der aus Oxo, (C₁₋₃ )-Alkyloxy, Halogen, Cyano, Nitro, Formyl, Hydroxy, Amino, Carboxy und Thio bestehenden Gruppe ausgewählten Reste substituiert sind;
Alkenyl für einen wie oben definierten Alkylrest steht, der weiterhin eine oder mehrere Doppelbindungen aufweist;
Alkinyl für einen wie oben definierten Alkylrest steht, der weiterhin eine oder mehrere Dreifachbindungen aufweist; und
Arylalkyl für einen wie oben definierten Alkylrest steht, der weiterhin eine oder mehrere durch Phenyl ersetzte CH₃-Gruppen aufweist.

15. Verbindungen nach einem der Ansprüche 1 bis 14 zur Verwendung als Medizin.

16. Pharmazeutische Zusammensetzung, enthaltend einen pharmazeutisch unbedenklichen Träger oder ein pharmazeutisch unbedenkliches Verdünnungsmittel und, als Wirkstoff, eine therapeutisch wirksame Menge einer Verbindung nach einem der Ansprüche 1 bis 14.

17. Pharmazeutische Zusammensetzung nach Anspruch 16, **dadurch gekennzeichnet, dass** sie weiterhin eine oder mehrere andere aus der Gruppe der Antidepressiva, Anxiolytika und Antipsychotika aus gewählte Verbindungen enthält.

18. Pharmazeutische Zusammensetzung nach Anspruch 16 oder 17, **dadurch gekennzeichnet, dass** sie in einer für eine orale Verabreichung geeigneten Form vorliegt.

19. Verfahren zur Herstellung einer pharmazeutischen Zusammensetzung nach Anspruch 16, **dadurch gekennzeichnet, dass** man den pharmazeutisch unbedenklichen Träger innig mit einer therapeutisch wirksamen Menge einer Verbindung nach einem der Ansprüche 1 bis 14 mischt.

20. Verfahren zur Herstellung einer pharmazeutischen Zusammensetzung nach Anspruch 17, **dadurch gekennzeichnet, dass** man einen pharmazeutisch unbedenklichen Träger innig mit einer therapeutisch wirksamen Menge einer Verbindung nach einem der Ansprüche 1 bis 14 und eine oder mehrere andere aus der Gruppe der Antidepressiva, Anxiolytika und Antipsychotika ausgewählten Verbindungen mischt.

21. Verwendung einer Verbindung nach einem der Ansprüche 1 bis 14 zur Herstellung eines Medikaments zur Prävention und/oder Behandlung von Krankheiten, bei denen ein Antagonismus des α₂-adrenergen Rezeptors, insbesondere ein Antagonismus des α_{2c}-adrenergen Rezeptors, von therapeutischem Nutzen ist.

22. Verwendung einer Verbindung nach einem der Ansprüche 1 bis 14 zur Herstellung eines Medikaments zur Prävention und/oder Behandlung von Erkrankungen des zentralen Nervensystems, affektiven Psychosen, Angstzuständen, mit Stress in Zusammenhang stehenden Erkrankungen, die mit Depression und/oder Angstzuständen assoziiert sind, kognitiven Störungen, Persönlichkeitsstörungen, schizoaffektiven Störungen, Parkinson-Krankheit, Demenz des Alzheimer-Typs, chronischen Schmerzleiden, neurodegenerativen Krankheiten, Suchterkrankungen, affektiven Psychosen und sexueller Dysfunktion.

23. Verwendung einer Verbindung nach einem der Ansprüche 1 bis 14 in Kombination mit einer oder mehreren anderen Verbindungen ausgewählt aus der Gruppe der Antidepressiva, Anxiolytika und Antipsychotika zur Herstellung eines Medikaments zur Prävention und/oder Behandlung von Erkrankungen des zentralen Nervensystems, affektiven Psychosen, Angstzuständen, mit Stress in Zusammenhang stehenden Erkrankungen, die mit Depression und/oder Angstzuständen assoziiert sind, kognitiven Störungen, Persönlichkeitsstörungen, schizoaffektiven Störungen, Parkinson-Krankheit, Demenz des Alzheimer-Typs, chronischen Schmerzleiden, neurodegenerativen Krankheiten, Suchterkrankungen, affektiven Psychosen und sexueller Dysfunktion.

## Revendications

1. Composé selon la formule générale (I) 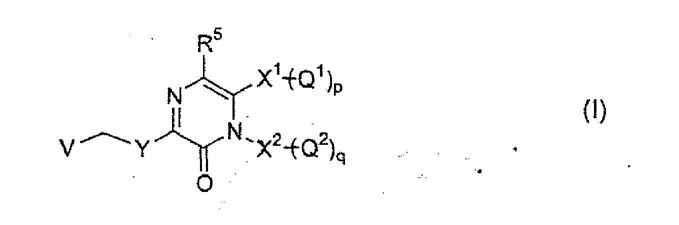 un sel d'addition d'acide ou de base pharmaceutiquement acceptable de celui-ci, une forme stéréochimiquement isomère de celui-ci, une forme N-oxyde de celui-ci ou un sel d'ammonium quaternaire de celui-ci, dans laquelle :
V est un radical naphtyle, où une unité CH dans le motif naphtyle peut éventuellement être remplacée par un atome de N ;
Y est un radical bivalent de formule (II) 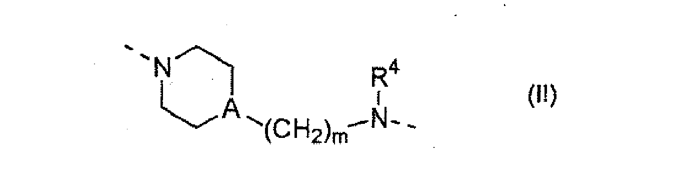
dans laquelle
A est un atome d'azote ou de carbone ;
m est un entier égal à zéro, 1 ou 2 ;
R⁴ est choisi dans le groupe comprenant hydrogène ; alkyle et phénylcarboxyalkyle ;
R⁵ est choisi dans le groupe comprenant hydrogène et halogéno ;
X¹, X² sont chacun, indépendamment l'un de l'autre, une liaison covalente, un radical hydrocarboné en (C₁₋₈) saturé ou insaturé, où une ou plusieurs unités -CH₂-bivalentes et/ou une ou plusieurs unités CH₃- monovalentes peuvent éventuellement être remplacées par une unité phényle bivalente ou monovalente respective ; et où un ou plusieurs atomes d'hydrogène peuvent être remplacés par un radical choisi dans le groupe comprenant oxo ; (C₁₋₃)alkyloxy ; halogèno ; cyano ; nitro ; formyle ; hydroxy ; amino ; trifluorométhyle ; m o n o- et di((C₁₋₃)alkyl)amino ; carboxy ; et thio ;
p, q sont chacun, indépendamment l'un de l'autre, un entier égal à 1 ou 2 ;
Q¹, Q² sont chacun, indépendamment l'un de l'autre, un radical choisi dans le groupe comprenant hydrogène ; -NR¹R² ; Pir ; -OR³ et Het ; où deux radicaux -OR³ peuvent être pris ensemble pour former un radical bivalent -O-(CH₂)ᵣ-O- où r est un entier égal à 1, 2 ou 3 ;
R¹ et R² sont chacun, indépendamment l'un de l'autre, un radical choisi dans le groupe comprenant hydrogène ; alkyle ; alcényle ; alcynyle ; aryle ; arylalkyle ; alkylcarbonyle ; alcénylcarbonyle; alkyloxy; alkyloxyalkyle; alkyloxycarbonyle ; alkyloxyalkylcarbonyle ; alkyloxycarbonylalkyle ; alkyloxycarbonylalkylcarbonyle ; alkylsulfonyle ; arylcarbonyle ; aryloxyalkyle ; arylalkylcarbonyle ; arylsulfonyle ; Het ; Het-alkyle ; Het-alkylcarbonyle ; Het-carbonyle ; Het-carbonylalkyle ; alkyl-NR^{a}R^{b} ; carbonyl-NR^{a}R^{b} ; carbonylalkyl-NR^{a}R^{b} ; alkylcarbonyl-NR^{a}R^{b} ; e t alkylcarbonylalkyl-NR^{a}R^{b} ; où R^{a} et R^{b} sont choisis chacun indépendamment dans le groupe comprenant hydrogène, alkyle, alkylcarbonyle, alkyloxyalkyle, alkyloxycarbonylalkyle, aryle, arylalkyle, Het et alkyl-NR^{c}R^{d}, où R^{c} et R^{d} sont chacun indépendamment l'un de l'autre hydrogène ou alkyle ;
Pir est un radical contenant au moins un N, par lequel il est lié au radical X, choisi dans le groupe comprenant pyrrolidinyle ; imidazolidinyle ; pyrazolidinyle ; pipéridinyle ; pipérazinyle ; pyrrolyle ; pyrrolinyle ; imidazolinyle ; pyrrazolinyle ; pyrrolyle ; imidazolyle ; pyrazolyle ; triazolyle ; azépyle ; diazépyle ; morpholinyle ; thiomorpholinyle ; indolyle ; isoindolyle ; indolinyle ; indazolyle ; benzimidazolyle ; e t 1,2,3,4-tétrahydro-isoquinoléinyle ; où chaque radical Pir est éventuellement substitué par 1, 2 ou 3 radicaux choisis dans le groupe comprenant hydroxy ; halogéno ; oxo ; (C₁₋₃)alkyle ; trifluorométhyle ; phényle ; benzyle ; pyrrolidinyle ; et pyridinyloxy ;
R³ est un radical choisi dans le groupe comprenant hydrogène ; alkyle ; aryle ; arylalkyle ; Het ; et Het-alkyle ;
Het est un radical hétérocyclique choisi dans le groupe comprenant pyrrolidinyle ; imidazolidinyle ; pyrazolidinyle ; pipéridinyle ; pipérazinyle ; pyrrolyle ; pyrrolinyle ; imidazolinyle ; pyrrazolinyle ; pyrrolyle ; imidazolyle ; pyrazolyle ; triazolyle ; pyridinyle ; pyridazinyle ; pyrimidinyle ; pyrazinyle ; triazinyle ; azépyle ; diazépyle ; morpholinyle ; thiomorpholinyle ; indolyle ; isoindolyle ; indolinyle ; indazolyle ; benzimidazolyle ; 1,2,3,4-tétrahydro-isoquinoléinyle ; furyle ; thiényle ; oxazolyle ; isoxazolyle ; thiazolyle ; thiadiazolyle ; isothiazolyle ; dioxolyle ; dithianyle ; tétrahydrofuryle ; tétrahydropyranyle ; quinoléinyle ; isoqu inoléinyle ; quinoxalinyle ; benzoxazolyle ; benzisoxazolyle ; benzothiazolyle ; benzisothiazolyle ; benzofuranyle ; benzothiényle ; benzopipéridinyle ; chroményle ; et imidazo[1,2-a]pyridinyle ; où chaque radical Het est éventuellement substitué par un ou plusieurs radicaux choisis dans le groupe comprenant halogéno ; oxo ; (C₁₋₃)alkyle ; (C₁₋₃)alkylcarbonyle ; (C₁₋₃)alcénylthio ; imidazolyl-(C₁₋₃)alkyle ; et (C₁₋₃)alkyloxycarbonyle ;
aryle est naphtalényle ou phényle, substitués chacun éventuellement par 1, 2 ou 3 substituants choisis chacun indépendamment l'un de l'autre dans le groupe comprenant oxo ; (C₁₋₃)alkyle ; (C₁₋₃)alkyloxy ; halogéno ; cyano ; nitro ; formyle ; hydroxy ; amino ; trifluorométhyle ; mono- et di((C₁₋₃)alkyl)amino ; carboxy ; et thio ;
alkyle est un radical hydrocarboné saturé droit ou ramifié ayant de 1 à 8 atomes de carbone ; ou est un radical hydrocarboné saturé cyclique ayant de 3 à 7 atomes de carbone ; ou est un radical hydrocarboné saturé cyclique ayant de 3 à 7 atomes de carbone lié à un radical hydrocarboné saturé droit ou ramifié ayant de 1 à 8 atomes de carbone ; où chaque radical est éventuellement substitué sur un ou plusieurs atomes de carbone par un ou plusieurs radicaux choisis dans le groupe comprenant oxo ; (C₁₋₃)alkyloxy ; halogéno ; cyano ; nitro ; formyle ; hydroxy ; amino ; carboxy ; et thio ;
alcényle est un radical alkyle tel que défini ci-dessus, ayant en outre une ou plusieurs doubles liaisons ;
alcynyle est un radical alkyle tel que défini ci-dessus, ayant en outre une ou plusieurs triples liaisons ; et
arylalkyle est un radical alkyle tel que défini ci-dessus, ayant en outre un ou plusieurs groupements CH₃- remplacés par phényle.

2. Composé selon la revendication 1, **caractérisé en ce que** V est choisi dans le groupe comprenant les radicaux (z-1), (z-2), (z-3), (z-4), (z-5) et (z-6) .

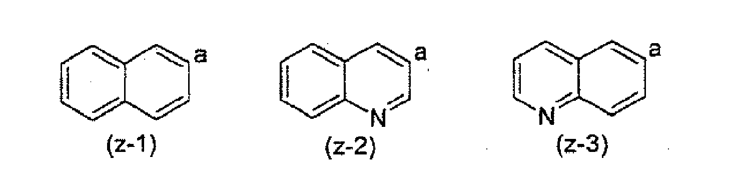

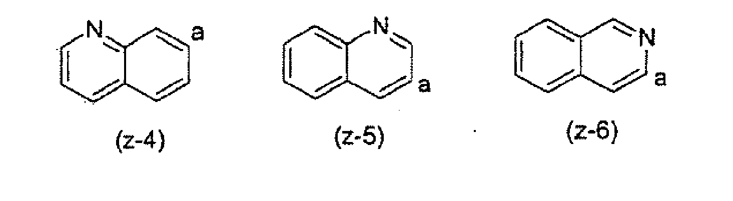

3. Composé selon l'une ou l'autre des revendications 1 et 2, **caractérisé en ce que** A est un atome de carbone, m est zéro et R⁴ est hydrogène.

4. Composé selon l'une quelconque des revendications 1 à 3, **caractérisé en ce que** le motif -CH₂-Y- est lié à V par l'atome désigné par « a ».

5. Composé selon l'une quelconque des revendications 1 à 4, **caractérisé en ce que** R⁵ est chloro.

6. Composé selon l'une quelconque des revendications 1 à 5, **caractérisé en ce que** X¹ et X² sont choisis chacun, indépendamment l'un de l'autre, dans le groupe comprenant une liaison covalente ; -CH₂- ; - CH₂CH₂- ; -CH₂CH₂CH₂- ; -CH₂CH₂CH₂CH₂- ; -CH₂CH=CHCH₂-; -CH₂C≡CCH₂- ; -CH(CH₃)CH(CH₃)- ; -C(=O)CH₂- ; - C (=O) CH₂CH₂- ; -C(=O)CH₂CH₂CH₂- ; -CH₂C(=O)- ; - CH₂CH₂C(=O)- ; -CH₂CH₂CH₂C (=O) - ; -CH₂CH₂CH₂CH₂C(=O)-; -CH₂CH₂C(=O)CH₂- ; -C₆H₄- ; -CH₂C₆H₄- ; - CH₂CH₂C₆H₄- ; -CH₂CH₂CH₂C₆H₄- ; -C₆H₄CH₂-; -C₆H₄CH₂CH₂-; -C₆H₄CH₂CH₂CH₂- ; -CH₂C₆H₄CH₂- ; -CH₂CH₂C₆H₄CH₂CH₂-; -C₆H₄C(=O)- ; -C₆H₄CH₂C(=O)- ; -C₆H₄CH₂CH₂C(=O)- ; et -CH₂CH₂C (=O) C₆H₄-.

7. Composé selon l'une quelconque des revendications 1 à 6, **caractérisé en ce que** X¹ et X² sont choisis chacun, indépendamment l'un de l'autre, dans le groupe comprenant une liaison covalente ; -CH₂- ; - CH₂CH₂- ; -CH₂CH₂CH₂- ; -CH₂CH₂CH₂CH₂- ; -CH₂CH=CHCH₂-; -CH₂C≡CCH₂- ; -CH₂C(=O)-, -CH₂CH₂C(=O)- ; - CH₂CH₂CH₂C(=O)- ; -CH₂CH₂CH₂CH₂C(=O)- ; -C₆H₄- ; - CH₂C₆H₄- ; -CH₂CH₂CH₂C₆H₄- ; -C₆H₄CH₂- ; -CH₂C₆H₄CH₂- ; -C₆H₄C(=O)- ; -C₆H₄CH₂C(=O)-, -C₆H₄CH₂CH₂C(=O)- ; et -CH₂CH₂C (=O) C₆H₄-.

8. Composé selon l'une quelconque des revendications 1 à 7, **caractérisé en ce qu'**un ou plusieurs atomes d'hydrogène dans chacun de X¹ et X² sont éventuellement remplacés par un radical choisi dans le groupe comprenant oxo ; (C₁₋₃)alkyloxy ; halogéno ; cyano ; nitro ; et formyle.

9. Composé selon l'une quelconque des revendications 1 à 8, **caractérisé en ce que** X¹ est une liaison covalente, Q¹ est hydrogène et p est 1.

10. Composé selon l'une quelconque des revendications 1 à 9, **caractérisé en ce que** R¹ et R² sont chacun, indépendamment l'un de l'autre, un radical choisi dans le groupe comprenant hydrogène ; alkyle ; alcényle ; alcynyle ; aryle ; arylalkyle ; alkylcarbonyle; alcénylcarbonyle ; alkyloxyalkyle; alkyloxycarbonyle ; alkyloxyalkylcarbonyle ; alkyloxycarbonylalkyle ; alkyloxycarbonylalkylcarbonyle ; arylcarbonyle ; aryloxyalkyle ; arylalkylcarbonyle ; Het-alkyle ; Het-alkylcarbonyle ; Het-carbonyle ; Het-carbonylalkyle ; alkyl-NR^{a}R^{b} ; carbonyl-NR^{a}R^{b} ; carbonylalkyl-NR^{a}R^{b} ; alkylcarbonyl-NR^{a}R^{b} ; et alkylcarbonylalkyl-NR^{a}R^{b} ; où R^{a} et R^{b} sont choisis chacun indépendamment dans le groupe comprenant hydrogène, alkyle, alkylcarbonyle, alkyloxyalkyle, alkyloxycarbonylalkyle, aryle, arylalkyle, Het et alkyl-NR^{c}R^{d}, où R^{c} et R^{d} sont chacun indépendamment l'un de l'autre hydrogène ou alkyle.

11. Composé selon l'une quelconque des revendications 1 à 10, **caractérisé en ce que** Pir est un radical contenant au moins un N, par lequel il est lié au radical X, choisi dans le groupe comprenant pyrrolidinyle ; pipéridinyle ; pipérazinyle ; imidazolyle ; morpholinyle ; isoindolyle ; où chaque radical Pir est éventuellement substitué par 1, 2 ou 3 radicaux choisis dans le groupe comprenant hydroxy ; halogène ; oxo ; (C₁₋₃)alkyle ; trifluorométhyle ; phényle ; benzyle ; pyrrolidinyle ; et pyridinyloxy.

12. Composé selon l'une quelconque des revendications 1 à 11, **caractérisé en ce que** Het est un radical hétérocyclique choisi dans le groupe comprenant pipéridinyle ; pipérazinyle ; triazolyle ; pyridinyle ; pyrimidinyle ; morpholinyle ; indolyle ; furyle ; thiényle ; isoxazolyle ; thiazolyle ; tétrahydrofuryle; tétrahydropyranyle ; quinoléinyle ; isoquinoléinyle ; benzofuranyle ; benzothiényle ; et benzopipéridinyle ; où chaque radical Het est éventuellement substitué par un ou plusieurs radicaux choisis dans le groupe comprenant oxo ; (C₁₋₃)alkyle ; (C₁₋₃)alkylcarbonyle ; et imidazolyl-(C₁₋₃)alkyle.

13. Composé selon l'une quelconque des revendications 1 à 12, **caractérisé en ce qu'**aryle est phényle, éventuellement substitué par 1, 2 ou 3 substituants choisis chacun indépendamment l'un de l'autre dans le groupe comprenant (C₁₋₃)alkyle ; halogéno et trifluorométhyle.

14. Composé selon la revendication 1, **caractérisé en ce que** :
V est choisi dans le groupe comprenant les radicaux (z-1), (z-2), (z-3), (z-5) et (z-6) ; où le motif -CH₂-Y- est lié à V par l'atome désigné par « a » ;
Y est un radical bivalent de formule (II) dans laquelle A est un atome d'azote ou de carbone ; m est un entier égal à zéro ou 2 ; et R⁴ est choisi dans le groupe comprenant hydrogène ; alkyle et phénylcarboxyalkyle ;
R⁵ est choisi dans le groupe comprenant hydrogène et halogéno ;
X¹, X² sont choisis chacun, indépendamment l'un de l'autre, dans le groupe comprenant une liaison covalente ; -CH₂- ; -CH₂CH₂- ;-CH₂CH₂CH₂- ; -CH₂CH₂CH₂CH₂- ; -CH₂CH=CHCH₂-; -CH₂C≡CCH₂- ; -CH₂C(=O)- ; -CH₂CH₂C(=O)-; -CH₂CH₂CH₂C(=O)- ; -CH₂CH₂CH₂CH₂C(=O)- ; -C₆H₄- ; -CH₂C₆H₄- ; -CH₂CH₂CH₂C₆H₄- ;-C₆H₄CH₂- ; -CH₂C₆H₄CH₂-; -C₆H₄C(=O)- ;-C₆H₄CH₂C(=O)- ; -C₆H₄CH₂CH₂C(=O)- ; et-CH₂CH₂C(=O)C₆H₄- ; et où un ou plusieurs atomes d'hydrogène peuvent être remplacés par un radical choisi dans le groupe comprenant oxo ; (C₁₋₃)alkyloxy ; halogéno ; cyano ; nitro ; et formyle ;
p, q sont chacun, indépendamment l'un de l'autre, un entier égal à 1 ou 2 ;
Q¹, Q² sont chacun, indépendamment l'un de l'autre, un radical choisi dans le groupe comprenant hydrogène ; -NR¹R² ; Pir ; -OR³ et Het ; où deux radicaux -OR³ peuvent être pris ensemble pour former un radical bivalent -O-(CH₂)ᵣ-O- où r est un entier égal à 1, 2 ou 3 ;
R¹ et R² sont chacun, indépendamment l'un de l'autre, un radical choisi dans le groupe comprenant hydrogène ; alkyle ; alcényle ; alcynyle ; aryle ; arylalkyle ; alkylcarbonyle; alcénylcarbonyle; alkyloxyalkyle; alkyloxycarbonyle ; alkyloxyalkylcarbonyle ; alkyloxycarbonylalkyle ; alkyloxycarbonylalkylcarbonyle ; arylcarbonyle ; aryloxyalkyle ; arylalkylcarbonyle ; Het-alkyle ; Het-alkylcarbonyle ; Het-carbonyle ; Het-carbonylalkyle ; alkyl-NR^{a}R^{b} ; carbonyl-NR^{a}R^{b} ; carbonylalkyl-NR^{a}R^{b} ; alkylcarbonyl-NR^{a}R^{b} ; e t alkylcarbonylalkyl-NR^{a}R^{b} ; où R^{a} et R^{b} sont choisis chacun indépendamment dans le groupe comprenant hydrogène, alkyle, alkylcarbonyle, alkyloxyalkyle, alkyloxycarbonylalkyle, aryle, arylalkyle, Het et alkyl-NR^{c}R^{d}, où R^{c} et R^{d} sont chacun indépendamment l'un de l'autre hydrogène ou alkyle ;
Pir est un radical contenant au moins un N, par lequel il est lié au radical X, choisi dans le groupe comprenant pyrrolidinyle ; pipéridinyle ; pipérazinyle ; imidazolyle ; morpholinyle ; isoindolyle ; où chaque radical Pir est éventuellement substitué par 1, 2 ou 3 radicaux choisis dans le groupe comprenant hydroxy ; halogéno ; oxo ; (C₁₋₃)alkyle ; t rifluorométhyle ; phényle ; benzyle ; pyrrolidinyle ; et pyridinyloxy ;
R³ est un radical choisi dans le groupe comprenant hydrogène ; alkyle ; aryle ; arylalkyle ; Het ; et Het-alkyle ;
Het est un radical hétérocyclique choisi dans le groupe comprenant pipéridinyle ; pipérazinyle ; triazolyle ; pyridinyle ; pyrimidinyle ; morpholinyle ; indolyle ; furyle ; thiényle ; isoxazolyle ; thiazolyle ; tétrahydrofuryle; tétrahydropyranyle ; quinoléinyle ; isoquinoléinyle ; benzofuranyle ; benzothiényle ; et benzopipéridinyle ; où chaque radical Het est éventuellement substitué par un ou plusieurs radicaux choisis dans le groupe comprenant oxo ; (C₁₋₃)alkyle ; (C₁₋₃)alkylcarbonyle ; et imidazolyl-(C₁₋₃)alkyle ;
aryle est phényle éventuellement substitué par 1, 2 ou 3 substituants choisis, chacun indépendamment l'un de l'autre, dans le groupe comprenant (C₁₋₃)alkyle ; halogéno et trifluorométhyle ;
alkyle est un radical hydrocarboné saturé droit ou ramifié ayant de 1 à 8 atomes de carbone ; ou est un radical hydrocarboné saturé cyclique ayant de 3 à 7 atomes de carbone ; ou est un radical hydrocarboné saturé cyclique ayant de 3 à 7 atomes de carbone lié à un radical hydrocarboné saturé droit ou ramifié ayant de 1 à 8 atomes de carbone ; où chaque radical est éventuellement substitué sur un ou plusieurs atomes de carbone par un ou plusieurs radicaux choisis dans le groupe comprenant oxo ; (C₁₋₃)alkyloxy ; halogéno ; cyano ; nitro ; formyle ; hydroxy ; amino ; carboxy ; et thio ;
alcényle est un radical alkyle tel que défini ci-dessus, ayant en outre une ou plusieurs doubles liaisons ;
alcynyle est un radical alkyle tel que défini ci-dessus, ayant en outre une ou plusieurs triples liaisons ; et
arylalkyle est un radical alkyle tel que défini ci-dessus, ayant en outre un ou plusieurs groupements CH₃- remplacés par phényle.

15. Composé selon l'une quelconque des revendications 1 à 14, destiné à être utilisé comme médicament.

16. Composition pharmaceutique comprenant un support ou un diluant pharmaceutiquement acceptable et, à titre de principe actif, une quantité thérapeutiquement efficace d'un composé selon l'une quelconque des revendications 1 à 14.

17. Composition pharmaceutique selon la revendication 16, **caractérisée en ce qu'**elle comprend en outre un ou plusieurs autres composés choisis dans le groupe comprenant les antidépresseurs, les anxiolytiques et les antipsychotiques.

18. Composition pharmaceutique selon l'une ou l'autre des revendications 16 et 17, **caractérisée en ce qu'**elle est sous une forme adaptée à l'administration par voie orale.

19. Procédé de préparation d'une composition pharmaceutique selon la revendication 16, **caractérisé en ce qu'**un support pharmaceutiquement acceptable est mélangé intimement avec une quantité thérapeutiquement efficace d'un composé selon l'une quelconque des revendications 1 à 14.

20. Procédé de préparation d'une composition pharmaceutique selon la revendication 17, **caractérisé en ce qu'**un support pharmaceutiquement acceptable est mélangé intimement avec une quantité thérapeutiquement efficace d'un composé selon l'une quelconque des revendications 1 à 14 et un ou plusieurs autres composés choisis dans le groupe comprenant les antidépresseurs, les anxiolytiques et les antipsychotiques.

21. Utilisation d'un composé selon l'une quelconque des revendications 1 à 14, pour la préparation d'un médicament destiné à la prévention et/ou au traitement de maladies pour lesquelles l'antagonisme du récepteur α₂-adrénergique, en particulier l'antagonisme du récepteur α_{2c}-adrénergique est thérapeutiquement utile.

22. Utilisation d'un composé selon l'une quelconque des revendications 1 à 14, pour la préparation d'un médicament destiné à la prévention et/ou au traitement des troubles du système nerveux central, des troubles de l'humeur, des troubles d'anxiété, des troubles liés au stress associés à la dépression et/ou à l'anxiété, des troubles cognitifs, des troubles de la personnalité, des troubles schizoaffectifs, de la maladie de Parkinson, de la démence de type Alzheimer, des affections de douleur chronique, des maladies neurodégénératives, des troubles de l'accoutumance, des troubles de l'humeur et du dysfonctionnement sexuel.

23. Utilisation d'un composé selon l'une quelconque des revendications 1 à 14, en combinaison avec un ou plusieurs autres composés choisis dans le groupe comprenant les antidépresseurs, les anxiolytiques et les antipsychotiques, pour la préparation d'un médicament destiné à la prévention et/ou au traitement des troubles du système nerveux central, des troubles de l'humeur, des troubles d'anxiété, des troubles liés au stress associés à la dépression et/ou à l'anxiété, des troubles cognitifs, des troubles de la personnalité, des troubles schizoaffectifs, de la maladie de Parkinson, de la démence de type Alzheimer, des affections de douleur chronique, des maladies neurodégénératives, des troubles de l'accoutumance, des troubles de l'humeur et du dysfonctionnement sexuel.
